# EUROPEAN PATENT APPLICATION

(11) **EP 4 748 312 A1**
(43) Date of publication of application: **27.05.2026**
(21) Application number: 24911569.2
(22) Date of filing: 27.12.2024
(51) Int. Cl.: A61B 5/1473, A61B 5/145, A61B 5/00, A61B 5/1486, A61B 5/282, A61B 5/308

(54) **MEASUREMENT METHOD, SYSTEM, AND RELATED APPARATUS**

(30) Priority: 29.12.2023 CN 202311865433
(71) Applicant: Huawei Technologies Co., Ltd., Shenzhen, Guangdong 518129 (CN)
(72) Inventor: LIU, Xiangyu, Shenzhen, Guangdong 518129 (CN)
(74) Representative: Grünecker Patent- und Rechtsanwälte PartG mbB
(86) International application number: PCT/CN2024/143450
(87) International publication number: WO 2025/140653

(57) **Abstract**

This application discloses a measurement method, a system, and a related apparatus. The method is applied to a first electronic device, the first electronic device includes a first electrode group and a second electrode group, and a distance between the first electrode group and the second electrode group is greater than a first distance. When the first electrode group and the second electrode group are implanted into a subcutaneous tissue, the method includes: determining a first physiological parameter by using the first electrode group; and determining an electrocardiography signal by using the first electrode group and the second electrode group. In this way, the first electronic device may obtain an electrocardiography signal of a user anytime and anywhere, and may further measure one or more other physiological parameters (for example, blood glucose, blood ketone, blood lactic acid, and uric acid) of the user at the same time, to improve health monitoring efficiency.

## Description

This application claims priority to Chinese Patent Application No. 202311865433.9, filed with the China National Intellectual Property Administration on December 29, 2023 and entitled "MEASUREMENT METHOD, SYSTEM, AND RELATED APPARATUS", which is incorporated herein by reference in its entirety.

### TECHNICAL FIELD

This application relates to the field of electronic technologies, and in particular, to a measurement method, a system, and a related apparatus.

### BACKGROUND

With continuous development of electronic technologies, an increasing quantity of electronic devices have a health monitoring function, so that a user can learn of a health status of the user in real time. Electrocardiography monitoring is an important part of the health monitoring function.

An electrocardiogram machine is a medical device with an electrocardiography monitoring function. During monitoring, the electrocardiogram machine may be in contact with different positions on the skin of the user through a plurality of wet electrodes, to obtain an electrocardiogram of the user.

However, the electrocardiogram machine is bulky, and is inconvenient for the user to carry. As a result, the user cannot perform electrocardiography monitoring anytime and anywhere.

### SUMMARY

This application provides a measurement method, a system, and a related apparatus, to measure an electrocardiography signal anytime and anywhere, and further measure one or more physiological parameters, thereby improving health monitoring efficiency.

According to a first aspect, this application provides a measurement method, applied to a first electronic device. The first electronic device includes a first electrode group and a second electrode group, and a distance between the first electrode group and the second electrode group is greater than a first distance. When the first electrode group and the second electrode group are implanted into a subcutaneous tissue, the method includes: determining a first physiological parameter by using the first electrode group; and determining an electrocardiography signal by using the first electrode group and the second electrode group.

In this way, an electrocardiography signal of a user may be measured anytime and anywhere, and physiological parameters such as blood glucose, blood ketone, blood lactic acid, and uric acid may be further measured.

In a possible implementation, the first electrode group includes a first working electrode and a first counter electrode, the second electrode group includes a second working electrode, and the first counter electrode forms a loop with the first working electrode. Determining the first physiological parameter by using the first electrode group specifically includes: determining the first physiological parameter by using the first working electrode and the first counter electrode. Determining the electrocardiography signal by using the first electrode group and the second electrode group specifically includes: determining the electrocardiography signal by using the first working electrode and the second working electrode, or determining the electrocardiography signal by using the first counter electrode and the second working electrode.

In this way, the first electrode group may form a dual-electrode system, and the first physiological parameter may be measured by using the dual-electrode system. The first working electrode (or the first counter electrode) in the first electrode group and any electrode in the second electrode group may be respectively used as a left arm LA electrode and a right arm RA electrode, to measure the electrocardiography signal.

In a possible implementation, the first electrode group includes a first working electrode and a first counter electrode, the second electrode group includes a second working electrode, the first counter electrode forms a loop with the first working electrode, and the first working electrode or the first counter electrode is connected to a right leg drive circuit. Determining the first physiological parameter by using the first electrode group specifically includes: determining the first physiological parameter by using the first working electrode and the first counter electrode. Determining the electrocardiography signal by using the first electrode group and the second electrode group specifically includes: determining the electrocardiography signal by using the first working electrode, the first counter electrode, and the second working electrode.

An electrode connected to the right leg drive circuit may be used as a right leg drive RLD electrode. The right leg drive circuit may be configured to cancel a common-mode signal.

In this way, the first electrode group may form a dual-electrode system, and the first physiological parameter may be measured by using the dual-electrode system. The first working electrode and the first counter electrode in the first electrode group and any electrode in the second electrode group may be respectively used as a left arm LA electrode, the right leg drive RLD electrode, and a right arm RA electrode, to measure the electrocardiography signal.

In a possible implementation, the first electrode group includes a first working electrode, a first reference electrode, and a first counter electrode, the first reference electrode is configured to control a voltage of the first working electrode, and the first counter electrode forms a loop with the first working electrode. Determining the first physiological parameter by using the first electrode group specifically includes: generating a first current by using the first working electrode; and determining the first physiological parameter based on the first current.

In this way, the first electrode group may form a three-electrode system, and the first current is generated and conducted by using the three-electrode system.

In a possible implementation, the second electrode group includes a second working electrode, a second reference electrode, and a second counter electrode, the second reference electrode is configured to control a voltage of the second working electrode, and the second counter electrode is configured to form a loop with the second working electrode. The method further includes: generating a second current by using the second working electrode; and determining a second physiological parameter based on the second current.

In this way, the second electrode group may form a three-electrode system, and the second current is generated and conducted by using the three-electrode system.

In a possible implementation, determining the electrocardiography signal by using the first electrode group and the second electrode group specifically includes: determining the electrocardiography signal by using any electrode in the first electrode group and any electrode in the second electrode group.

One electrode in the first electrode group and one electrode in the second electrode group may be respectively selected as a left arm electrode and a right arm electrode. In this way, the electrocardiography signal may be measured by using the left arm electrode and the right arm electrode.

In a possible implementation, determining the electrocardiography signal by using the first electrode group and the second electrode group specifically includes: determining the electrocardiography signal by using any two electrodes in the first electrode group and any electrode in the second electrode group; or determining the electrocardiography signal by using any electrode in the first electrode group and any two electrodes in the second electrode group.

One electrode in the first electrode group and one electrode in the second electrode group may be respectively selected as a left arm electrode and a right arm electrode. A right leg drive electrode may be an electrode in the first electrode group, or may be an electrode in the second electrode group. In this way, the electrocardiography signal may be measured by using the left arm electrode, the right arm electrode, and the right leg drive electrode. The right leg drive electrode is configured to cancel a common-mode signal by using a right leg drive circuit.

In this way, an electrochemical electrode in the first electrode group may be configured to measure the first physiological parameter, and may also be configured to measure the electrocardiography signal.

In a possible implementation, determining the electrocardiography signal by using the any two electrodes in the first electrode group and the any electrode in the second electrode group specifically includes: determining the electrocardiography signal by using the first counter electrode, the first working electrode, and the second reference electrode when the first counter electrode is connected to the right leg drive circuit.

In a possible implementation, determining the electrocardiography signal by using the any electrode in the first electrode group and the any two electrodes in the second electrode group specifically includes: determining the electrocardiography signal by using the second counter electrode, the second working electrode, and the first reference electrode when the second counter electrode is connected to the right leg drive circuit.

It should be noted that the foregoing two implementations are merely two examples. In this application, another electrode in the first electrode group and another electrode in the second electrode group may be selected to measure the electrocardiography signal. This is not limited in this application.

In a possible implementation, the first electrode group further includes a first electrocardiography electrode and a second electrocardiography electrode, the second electrode group further includes a third electrocardiography electrode, and the second electrocardiography electrode is connected to a right leg drive circuit. Determining the electrocardiography signal by using the first electrode group and the second electrode group specifically includes: determining the electrocardiography signal by using the first electrocardiography electrode, the second electrocardiography electrode, and the third electrocardiography electrode.

In this way, the electrocardiography signal may be measured by using a separately disposed electrocardiography electrode.

In a possible implementation, the first electronic device further includes a first microneedle sensor and a second microneedle sensor, the first microneedle sensor includes the first electrode group, and the second microneedle sensor includes the second electrode group.

The microneedle sensor may be a sensor whose form is similar to that of a microneedle, and a plurality of electrodes may be disposed inside the microneedle sensor.

In this way, the first electrode group and the second electrode group may be disposed in the microneedle sensor.

In a possible implementation, the first electronic device further includes a first array sensor and a second array sensor, the first array sensor includes the first electrode group, and the second array sensor includes the second electrode group.

An array sensor may be a sensor including a plurality of electrodes arranged in an array. In a possible implementation, the array sensor may alternatively be a plurality of electrodes arranged in an array.

In this way, the first electrode group and the second electrode group may be separately disposed in the array sensor in an array.

In a possible implementation, the first electronic device further includes a first microneedle sensor and a second array sensor, the first microneedle sensor includes the first electrode group, and the second array sensor includes the second electrode group.

In this way, the first electrode group may be disposed in the microneedle sensor, and the second electrode group may be disposed in the array sensor in an array.

In a possible implementation, before determining the first physiological parameter by using the first electrode group, the method further includes: determining that a first condition is satisfied, where the first condition includes any one or more of the following: first information sent by a second electronic device is received, where the first information is used to indicate the first electronic device to determine the first physiological parameter; it is detected that the first electrode group and the second electrode group are implanted into the subcutaneous tissue; and it is detected that the electrocardiography signal is abnormal.

In this way, the first condition may be a trigger condition for measuring the first physiological parameter.

In a possible implementation, before determining the electrocardiography signal by using the first electrode group and the second electrode group, the method further includes: determining that a second condition is satisfied, where the second condition includes any one or more of the following: second information sent by the second electronic device is received, where the second information is used to indicate the first electronic device to determine the electrocardiography signal; it is detected that the first electrode group and the second electrode group are implanted into the subcutaneous tissue; and it is detected that the first physiological parameter is not within a first range.

In this way, the second condition may be a trigger condition for determining the electrocardiography signal.

In a possible implementation, after determining the first physiological parameter by using the first electrode group, the method further includes: outputting the first physiological parameter, or sending the first physiological parameter to the second electronic device.

In this way, the first physiological parameter may be output, or the first physiological parameter may be output by another electronic device.

In a possible implementation, after determining the electrocardiography signal by using the first electrode group and the second electrode group, the method further includes: outputting the electrocardiography signal, or sending the electrocardiography signal to the second electronic device.

In this way, the electrocardiography signal may be output, or the electrocardiography signal may be output by another electronic device.

In a possible implementation, the first physiological parameter may include but is not limited to any one or more of the following: blood glucose, blood ketone, uric acid, blood lactic acid, and the like.

According to a second aspect, this application provides an electronic device. The electronic device is a first electronic device, and includes a first electrode group and a second electrode group. A distance between the first electrode group and the second electrode group is greater than a first distance. The first electrode group is configured to determine a first physiological parameter when the first electrode group is implanted into a subcutaneous tissue. The second electrode group is configured to determine a second physiological parameter when the second electrode group is implanted into the subcutaneous tissue. The first electrode group and the second electrode group are further configured to determine an electrocardiography signal when the first electrode group and the second electrode group are implanted into the subcutaneous tissue.

In a possible implementation, the first electrode group includes a first working electrode and a first counter electrode, the second electrode group includes a second working electrode and a second counter electrode, the first counter electrode forms a loop with the first working electrode, and the second counter electrode forms a loop with the second working electrode. That the first electrode group is configured to determine the first physiological parameter when the first electrode group is implanted into the subcutaneous tissue specifically includes: The first working electrode is configured to determine the first physiological parameter when the first working electrode is implanted into the subcutaneous tissue. That the second electrode group is configured to determine the second physiological parameter when the second electrode group is implanted into the subcutaneous tissue specifically includes: The second working electrode is configured to determine the second physiological parameter when the second working electrode is implanted into the subcutaneous tissue. That the first electrode group and the second electrode group are further configured to determine the electrocardiography signal when the first electrode group and the second electrode group are implanted into the subcutaneous tissue specifically includes: The first working electrode, the first counter electrode, and the second working electrode are further configured to determine the electrocardiography signal when the first working electrode, the first counter electrode, and the second working electrode are implanted into the subcutaneous tissue.

In a possible implementation, the first electronic device further includes a microcontroller unit MCU, the first electrode group includes a first working electrode, a first reference electrode, and a first counter electrode, and the second electrode group includes a second working electrode, a second reference electrode, and a second counter electrode. That the first electrode group is configured to determine the first physiological parameter when the first electrode group is implanted into the subcutaneous tissue specifically includes: The first working electrode is configured to generate a first current when the first working electrode is implanted into the subcutaneous tissue; the first reference electrode is configured to control a voltage of the first working electrode; the first counter electrode is configured to form a loop with the first working electrode; and the MCU is configured to determine the first physiological parameter based on the first current. That the second electrode group is configured to determine the second physiological parameter when the second electrode group is implanted into the subcutaneous tissue specifically includes: The second working electrode is configured to generate a second current when the second working electrode is implanted into the subcutaneous tissue; the second reference electrode is configured to control a voltage of the second working electrode; the second counter electrode is configured to form a loop with the second working electrode; and the MCU is configured to determine the second physiological parameter based on the second current.

In a possible implementation, that the first electrode group and the second electrode group are further configured to determine the electrocardiography signal when the first electrode group and the second electrode group are implanted into the subcutaneous tissue specifically includes: Any two electrodes in the first electrode group and any electrode in the second electrode group are configured to determine the electrocardiography signal when the first electrode group and the second electrode group are implanted into the subcutaneous tissue; or any electrode in the first electrode group and any two electrodes in the second electrode group are configured to determine the electrocardiography signal when the first electrode group and the second electrode group are implanted into the subcutaneous tissue.

One electrode in the first electrode group and one electrode in the second electrode group may be respectively selected as a left arm electrode and a right arm electrode. A right leg drive electrode may be an electrode in the first electrode group, or may be an electrode in the second electrode group. In this way, the electrocardiography signal may be measured by using the left arm electrode, the right arm electrode, and the right leg drive electrode. The right leg drive electrode is configured to cancel a common-mode signal by using a right leg drive circuit.

In a possible implementation, that the any two electrodes in the first electrode group and the any electrode in the second electrode group are configured to determine the electrocardiography signal when the first electrode group and the second electrode group are implanted into the subcutaneous tissue specifically includes: When the first counter electrode is connected to the right leg drive circuit, the first counter electrode, the first working electrode, and the second reference electrode are configured to determine the electrocardiography signal when the first electrode group and the second electrode group are implanted into the subcutaneous tissue.

In a possible implementation, that the any electrode in the first electrode group and the any two electrodes in the second electrode group are configured to determine the electrocardiography signal when the first electrode group and the second electrode group are implanted into the subcutaneous tissue specifically includes: When the second counter electrode is connected to the right leg drive circuit, the second counter electrode, the second working electrode, and the first reference electrode are configured to determine the electrocardiography signal when the first electrode group and the second electrode group are implanted into the subcutaneous tissue.

It should be noted that the foregoing two implementations are merely two examples. In this application, another electrode in the first electrode group and another electrode in the second electrode group may be selected to measure the electrocardiography signal. This is not limited in this application.

In a possible implementation, the first electrode group further includes a first electrocardiography electrode and a second electrocardiography electrode, the second electrode group further includes a third electrocardiography electrode, and the second electrocardiography electrode is connected to a right leg drive circuit. That the first electrode group and the second electrode group are further configured to determine the electrocardiography signal when the first electrode group and the second electrode group are implanted into the subcutaneous tissue specifically includes: The first electrocardiography electrode, the second electrocardiography electrode, and the third electrocardiography electrode are configured to determine the electrocardiography signal when the first electrode group and the second electrode group are implanted into the subcutaneous tissue.

In a possible implementation, the first electronic device further includes a first microneedle sensor and a second microneedle sensor, the first microneedle sensor includes the first electrode group, and the second microneedle sensor includes the second electrode group.

In a possible implementation, the first electronic device further includes a first array sensor and a second array sensor, the first array sensor includes the first electrode group, and the second array sensor includes the second electrode group.

In a possible implementation, the first electronic device further includes a first microneedle sensor and a second array sensor, the first microneedle sensor includes the first electrode group, and the second array sensor includes the second electrode group.

In a possible implementation, the first electronic device further includes a communication module. The communication module is configured to send the first physiological parameter to a second electronic device; and the communication module is further configured to send the electrocardiography signal to the second electronic device.

In a possible implementation, the first electronic device further includes an output module. The output module is configured to output the first physiological parameter; and the output module is further configured to output the electrocardiography signal.

According to a third aspect, this application provides a measurement circuit, including a first electrode group, a second electrode group, a first electrochemical circuit module, a second electrochemical circuit module, an electrocardiography circuit module, and a microcontroller unit MCU. The first electrode group is connected to the first electrochemical circuit module, and the first electrode group is connected to the electrocardiography circuit module. The second electrode group is connected to the second electrochemical circuit module, and the second electrode group is connected to the electrocardiography circuit module. The MCU is connected to the first electrochemical circuit module, the second electrochemical circuit module, and the electrocardiography circuit module. The first electrode group is configured to generate a first current signal. The first electrode group is further configured to conduct the first current signal to the first electrochemical circuit module. The first electrochemical circuit module is configured to determine a second current signal based on the first current signal. The first electrochemical circuit module is further configured to conduct the second current signal to the MCU. The MCU is configured to determine a first physiological parameter based on the second current signal. The second electrode group is configured to generate a third current signal. The second electrode group is further configured to conduct the third current signal to the second electrochemical circuit module. The second electrochemical circuit module is configured to determine a fourth current signal based on the third current signal. The second electrochemical circuit module is further configured to conduct the fourth current signal to the MCU. The MCU is configured to determine a second physiological parameter based on the fourth current signal. The first electrode group and the second electrode group are configured to obtain a first electrocardiography signal. The first electrode group and the second electrode group are further configured to conduct the first electrocardiography signal to the electrocardiography circuit module. The electrocardiography circuit module is configured to determine a second electrocardiography signal based on the first electrocardiography signal. The electrocardiography circuit module is further configured to conduct the second electrocardiography signal to the MCU.

In a possible implementation, the first electrode group includes a first working electrode, a first reference electrode, and a first counter electrode; the first electrochemical circuit module includes a first potentiostat circuit and a first transimpedance circuit; the first potentiostat circuit is configured to control voltages of the first working electrode and the first reference electrode; and the first transimpedance circuit is configured to amplify the first current signal. That the first electrode group is connected to the first electrochemical circuit module specifically includes: The first working electrode, the first reference electrode, and the first counter electrode are connected to the first potentiostat circuit; and the first working electrode is connected to the first transimpedance circuit.

In a possible implementation, the second electrode group includes a second working electrode, a second reference electrode, and a second counter electrode; the second electrochemical circuit module includes a second potentiostat circuit and a second transimpedance circuit; the second potentiostat circuit is configured to control voltages of the second working electrode and the second reference electrode; and the second transimpedance circuit is configured to amplify the third current signal. That the second electrode group is connected to the second electrochemical circuit module specifically includes: The second working electrode, the second reference electrode, and the second counter electrode are connected to the second potentiostat circuit; and the second working electrode is connected to the second transimpedance circuit.

In a possible implementation, the electrocardiography circuit module includes a right leg drive circuit, an amplification circuit, and a filter circuit, the amplification circuit is connected to the filter circuit, the right leg drive circuit is configured to cancel a common-mode signal, the amplification circuit is configured to amplify the first electrocardiography signal, and the filter circuit is configured to perform filtering. That the first electrode group is connected to the electrocardiography circuit module specifically includes: The first working electrode is connected to the amplification circuit, and the first counter electrode is connected to the right leg drive circuit. That the second electrode group is connected to the electrocardiography circuit module specifically includes: The second reference electrode is connected to the amplification circuit. That the first electrode group and the second electrode group are configured to obtain the first electrocardiography signal specifically includes: The first working electrode and the second reference electrode are configured to obtain the first electrocardiography signal. That the first electrode group and the second electrode group are further configured to conduct the first electrocardiography signal to the electrocardiography circuit module specifically includes: The first working electrode and the second reference electrode are configured to conduct the first electrocardiography signal to the amplification circuit. That the electrocardiography circuit module is configured to determine the second electrocardiography signal based on the first electrocardiography signal specifically includes: The amplification circuit is configured to: amplify the first electrocardiography signal, and then conduct an amplified first electrocardiography signal to the filter circuit; and the filter circuit is configured to determine the second electrocardiography signal based on the amplified first electrocardiography signal. That the electrocardiography circuit module is further configured to conduct the second electrocardiography signal to the MCU specifically includes: The filter circuit is further configured to conduct the second electrocardiography signal to the MCU.

In a possible implementation, the first electrode group further includes a first electrocardiography electrode and a second electrocardiography electrode, the second electrode group further includes a third electrocardiography electrode, the electrocardiography circuit module includes a right leg drive circuit, an amplification circuit, and a filter circuit, the amplification circuit is connected to the filter circuit, the right leg drive circuit is configured to cancel a common-mode signal, the amplification circuit is configured to amplify the first electrocardiography signal, and the filter circuit is configured to perform filtering. That the first electrode group is connected to the electrocardiography circuit module specifically includes: The first electrocardiography electrode is connected to the amplification circuit, and the second electrocardiography electrode is connected to the right leg drive circuit. That the second electrode group is connected to the electrocardiography circuit module specifically includes: The third electrocardiography electrode is connected to the amplification circuit. That the first electrode group and the second electrode group are configured to conduct an electrocardiography signal to the electrocardiography circuit module specifically includes: The first electrocardiography electrode and the third electrocardiography electrode are configured to conduct the electrocardiography signal to the amplification circuit. That the electrocardiography circuit module is configured to determine the second electrocardiography signal based on the first electrocardiography signal specifically includes: The amplification circuit is configured to: amplify the first electrocardiography signal, and then conduct an amplified first electrocardiography signal to the filter circuit. The filter circuit is configured to determine the second electrocardiography signal based on the amplified first electrocardiography signal. That the electrocardiography circuit module is further configured to conduct the second electrocardiography signal to the MCU specifically includes: The filter circuit is further configured to conduct the second electrocardiography signal to the MCU.

According to a fourth aspect, this application provides a measurement circuit, including a first electrode group, a second electrode group, a first multiplexer, a first electrochemical circuit module, an electrocardiography circuit module, and a microcontroller unit MCU. The first multiplexer includes a first group of input ports, a second group of input ports, and a first group of output ports, and the first multiplexer is configured to connect the first group of input ports or the second group of input ports. The first electrode group is connected to the first group of input ports, and the first electrode group is connected to the electrocardiography circuit module. The second electrode group is connected to the second group of input ports, and the second electrode group is connected to the electrocardiography circuit module. The first group of output ports are connected to the first electrochemical circuit module. The MCU is connected to the first electrochemical circuit module and the electrocardiography circuit module. The first electrode group is configured to generate a first current signal. The first electrode group is further configured to conduct the first current signal to the first multiplexer. The first multiplexer is configured to conduct the first current signal to the first electrochemical circuit module when the first group of input ports are connected. The first electrochemical circuit module is configured to determine a second current signal based on the first current signal. The first electrochemical circuit module is further configured to send the second current signal to the MCU. The MCU is configured to determine a first physiological parameter based on the second current signal. The second electrode group is configured to generate a third current signal. The second electrode group is further configured to conduct the third current signal to the first multiplexer. The first multiplexer is configured to conduct the third current signal to the first electrochemical circuit module when the second group of input ports are connected. The first electrochemical circuit module is further configured to determine a fourth current signal based on the third current signal. The first electrochemical circuit module is further configured to conduct the fourth current signal to the MCU. The MCU is configured to determine a second physiological parameter based on the fourth current signal. The first electrode group and the second electrode group are configured to obtain a first electrocardiography signal. The first electrode group and the second electrode group are further configured to conduct the first electrocardiography signal to the electrocardiography circuit module. The electrocardiography circuit module is configured to determine a second electrocardiography signal based on the first electrocardiography signal. The electrocardiography circuit module is further configured to conduct the second electrocardiography signal to the MCU.

In a possible implementation, the first electrode group includes a first working electrode, a first reference electrode, and a first counter electrode; the first group of input ports include a first input port, a second input port, and a third input port; the second electrode group includes a second working electrode, a second reference electrode, and a second counter electrode; and the second group of input ports include a fourth input port, a fifth input port, and a sixth input port. The first working electrode is connected to the first input port, the first reference electrode is connected to the second input port, and the first counter electrode is connected to the third input port. The second working electrode is connected to the fourth input port, the second reference electrode is connected to the fifth input port, and the second counter electrode is connected to the sixth input port.

In a possible implementation, the first group of output ports include a first output port, a second output port, and a third output port. That the first multiplexer is configured to connect the first group of input ports specifically includes: The first output port is configured to be connected to the first input port, the second output port is configured to be connected to the second input port, and the third output port is configured to be connected to the third input port. That the first multiplexer is configured to connect the second group of input ports specifically includes: The first output port is configured to be connected to the fourth input port, the second output port is configured to be connected to the fifth input port, and the third output port is configured to be connected to the sixth input port.

In a possible implementation, the first electrochemical circuit module includes a first potentiostat circuit and a first transimpedance circuit; the first potentiostat circuit is configured to control voltages of the first working electrode and the first reference electrode; and the first transimpedance circuit is configured to amplify the first current signal. That the first group of output ports are connected to the first electrochemical circuit module specifically includes: The first output port, the second output port, and the third output port are connected to the first potentiostat circuit; and the first output port is connected to the first transimpedance circuit.

It may be understood that the measurement circuit provided in the fourth aspect may be combined with any possible implementation of the measurement circuit provided in the third aspect.

According to a fifth aspect, this application provides a measurement circuit, including a first electrode group, a second electrode group, a second multiplexer, a third multiplexer, a fourth multiplexer, a first electrochemical circuit module, an electrocardiography circuit module, and a microcontroller unit MCU. The second multiplexer includes a third group of input ports, a fourth group of input ports, and a second group of output ports. The second multiplexer is configured to connect the third group of input ports or the fourth group of input ports. The first electrode group is connected to the third group of input ports, and the first electrode group is connected to the electrocardiography circuit module. The second electrode group is connected to the fourth group of input ports, and the second electrode group is connected to the electrocardiography circuit module. The second group of output ports are connected to the first electrochemical circuit module by using the third multiplexer and the fourth multiplexer, and the second output ports are connected to the electrocardiography circuit module by using the third multiplexer and the fourth multiplexer. The third multiplexer and the fourth multiplexer are configured to control the second group of output ports to be connected to the first electrochemical circuit module or the electrocardiography circuit module. The MCU is connected to the first electrochemical circuit module and the electrocardiography circuit module. The first electrode group is configured to generate a first current signal. The first electrode group is further configured to conduct the first current signal to the second multiplexer. The second multiplexer is configured to conduct the first current signal to the first electrochemical circuit module when the second multiplexer is connected to the third group of input ports and the third multiplexer and the fourth multiplexer control the second group of output ports to be connected to the first electrochemical circuit module. The first electrochemical circuit module is configured to determine a second current signal based on the first current signal. The first electrochemical circuit module is further configured to send the second current signal to the MCU. The MCU is configured to determine a first physiological parameter based on the second current signal. The second electrode group is configured to generate a third current signal. The second electrode group is further configured to conduct the third current signal to the first multiplexer. The first multiplexer is configured to conduct the third current signal to the first electrochemical circuit module when the second multiplexer is connected to the fourth group of input ports and the third multiplexer and the fourth multiplexer control the second group of output ports to be connected to the first electrochemical circuit module. The first electrochemical circuit module is further configured to determine a fourth current signal based on the third current signal. The first electrochemical circuit module is further configured to conduct the fourth current signal to the MCU. The MCU is configured to determine a second physiological parameter based on the fourth current signal. The first electrode group and the second electrode group are configured to obtain a first electrocardiography signal. The first electrode group and the second electrode group are further configured to conduct the first electrocardiography signal to the electrocardiography circuit module when the third multiplexer and the fourth multiplexer control the second group of output ports to be connected to the electrocardiography circuit module. The electrocardiography circuit module is configured to determine a second electrocardiography signal based on the first electrocardiography signal. The electrocardiography circuit module is further configured to conduct the second electrocardiography signal to the MCU.

It may be understood that the measurement circuit provided in the fifth aspect may be combined with any possible implementation of the measurement circuit provided in the third aspect and the fourth aspect.

According to a sixth aspect, this application provides a chip system, used in a first electronic device. The chip system includes a processing circuit and an interface circuit. The interface circuit is configured to receive code instructions and transmit the code instructions to the processing circuit. The processing circuit is configured to run the code instructions, to enable the chip system to perform the measurement method in any one of the possible implementations of any one of the foregoing aspects.

According to a seventh aspect, an embodiment of this application provides a readable storage medium, including instructions. When the instructions are run on a first electronic device, the first electronic device is enabled to perform the measurement method in any one of the possible implementations of any one of the foregoing aspects.

According to an eighth aspect, an embodiment of this application provides a computer program product. When the computer program product runs on a first electronic device, the first electronic device is enabled to perform the measurement method in any one of the possible implementations of any one of the foregoing aspects.

For beneficial effects of the second aspect to the eighth aspect, refer to the beneficial effects of the first aspect.

### BRIEF DESCRIPTION OF DRAWINGS

FIG. 1A is a diagram of a scenario in which a physiological parameter is measured by using a three-electrode system according to an embodiment of this application;
FIG. 1B is a diagram of a system architecture of a measurement system 10 according to an embodiment of this application;
FIG. 2A is a diagram of a hardware structure of an electronic device 100 according to an embodiment of this application;
FIG. 2B is a diagram of a hardware structure of an electronic device 200 according to an embodiment of this application;
FIG. 3A is a diagram of a device form of an electronic device 200 according to an embodiment of this application;
FIG. 3B is a diagram of an internal structure of an electronic device 200 according to an embodiment of this application;
FIG. 3C is a diagram of distribution of electrodes on a microneedle sensor 303 according to an embodiment of this application;
FIG. 3D is a diagram of a device form of another electronic device 200 according to an embodiment of this application;
FIG. 3E is a diagram of an internal structure of another electronic device 200 according to an embodiment of this application;
FIG 4A and FIG. 4B are diagrams of two connection relationships between circuit modules and processors according to an embodiment of this application;
FIG. 4C to FIG. 4J are diagrams of a group of measurement circuits according to an embodiment of this application;
FIG. 5A is a schematic flowchart of a measurement method according to an embodiment of this application;
FIG. 5B is a diagram of a waveform of an electrocardiography signal according to an embodiment of this application;
FIG. 5C shows an electrocardiogram according to an embodiment of this application;
FIG. 6 is a schematic flowchart of another measurement method according to an embodiment of this application;
FIG. 7A to FIG. 7F are diagrams of a group of output interfaces of physiological parameters according to an embodiment of this application;
FIG. 7G to FIG. 7J are diagrams of a group of output interfaces of electrocardiography signals according to an embodiment of this application;
FIG. 7K and FIG. 7L are diagrams of a group of interfaces for triggering electrocardiography monitoring when a physiological parameter is abnormal according to an embodiment of this application;
FIG. 7M and FIG. 7N are diagrams of a group of interfaces for executing a Health Glance function according to an embodiment of this application;
FIG. 8 is a diagram of functional modules of an electronic device 200 according to an embodiment of this application;
FIG. 9 is a diagram of functional modules of a measurement system 10 according to an embodiment of this application;
FIG. 10 is a diagram of a physical entity apparatus of an electronic device 300 according to an embodiment of this application; and
FIG. 11 is a schematic flowchart of a measurement method according to an embodiment of this application.

### DESCRIPTION OF EMBODIMENTS

The following clearly and completely describes the technical solutions in embodiments of this application with reference to the accompanying drawings. In the descriptions of embodiments of this application, "/" indicates or, unless otherwise specified. For example, A/B may indicate A or B. In this specification, "and/or" describes only an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more than two.

The following terms "first" and "second" are merely intended for a purpose of description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language like Java or an extensible markup language (extensible markup language, XML). Interface source code is parsed and rendered on an electronic device, and is finally presented as content that can be recognized by the user. A frequently-used representation form of the user interface is a graphical user interface (graphical user interface, GUI), and is a user interface that is displayed in a graphical manner and that is related to a computer operation. The user interface may be a visual interface element like a text, an icon, a button, a menu, a tab, a text box, a dialog box, a status bar, a navigation bar, or a widget that is displayed on a display of the electronic device.

The following describes some terms in embodiments of this application.

Electrocardiogram: The electrocardiogram (electrocardiogram, ECG) is a curve of bioelectric potential changes generated during heart beating, and is an important physiological indicator indicating a process of occurrence, propagation, and recovery of cardiac electrical excitation.

Electrocardiography: The heart is excited by the pacemaker, atriums, and ventricles in succession in each cardiac cycle, accompanied by bioelectric changes, and these bioelectric changes are referred to as electrocardiography. The heart pumps blood at a rhythm, and a systolic and diastolic rhythm of the heart is controlled by an electrocardiography activity. In normal cases, a sinus node regularly emits impulses, and the impulses pass through a special conduction system, so that entire myocardium generates electric impulses. An electric field generated by the electric impulses spreads throughout the body, and generated tiny currents are conducted to different positions of the human body through body tissues, so that different potentials are generated in different parts of the human body. Therefore, electrodes are separately disposed at different positions on a body surface, so that a potential difference between the different positions on the body surface can be measured, to determine an electrocardiogram.

Electrode lead system: The electrode lead system indicates a quantity and an arrangement manner of a plurality of electrodes for measuring electrocardiography. The electrode lead system may include but is not limited to any one or more of the following: an international standard 12-lead system, a bipolar lead system, and the like.

International standard 12-lead system: In the international standard 12-lead system, 10 electrodes need to be disposed on the body surface. The 10 electrodes are respectively located on the left arm (left arm, LA), right arm (right arm, RA), left leg (left leg, LL), and right leg (right leg, RL), and the remaining six electrodes are located on the chest. The electrode on the right leg may be used as a reference electrode, and the remaining nine electrodes may be used as electrocardiography monitoring electrodes, to measure an electrocardiography signal of the body surface of the human body (for example, a potential difference between different parts of the body surface), and determine an electrocardiogram based on the measured electrocardiography signal.

Bipolar lead system: The bipolar lead system may include three electrodes: a left arm (LA) electrode, a right arm (RA) electrode, and a right leg (RL) electrode. Generally, the left arm electrode is disposed on the left arm, the right arm electrode is disposed on the right arm, and the right leg electrode is disposed on the right leg. It should be noted that the three electrodes may alternatively be separately disposed at other positions of the human body. In the bipolar lead system, an electrocardiography signal of the body surface may be obtained by measuring a potential difference between two limbs, and an electrocardiogram is determined based on the measured electrocardiography signal. The left arm electrode and the right arm electrode are configured to obtain the electrocardiography signal of the body surface (for example, a potential difference between the left arm and the right arm), and the right leg electrode may be used as a reference electrode.

Common-mode interference: During electrocardiography measurement, the right leg may be considered as grounded. In this case, the LA electrode and the RA electrode measure a ground-based electrocardiography signal. However, ground impedance exists when the human body is grounded, and the ground impedance introduces common-mode interference to generate a common-mode signal. A right leg drive (right leg drive, RLD) circuit may cancel the common-mode signal. The RLD circuit may be connected to the RL electrode, to cancel the signal, thereby improving measurement accuracy.

Right leg drive electrode: The right leg drive (RLD) electrode is an RL electrode connected to the RLD circuit. The RLD electrode, the LA electrode, and the RA electrode may form a bipolar lead system, to measure an electrocardiography signal. In embodiments of this application, an electrode configured to measure the electrocardiography signal may also be referred to as an electrocardiography electrode. When the electrocardiography signal is measured by using the bipolar lead system, the electrocardiography electrode may include the LA electrode, the RA electrode, and the RLD electrode.

Blood glucose: The blood glucose refers to glucose in blood. The glucose is an important component of the human body and an important source of energy. A healthy human body needs a large amount of sugar every day to provide energy for normal operation of various tissues and organs. The blood glucose needs to be kept within a specific range to satisfy needs of all organs and tissues in the body. Excessively high blood glucose is likely to cause diabetes, and excessively low blood glucose is likely to cause insufficient energy for organs in the human body, resulting in serious consequences.

Blood ketone: The blood ketone refers to ketone content in the blood. Ketone bodies are generated during metabolism of muscles and fats through exercise, and then enter the blood. The blood ketone within a normal range does not have a negative impact on the human body. When the blood ketone is excessively high (for example, greater than a specific value), there is a risk of acidosis for the human body.

Blood lactic acid: The blood lactic acid (blood lactic acid) refers to a concentration of lactic acid in the blood. The blood lactic acid is an intermediate product of glucose metabolism in the body, and is mainly produced by red blood cells, striated muscles, and brain tissues. The concentration of the lactic acid in the blood mainly depends on a synthesis speed and metabolism rate of the liver and kidneys. Lactic acid monitoring is to measure the concentration of the lactic acid in the blood, and the lactic acid monitoring helps determine whether liver and kidney functions of a user are normal.

Uric acid: The uric acid is a final product of purine metabolism and is trioxypurine, and an enol form of the uric acid is weakly acidic. The human body contains the uric acid, and the uric acid can be excreted in urine. An imbalance between uric acid production and excretion in the body is likely to cause an increase in blood uric acid, leading to a disease. The uric acid is mainly generated in the liver and mostly filtered by the glomerulus and excreted in urine. Therefore, uric acid monitoring helps determine whether the liver and kidney functions of the user are normal.

Electrochemical electrode: The electrochemical electrode reacts with a specific substance to generate a current or voltage. In embodiments of this application, the electrochemical electrode may be configured to measure one or more physiological parameters, for example, blood glucose, blood ketone, uric acid, and blood lactic acid. Based on different functions, the electrochemical electrode may include the following types: a working electrode, a counter electrode, and a reference electrode. A plurality of electrochemical electrodes with different functions may form an electrochemical system to measure a physiological parameter. Common electrochemical systems include a dual-electrode system and a three-electrode system.

Dual-electrode system: The dual-electrode system may include a working electrode and a counter electrode, or a working electrode and a reference electrode. In the dual-electrode system, the working electrode may be configured to generate a researched reaction, and the counter electrode (or the reference electrode) may form a loop with the working electrode, and may further control a voltage of the working electrode.

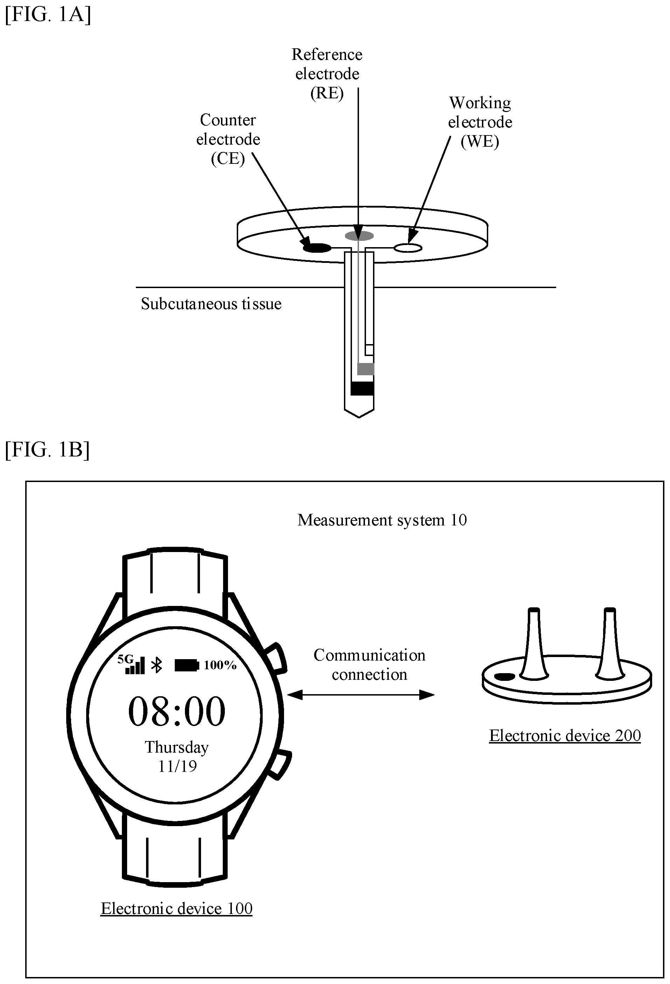

Three-electrode system: The three-electrode system may include a working electrode, a reference electrode, and a counter electrode. In the three-electrode system, the working electrode may be configured to generate a researched reaction, the counter electrode may form a loop with the working electrode, and the reference electrode may control a voltage of the working electrode. For a specific example of the three-electrode system, refer to related content in the embodiment shown in FIG. 1A below.

Continuous glucose monitoring device: The continuous glucose monitoring (continuous glucose monitoring, CGM) device is an electronic device configured to measure blood glucose. The CGM device may include an electrochemical electrode, and a glucose enzyme (for example, glucose oxidase) may be disposed in the electrochemical electrode. After the CGM device is implanted into the skin of the user, the glucose enzyme in the electrochemical electrode may react with glucose in tissue fluid to generate a current. The CGM device may measure the current generated when the glucose enzyme reacts with the glucose, determine a concentration of the glucose in the tissue fluid of the user based on a magnitude of the current, and determine a concentration of glucose in the blood of the user, namely, a blood glucose value of the user, based on the concentration of the glucose in the tissue fluid.

Enzyme: The enzyme is a protein or ribonucleic acid (ribonucleic acid, RNA) that is produced by a living cell and that has high specificity and high catalytic efficiency to a substrate of the enzyme. A capability of the enzyme to catalyze a chemical reaction is referred to as enzyme activity (also referred to as enzyme vitality). The enzyme activity is related to a temperature. Different types of enzymes correspond to different most suitable temperatures. When a temperature of an environment in which the enzyme is located is the most suitable temperature, the enzyme has the highest activity and strongest catalytic capability.

For example, FIG. 1A is a diagram of a scenario in which a physiological parameter is measured by using a three-electrode system according to an embodiment of this application.

As shown in FIG. 1A, the three-electrode system may include a working electrode, a reference electrode, and a counter electrode. The working electrode (working electrode, WE) is also referred to as a research electrode, and the working electrode is an electrode configured to perform a researched reaction. An electrochemical electrode for measuring blood glucose is used as an example. The working electrode may have a substance (for example, glucose oxidase) that can react with glucose, and the working electrode may be configured to react with the glucose to generate a reaction current. The counter electrode (counter electrode, CE) is also referred to as an auxiliary electrode. The counter electrode may form a loop with the working electrode, so that the current of the working electrode is smooth, to ensure that the researched reaction occurs on the working electrode. The reference electrode (reference electrode, RE) is an electrode with a known electric potential and close to an ideal non-polarized electrode. The reference electrode is almost free of current and is configured to control a voltage of the working electrode.

When the working electrode, the reference electrode, and the counter electrode are implanted into a subcutaneous tissue, the working electrode may react with a target substance (for example, glucose, a ketone body, uric acid, or lactic acid) to generate the reaction current. A value of the physiological parameter may be determined by measuring a magnitude of the reaction current.

The following describes a system architecture of a measurement system 10 according to an embodiment of this application.

As shown in FIG. 1B, the measurement system 10 may include an electronic device 100 and an electronic device 200. A communication connection may be established between the electronic device 100 and the electronic device 200. The communication connection may be a wired connection or a wireless connection. The wireless communication connection may be a wireless communication connection established by the electronic device 100 and the electronic device 200 by using any one of wireless communication technologies such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, a NearLink (NearLink) connection, or intrabody communication (intrabody communication, IBC).

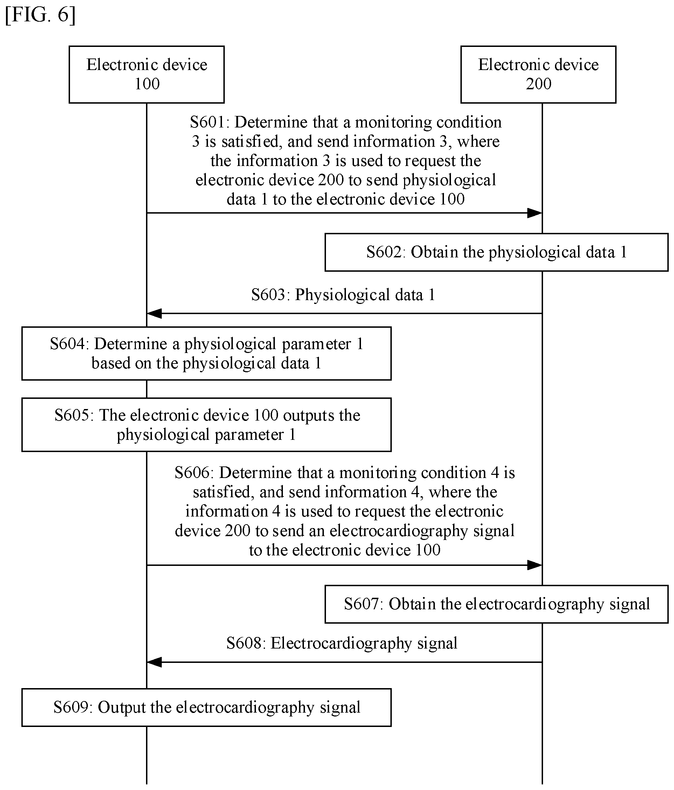

In some embodiments, the electronic device 100 may send information 3 to the electronic device 200 when detecting that a monitoring condition 3 is satisfied. The information 3 is used to request the electronic device 200 to send physiological data 1 to the electronic device 100, and the physiological data 1 is used to determine a physiological parameter 1 (for example, blood glucose, blood ketone, uric acid, or blood lactic acid) of a user. In some embodiments, the electronic device 100 may alternatively send information 4 to the electronic device 200 when detecting that a monitoring condition 4 is satisfied. The information 4 is used to request the electronic device 200 to send an electrocardiography signal to the electronic device 100. For specific content of the monitoring condition 3 and the monitoring condition 4, refer to related descriptions in the embodiment shown in FIG. 6 below. Details are not described herein. In some embodiments, the electronic device 100 may alternatively send information 5 to the electronic device 200. The information 5 is used to request the electronic device 200 to send the electrocardiography signal and the physiological data 1 to the electronic device 100.

The electronic device 100 may receive physiological data sent by the electronic device 200, and determine and output a physiological parameter of the user based on the physiological data. The electronic device 100 may further receive and output the electrocardiography signal sent by the electronic device 200.

The electronic device 200 may include a plurality of electrochemical electrodes, and the electrochemical electrode may react with a specific chemical substance to generate a reaction current. The electronic device 200 may obtain the physiological data of the user. The physiological data may be used to determine any one or more of physiological parameters such as blood glucose, blood ketone, uric acid, and blood lactic acid. The electronic device 200 may further obtain the electrocardiography signal of the user by using a plurality of electrocardiography electrodes. It should be noted that, in some embodiments, the plurality of electrocardiography electrodes may be a plurality of electrodes in the plurality of electrochemical electrodes, or may be electrodes different from the plurality of electrochemical electrodes. In some other embodiments, one or more of the plurality of electrocardiography electrodes may be the same as one or more of the plurality of electrochemical electrodes.

In some embodiments, the electronic device 200 may receive the information 3 and send the physiological data of the user to the electronic device 100 in response to the information 3. The electronic device 200 may alternatively receive the information 4 and send the electrocardiography signal to the electronic device 100 in response to the information 4.

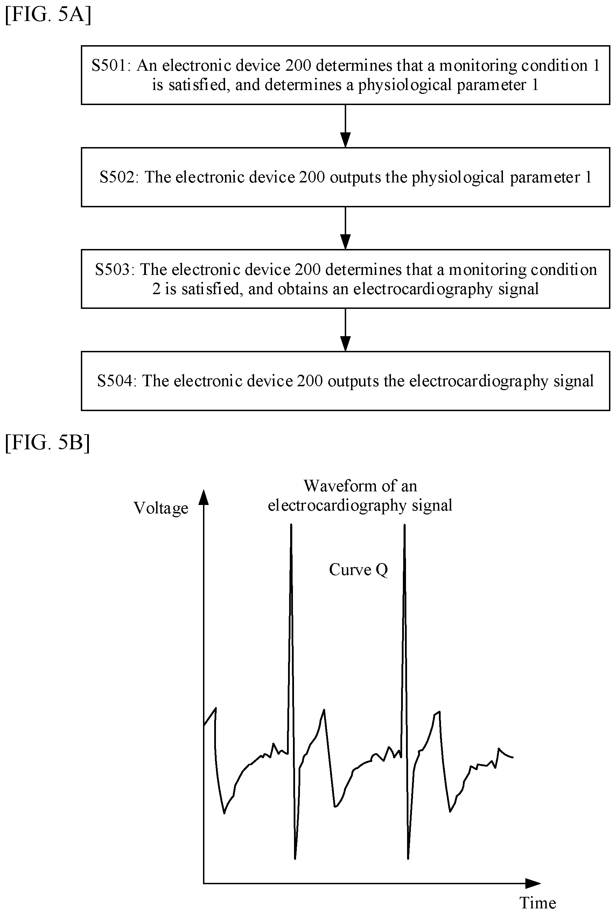

In some other embodiments, the electronic device 200 may further determine and output the physiological parameter 1 of the user (for example, send the physiological parameter 1 to the electronic device 100) when detecting that a monitoring condition 1 is satisfied. The electronic device 200 may further obtain and output the electrocardiography signal of the user (for example, send the electrocardiography signal to the electronic device 100) when detecting that a monitoring condition 2 is satisfied. For specific content of the monitoring condition 1 and the monitoring condition 2, refer to related descriptions in the embodiment shown in FIG. 5A below. Details are not described herein.

In this embodiment of this application, the electronic device 100 may be a wearable device like a watch or a band, or may be a mobile phone, a display, a tablet computer, a computer, or the like. The electronic device 200 may be configured to measure one or more physiological parameters, and may be further configured to measure the electrocardiography signal. Device types of the electronic device 100 and the electronic device 200 are not limited in this application.

It may be understood that the measurement system 10 shown in FIG. 1B is merely an example. In embodiments of this application, the measurement system 10 may alternatively include more or fewer electronic devices than those in the foregoing embodiment, or include electronic devices whose device forms are different from those in the foregoing embodiment. This is not limited in this application.

The following describes a hardware structure of the electronic device 100 according to embodiments of this application.

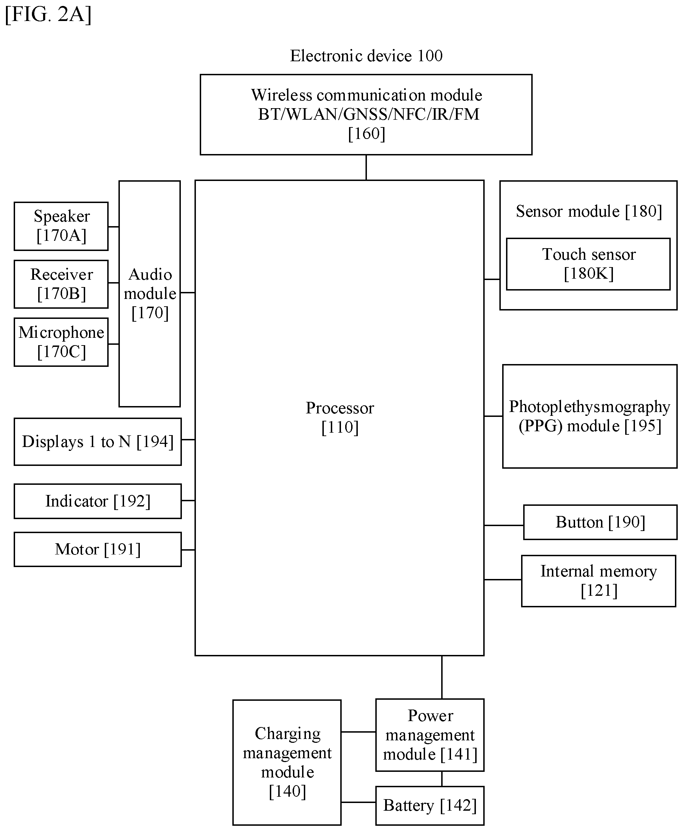

FIG. 2A is a diagram of a hardware structure of the electronic device 100 according to an embodiment of this application.

The electronic device 100 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, a vehicle-mounted device, a smart home device, and/or a smart city device. A specific type of the electronic device is not limited in embodiments of this application.

The electronic device 100 may include a processor 110, an internal memory 121, a charging management module 140, a power management module 141, a battery 142, a sensor module 180, a display 194, and the like. Optionally, the electronic device 100 may further include any one or more of the following: a wireless communication module 160, an audio module 170, a button 190, a motor 191, an indicator 192, a photoplethysmography (photoplethysmography, PPG) module 195, an airbag, and the like. The audio module 170 may include any one or more of the following: a speaker 170A, a receiver 170B, and a microphone 170C. The sensor module 180 may include a touch sensor 180K.

It may be understood that the structure illustrated in this embodiment of the present invention does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor 110 may directly invoke the instructions or the data from the memory. This avoids repeated access and reduces waiting time of the processor 110, thereby improving system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The charging management module 140 is configured to receive a charging input from a charger. The charger may be a wireless charger or a wired charger. In some embodiments of wired charging, the charging management module 140 may receive a charging input of a wired charger. In some embodiments of wireless charging, the charging management module 140 may receive a wireless charging input through a wireless charging coil of the electronic device 100. When charging the battery 142, the charging management module 140 may further supply power to the electronic device through the power management module 141.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same component.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, a NearLink (NearLink) connection, intrabody communication (intrabody communication, IBC), or the like. For example, when two electronic devices communicate with each other by using an intrabody communication solution, the two electronic devices each have at least one electrode in contact with skin, and the two electronic devices receive/send information from/to each other through a human body by using the electrodes in contact with the skin. The wireless communication module 160 may be one or more components integrating at least one communication processing module.

The electronic device 100 implements a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, or the like. The display 194 includes a display panel. The display panel may be a liquid crystal display (liquid crystal display, LCD), or the display panel may be an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device 100 may include one or N displays 194, where N is a positive integer greater than 1.

The internal memory 121 may include one or more random access memories (random access memories, RAMs) and one or more nonvolatile memories (nonvolatile memories, NVMs). The random access memory may be directly read and written by the processor 110, may be configured to store an executable program (for example, machine instructions) of an operating system or another running program, and may be further configured to store data of a user, data of an application, and the like. The nonvolatile memory may also store the executable program, the data of the user, the data of the application, and the like, which may be loaded into the random access memory in advance for the processor 110 to directly perform reading and writing.

The electronic device 100 may implement audio functions, for example, music playing and recording, by using the audio module 170, the speaker 170A, the receiver 170B, the microphone 170C, the application processor, and the like.

The audio module 170 is configured to convert digital audio information into an analog audio signal for output, and is also configured to convert an analog audio input into a digital audio signal. The audio module 170 may be further configured to encode and decode an audio signal. In some embodiments, the audio module 170 may be disposed in the processor 110, or a part of functional modules of the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an electrical audio signal into a sound signal. When a call is answered or a voice message is received through the electronic device 100, the receiver 170B may be put close to a human ear to listen to a voice.

The microphone 170C, also referred to as a "mike" or a "mic", is configured to convert a sound signal into an electrical signal. When making a call or sending a voice message, the user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and recognize a sound source, so as to implement a directional recording function or the like.

The touch sensor 180K is also referred to as a "touch component". The touch sensor 180K may be disposed in the display 194, and the touch sensor 180K and the display 194 form a touchscreen, which is also referred to as a "touch screen". The touch sensor 180K is configured to detect a touch operation performed on or near the touch sensor 180K. The touch sensor may transfer the detected touch operation to the application processor to determine a type of a touch event. A visual output related to the touch operation may be provided through the display 194. In some other embodiments, the touch sensor 180K may alternatively be disposed on a surface of the electronic device 100 at a position different from that of the display 194.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playing) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, or the like.

The PPG module 195 is an optional component. The PPG module 195 may include a transmitter and a receiver. The transmitter may be configured to transmit infrared light or green light, and the receiver may be configured to receive infrared light or green light reflected by a biological tissue (for example, skin or blood). In some embodiments, the PPG module 195 may measure any one or more of the following physiological information: a blood oxygen concentration, a heart rate, blood pressure, a respiratory rate, and the like.

In some embodiments, the sensor module 180 of the electronic device 100 may further include any one or more of the following sensors: an acceleration sensor, a barometric pressure sensor, a temperature sensor, a gyroscope sensor, and the like.

The acceleration sensor may detect magnitudes of accelerations of the electronic device 100 in various directions (generally on three axes). When the electronic device 100 is still, a magnitude and a direction of gravity may be detected. The acceleration sensor may be further configured to recognize a posture of the electronic device, and is used in an application like switching between a landscape mode and a portrait mode or a pedometer.

The barometric pressure sensor may be configured to measure barometric pressure. In some embodiments, the barometric pressure sensor may also be configured to measure water pressure.

The temperature sensor may be configured to measure a body temperature of the user, or may be configured to measure a temperature of an environment in which the user is located.

The gyroscope sensor may be configured to determine a motion posture of the electronic device 100. In some embodiments, an angular velocity of the electronic device 100 around three axes (namely, axes x, y, and z) may be determined by using the gyroscope sensor.

In some embodiments, the electronic device 100 may further include the airbag, and the airbag may be used to measure blood pressure.

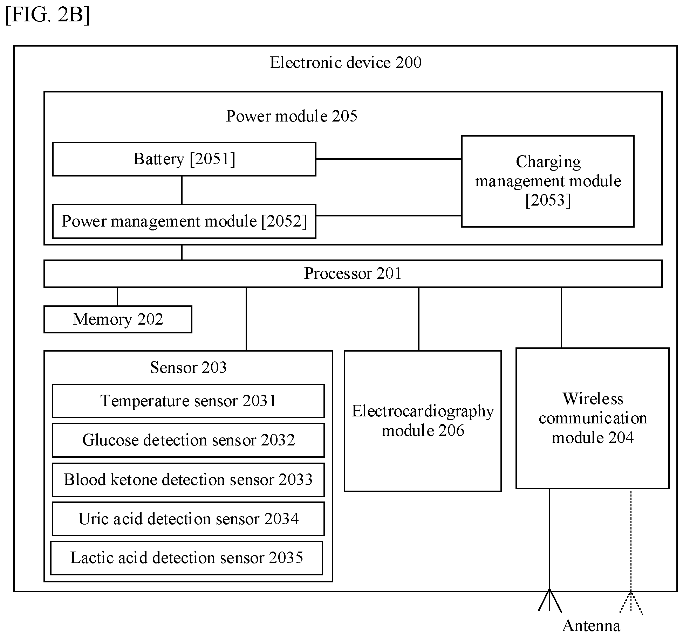

FIG. 2B is a diagram of a hardware structure of the electronic device 200 according to an embodiment of this application.

As shown in FIG. 2B, the electronic device 200 includes a processor 201, a memory 202, a sensor 203, a wireless communication module 204, a power module 205, an electrocardiography module 206, and the like.

It may be understood that the structure illustrated in this embodiment of the present invention does not constitute a specific limitation on the electronic device. In some other embodiments of this application, the electronic device may include more or fewer components than those shown in the figure, or some components may be combined, or some components may be split, or different component arrangements may be used. The components shown in the figure may be implemented by hardware, software, or a combination of software and hardware.

The processor 201 may include one or more processing units. For example, the processor 201 may be a modem processor, a digital signal processor, a controller, a baseband processor, a neural-network processing unit, and/or the like. Different processing units may be independent components, or may be integrated into one or more processors. The processor 201 may also be referred to as a microcontroller unit (microcontroller unit, MCU).

The controller may generate an operation control signal based on an instruction operation code and a time sequence signal, to complete control of instruction reading and instruction execution.

A memory may be further disposed in the processor 201, and is configured to store instructions and data. In some embodiments, the memory in the processor 201 is a cache. The memory may store instructions or data just used or cyclically used by the processor 201. If the processor 201 needs to use the instructions or the data again, the processor 201 may directly invoke the instruction or the data from the memory. This avoids repeated access and reduces waiting time of the processor 201, thereby improving system efficiency.

The wireless communication module 204 may provide a wireless communication solution that is applied to the electronic device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, a NearLink (NearLink) connection, intrabody communication (intrabody communication, IBC), or the like. For example, when two electronic devices communicate with each other by using an intrabody communication solution, the two electronic devices each have at least one electrode in contact with skin, and the two electronic devices receive/send information from/to each other through a human body by using the electrodes in contact with the skin. The wireless communication module 204 may be one or more components integrating at least one communication processing module. The wireless communication module 204 receives an electromagnetic wave through an antenna, performs frequency modulation and filtering on an electromagnetic wave signal, and sends a processed signal to the processor 201. The wireless communication module 204 may further receive a to-be-sent signal from the processor 201, perform frequency modulation and amplification on the signal, and convert the signal into an electromagnetic wave for radiation through the antenna.

The memory 202 may include one or more random access memories and one or more nonvolatile memories.

The nonvolatile memory may include a magnetic disk storage component and a flash memory.

The random access memory may be directly read and written by the processor 201, may be configured to store an executable program (for example, machine instructions) of an operating system or another running program, and may be further configured to store data of a user, data of an application, and the like.

The nonvolatile memory may also store the executable program, the data of the user, the data of the application, and the like, which may be loaded into the random access memory in advance for the processor 201 to directly perform reading and writing.

The sensor 203 may include but is not limited to any one or more of the following: a glucose detection sensor 2032, a blood ketone detection sensor 2033, a uric acid detection sensor 2034, a lactic acid detection sensor 2035, and the like. Optionally, the sensor 203 may further include a temperature sensor 2031.

The temperature sensor 2031 is configured to detect a temperature. In some embodiments, the electronic device 100 determines a skin temperature of the user and/or an ambient temperature by using the temperature detected by the temperature sensor 2031.

In some embodiments, the electronic device 200 may measure blood glucose of the user. During specific implementation, the electronic device 200 may measure a tissue fluid glucose concentration (tissue fluid glucose) by using the glucose detection sensor 2032, to obtain a glucose concentration (blood glucose) in plasma (blood) through calculation.

The glucose detection sensor 2032 is configured to detect a glucose concentration. In some embodiments, the glucose detection sensor 2032 may determine the glucose concentration by detecting consumption of oxygen under a catalytic action of glucose oxidase or H2O2 generated by a glucose oxidation reaction in tissue fluid. In some embodiments, the glucose detection sensor 2032 connects the glucose oxidase to an electrode surface by using an electronic medium like a nanomaterial, metal osmium, ferrocene, or benzoquinone, then implements electron transfer through a series of oxidation-reduction reactions, and further determines the glucose concentration.

In a process in which the user measures blood glucose by using the electronic device 200, the user may implant the electronic device 200 into a subcutaneous tissue, and the electronic device 200 measures the tissue fluid glucose concentration by using an electrode of the glucose detection sensor 2032, to determine the blood glucose concentration of the user.

In some embodiments, the electronic device 200 may measure blood ketone of the user. During specific implementation, the electronic device 200 may measure a ketone body concentration in tissue fluid by using the blood ketone detection sensor 2033, to obtain a ketone concentration (blood ketone) in the plasma (blood) through calculation. Alternatively, the electronic device 200 may measure the blood ketone concentration in the blood.

The blood ketone detection sensor 2033 is configured to detect the blood ketone concentration. In a process in which the user measures blood ketone by using the electronic device 200, the user may implant the electronic device 200 into the subcutaneous tissue, and the electronic device 200 measures the blood ketone concentration in the tissue fluid by using an electrode of the blood ketone detection sensor 2033, to determine the blood ketone concentration of the user. In some other embodiments, the user may alternatively drop sampled blood into the electronic device 200, and the electronic device 200 measures the blood ketone concentration in the blood by using the electrode of the blood ketone detection sensor 2033.

In some embodiments, the electronic device 200 may measure uric acid in the body of the user. During specific implementation, the electronic device 200 may measure a ketone body concentration in the tissue fluid by using the uric acid detection sensor 2034, to obtain a uric acid concentration (uric acid) in the plasma (blood) through calculation. Alternatively, the electronic device 200 may measure the uric acid concentration in the blood, or measure a uric acid concentration in urine of the user.

The uric acid detection sensor 2034 is configured to detect the uric acid concentration. In a process in which the user measures uric acid by using the electronic device 200, the user may implant the electronic device 200 into the subcutaneous tissue, and the electronic device 200 measures a uric acid concentration in the tissue fluid by using an electrode of the uric acid detection sensor 2034, to determine the uric acid concentration of the user. In some other embodiments, the user may alternatively drop sampled blood (or urine) into the electronic device 200, and the electronic device 200 measures the uric acid concentration in the blood (or the urine) by using the electrode of the uric acid detection sensor 2034.

The lactic acid detection sensor 2035 is configured to detect a blood lactic acid concentration. In a process in which the user measures blood lactic acid by using the electronic device 200, the user may implant the electronic device 200 into the subcutaneous tissue, and the electronic device 200 measures a lactic acid concentration in the tissue fluid by using an electrode of the lactic acid detection sensor 2035, to determine the blood lactic acid concentration of the user. In some other embodiments, the user may alternatively drop sampled blood (or tissue fluid) into the electronic device 200, and the electronic device 200 measures the lactic acid concentration in the blood (or the tissue fluid) by using the electrode of the lactic acid detection sensor 2035.

In some embodiments, the electronic device 200 may send collected physiological data to another device, for example, the electronic device 100, through the wireless communication module 204.

The memory 202 may be configured to store the physiological data collected by the electronic device 200, and optionally may be further configured to store a body temperature of the user.

The power module 205 may include a battery 2051 and a power management module 2052. Optionally, the power module 205 may further include a charging management module 2053, and the like.

The charging management module 2053 is configured to receive a charging input from a charger. The charger may be a wireless charger or a wired charger. In some embodiments of wired charging, the charging management module 2053 may receive a charging input of a wired charger. In some embodiments of wireless charging, the charging management module 2053 may receive a wireless charging input through a wireless charging coil of the electronic device 100. When charging the battery 2051, the charging management module 2053 may further supply power to the electronic device through the power management module 2052.

The power management module 2052 is configured to connect to the battery 2051, the charging management module 2053, and the processor 110. The power management module 2052 receives an input of the battery 2051 and/or the charging management module 2053, and supplies power to the processor 201, the memory 202, the wireless communication module 204, or the like. The power management module 2052 may be further configured to monitor parameters such as a battery capacity, a battery cycle count, and a battery health status (electric leakage or impedance). In some other embodiments, the power management module 2052 may alternatively be disposed in the processor 201. In some other embodiments, the power management module 2052 and the charging management module 2053 may alternatively be disposed in a same component.

The electrocardiography module 206 may have an electrocardiography monitoring function, and is configured to measure an electrocardiography signal of the user and obtain an electrocardiogram of the user.

It should be understood that FIG. 2B merely shows an example of the hardware structure of the electronic device 200. In another embodiment of this application, the electronic device 200 may include more or fewer components. This is not limited in embodiments of this application.

Embodiments of this application provide a measurement method, applied to a first electronic device (also referred to as the electronic device 200). The first electronic device includes a first electrode group (also referred to as an electrode group 1) and a second electrode group (also referred to as an electrode group 2), a distance between the first electrode group and the second electrode group is greater than a first distance. When the first electrode group and the second electrode group are implanted into a subcutaneous tissue, the method includes: determining a first physiological parameter by using the first electrode group; and determining an electrocardiography signal by using the first electrode group and the second electrode group.

In this way, the electronic device 200 may obtain an electrocardiography signal of a user anytime and anywhere, and may further measure one or more other physiological parameters (for example, blood glucose, blood ketone, blood lactic acid, and uric acid) of the user at the same time, to improve health monitoring efficiency.

The following describes a device form and an internal structure of the electronic device 200 according to embodiments of this application.

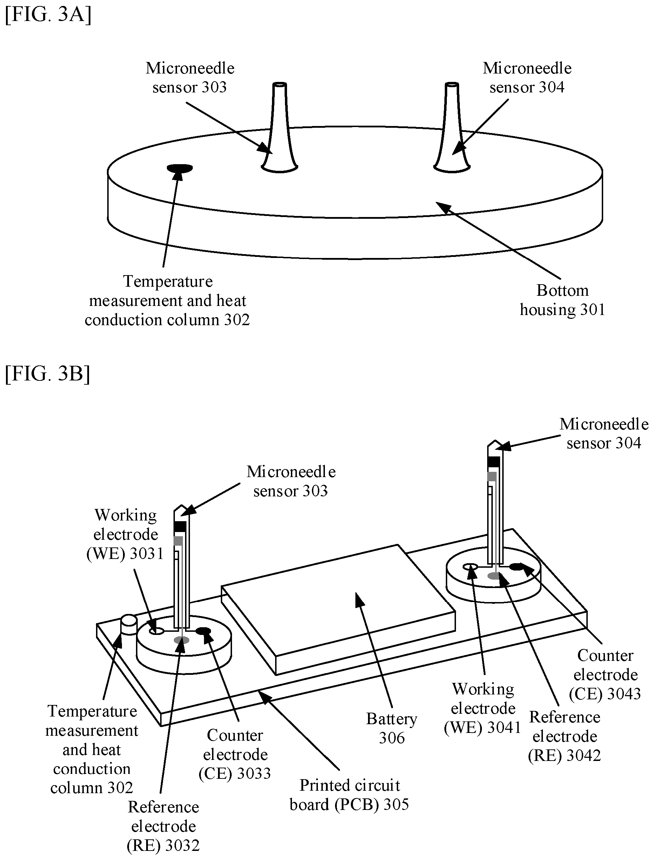

FIG. 3A is a diagram of a device form of an electronic device 200 according to an embodiment of this application.

As shown in FIG. 3A, the electronic device 200 may include a bottom housing 301, and a plurality of microneedle sensors, for example, a microneedle sensor 303 and a microneedle sensor 304. Optionally, the electronic device 200 may further include a temperature measurement and heat conduction column 302.

In some embodiments, the bottom housing 301 may be connected to the temperature measurement and heat conduction column 302, and the bottom housing 301 may be further connected to the plurality of microneedle sensors. In some embodiments, the temperature measurement and heat conduction column 302 and the plurality of microneedle sensors may be embedded in the bottom housing 301, or may be soldered to the bottom housing 301. A specific connection manner is not limited in this application.

The temperature measurement and heat conduction column 302 may be configured to measure a body temperature of a user, for example, measure a body surface temperature of the user or measure a temperature of a subcutaneous tissue of the user. In some embodiments, the temperature measurement and heat conduction column 302 may be in contact with the skin of the user, to measure the body surface temperature of the user. In some embodiments, the temperature measurement and heat conduction column 302 may alternatively be implanted into the subcutaneous tissue. In some other embodiments, the temperature measurement and heat conduction column 302 may alternatively be replaced with one or more temperature electrodes. The one or more temperature electrodes may alternatively be disposed in the plurality of microneedle sensors and implanted into the subcutaneous tissue.

The microneedle sensor may be implanted into the subcutaneous tissue of the user. One electrode group may be disposed in each microneedle sensor, and each electrode group may be configured to measure one or more physiological parameters. Each electrode group may include two or more electrochemical electrodes. In some embodiments, the electrode group may include one or more working electrodes and one or more counter electrodes (or reference electrodes). The two types of electrochemical electrodes may form one or more dual-electrode systems, and one or more physiological parameters may be measured by using the one or more dual-electrode systems. In some other embodiments, the electrode group may include one or more working electrodes, one or more counter electrodes, and one or more reference electrodes. The three types of electrochemical electrodes may form one or more three-electrode systems, and one or more physiological parameters may be measured by using the one or more three-electrode systems. In some other embodiments, optionally, one or more electrocardiography electrodes, for example, one or more of electrodes such as an LA electrode, an RA electrode, and an RLD electrode, may be separately disposed in the electrode group.

For example, the plurality of microneedle sensors may include the microneedle sensor 303 and the microneedle sensor 304. The microneedle sensor 303 may include an electrode group 1, and the microneedle sensor 304 may include an electrode group 2. The electrode group 1 may include at least one working electrode and at least one counter electrode. Optionally, the electrode group 1 may further include one or more reference electrodes. Further, optionally, the electrode group 1 may further include one or more electrocardiography electrodes. Similarly, the electrode group 2 may include at least one working electrode and at least one counter electrode. Optionally, the electrode group 2 may further include one or more reference electrodes. Further, optionally, the electrode group 2 may further include one or more electrocardiography electrodes. The electrode group 1 may be configured to measure a physiological parameter of the user, and the electrode group 2 may also be configured to measure the physiological parameter of the user. The physiological parameter may include but is not limited to any one or more of the following: blood glucose, blood ketone, uric acid, blood lactic acid, and the like. The electrode group 1 and the electrode group 2 may measure a same physiological parameter or different physiological parameters.

For example, the electrode group 1 in the microneedle sensor 303 may be configured to measure the blood glucose of the user, and the electrode group 2 in the microneedle sensor 304 may be configured to measure the blood ketone of the user. It may be understood that this embodiment herein is merely an example for describing the following: Each microneedle sensor may be configured to measure one or more physiological parameters. In this embodiment of this application, the microneedle sensor 303 and the microneedle sensor 304 may alternatively be configured to measure physiological parameters different from those in the foregoing embodiment. This is not limited in this application.

It should be noted that a distance between the microneedle sensor 303 and the microneedle sensor 304 is greater than or equal to a preset distance (for example, 3 centimeters). In this way, it can be ensured that an electrocardiography signal of the user can be obtained by using the two microneedle sensors.

It may be understood that the embodiment shown in FIG. 3A is merely an example. In embodiments of this application, the electronic device 200 may alternatively have a device form different from that in the foregoing embodiment, for example, include more microneedle sensors than those in the foregoing embodiment, or include more or fewer elements than those in the foregoing embodiment, or include elements different from those in the foregoing embodiment. This is not limited in this application.

FIG. 3B is a diagram of an internal structure of the electronic device 200 according to an embodiment of this application.

As shown in FIG. 3B, the electronic device 200 may include the microneedle sensor 303, the microneedle sensor 304, a printed circuit board (printed circuit board, PCB) 305, and a battery 306. Optionally, the electronic device 200 may further include a temperature measurement and heat conduction column 302.

The temperature measurement and heat conduction column 302 may be connected to the PCB 305, to transmit a measured temperature to the PCB 305. For other specific content of the temperature measurement and heat conduction column 302, refer to the related descriptions in the embodiment shown in FIG. 3A. Details are not described herein again.

The electrode group 1 may be disposed in the microneedle sensor 303. The electrode group 1 may include a plurality of electrochemical electrodes, for example, a working electrode (WE) 3031 and a counter electrode (CE) 3033. Optionally, the electrode group 1 may further include a reference electrode (RE) 3032. The electrode group 1 is configured to measure a physiological parameter 1 (for example, blood glucose, blood ketone, uric acid, or blood lactic acid). In the electrode group 1, the working electrode 3031 may react with a target substance 1 corresponding to the physiological parameter 1, to generate a reaction current. For example, if the physiological parameter 1 is blood glucose, the target substance 1 may be glucose. For another example, if the physiological parameter 1 is blood ketone, the target substance may be a ketone body. The reference electrode 3032 may be configured to control a voltage of the working electrode 3031. The counter electrode 3033 may be configured to form a loop with the working electrode 3031, to ensure generation and conduction of the reaction current. In some embodiments, the electrode group 1 may form a dual-electrode system by using the working electrode 3031 and the counter electrode 3033, and form a loop by using the dual-electrode system, to ensure generation and conduction of the reaction current. In some other embodiments, the electrode group 1 may form a three-electrode system by using the working electrode 3031, the counter electrode 3033, and the reference electrode 3032, and form a loop by using the three-electrode system, to ensure generation and conduction of the reaction current. The dual-electrode system and the three-electrode system may be used to measure the physiological parameter 1.

The electrode group 2 may be disposed in the microneedle sensor 304. The electrode group 2 may include a plurality of electrochemical electrodes, for example, a working electrode (WE) 3041 and a counter electrode (CE) 3043. Optionally, the electrode group 2 may further include a reference electrode (RE) 3042. The electrode group 2 is configured to measure a physiological parameter 2 (for example, blood glucose, blood ketone, uric acid, or blood lactic acid). It should be noted that the physiological parameter 1 may be different from the physiological parameter 2. In some embodiments, the physiological parameter 1 may alternatively be the same as the physiological parameter 2. For functions of the electrodes in the electrode group 2, refer to the functions of the electrodes in the electrode group 1. The electrode group 2 may also form a dual-electrode system or a three-electrode system based on types and a quantity of electrochemical electrodes, and measure the physiological parameter 2 by using the dual-electrode system or the three-electrode system.

The electrode group 1 and the electrode group 2 may be further configured to measure an electrocardiography signal of the user.

In some embodiments, the electrochemical electrode in the electrode group 1 and the electrochemical electrode in the electrode group 2 may be used as electrocardiography electrodes to measure the electrocardiography signal. Any electrode in the electrode group 1 and any electrode in the electrode group 2 may be respectively used as an LA electrode and an RA electrode. An RLD electrode may be an electrode in the electrode group 1 or an electrode in the electrode group 2, and the RLD electrode is connected to a right leg drive circuit. The right leg drive circuit is configured to cancel a common-mode signal. For example, the RE 3032 in the microneedle sensor 303 may be used as the LA electrode, the WE 3041 in the microneedle sensor 304 may be used as the RA electrode, and the CE 3043 may be used as the RLD electrode. It may be understood that this embodiment herein is merely an example. In embodiments of this application, another electrode in the electrode group 1 and another electrode in the electrode group 2 may alternatively be selected as the electrocardiography electrodes to measure the electrocardiography signal. This is not limited in this application. In the foregoing case, a part of electrochemical electrodes in the electrode group 1 and the electrode group 2 may measure both a physiological parameter of the user and the electrocardiography signal of the user.

In some other embodiments, separate electrocardiography electrodes may be disposed in the electrode group 1 and the electrode group 2, to measure the electrocardiography signal. The LA electrode and the RA electrode are respectively disposed in different electrode groups, and the RLD electrode may be disposed in any electrode group. In this case, the electrocardiography signal may be measured by using the electrocardiography electrode, and the electrocardiography electrode is only configured to measure the electrocardiography signal.

In some other embodiments, separate electrocardiography electrodes may be disposed in the electrode group 1 and the electrode group 2, and a part of electrochemical electrodes in the electrode group 1 and the electrode group 2 may also be used as electrocardiography electrodes. For example, the RLD electrode may be disposed in the electrode group 1, and one electrochemical electrode may be selected from each of the electrode group 1 and the electrode group 2 as the LA electrode and the RA electrode. In this way, the electrocardiography signal may alternatively be measured by using the RLD electrode and the plurality of electrochemical electrodes. It may be understood that this is merely an example for description. A part of electrodes in the electrocardiography electrodes may be the electrochemical electrodes in the electrode group 1 and the electrode group 2. In embodiments of this application, more or fewer electrocardiography electrodes than those in the foregoing embodiment, or electrocardiography electrodes different from those in the foregoing embodiment may be disposed, or another electrochemical electrode may be used as the electrocardiography electrode to measure the electrocardiography signal. This is not limited in this application.

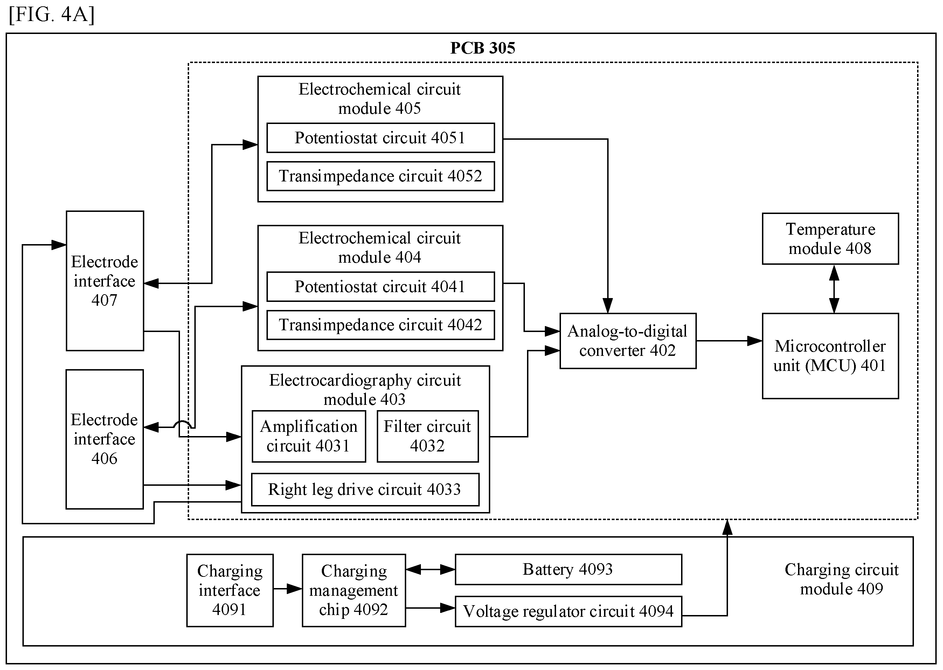

The PCB 305 may obtain the electrocardiography signal of the user through the microneedle sensor 303 and the microneedle sensor 304, and may further obtain physiological data 1 of the user through the microneedle sensor 303. The physiological data 1 is used to determine the physiological parameter 1. The PCB 305 may further obtain physiological data 2 of the user through the microneedle sensor 304. The physiological data 2 may be used to determine the physiological parameter 2. The PCB 305 may include a plurality of circuit modules. For internal circuit composition of the plurality of circuit modules and a connection relationship between the circuit modules, refer to related content in the following embodiments shown in FIG. 4A to FIG. 4J. Details are not described herein.

The battery 306 may be configured to supply power to a plurality of modules in the electronic device 200, for example, supply power to the PCB 305. In some embodiments, the battery 306 may be a lithium battery. In some other embodiments, the battery 306 may be a battery made of another material. This is not limited in this application.

It may be understood that the embodiment shown in FIG. 3B is merely an example. In some embodiments, the electronic device 200 may further include more microneedle sensors than those in the foregoing embodiment, and the microneedle sensor may also include more or fewer electrochemical electrodes than those in the foregoing embodiment. This is not limited in this application.

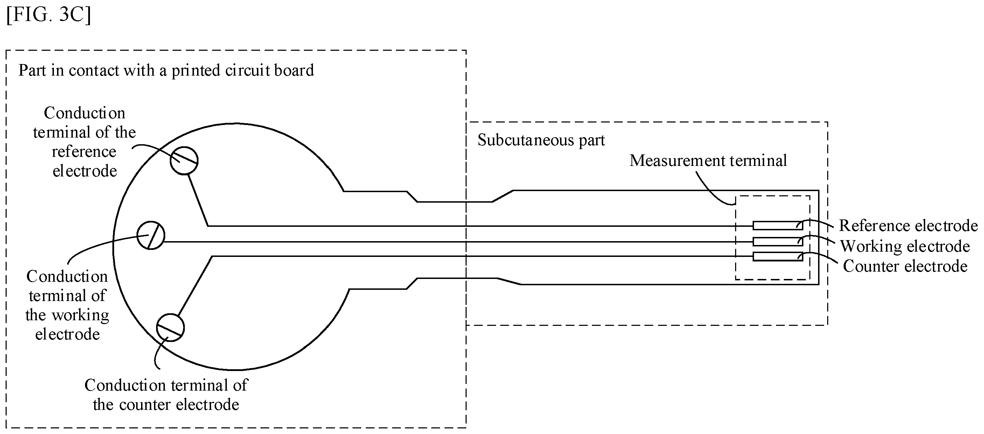

FIG. 3C is a diagram of distribution of electrodes in the microneedle sensor 303 according to an embodiment of this application.

As shown in FIG. 3C, the microneedle sensor 303 may include a supradermal part and a subcutaneous part. The subcutaneous part is configured to be implanted into a subcutaneous tissue of a user, and the supradermal part is configured to be connected to the PCB 305. For example, the microneedle sensor 303 may be a sheet of an irregular shape, and the sheet may include two opposite surfaces, namely, a surface A and a surface B.

The electrode group 1 may be disposed in the microneedle sensor 303. For specific descriptions of the electrode group 1, refer to related content in the embodiments shown in FIG. 3A and FIG. 3B. For example, the electrode group 1 may include the working electrode 3031, the reference electrode 3032, and the counter electrode 3033. In some embodiments, all electrochemical electrodes in the electrode group 1 may be disposed on the surface A of the microneedle sensor 303. Each electrochemical electrode may include a measurement terminal, a conduction terminal, and a conducting wire used to connect the measurement terminal and the conduction terminal. Measurement terminals of the one or more electrochemical electrodes are all disposed on a surface A of the subcutaneous part, and conduction terminals of the one or more electrochemical electrodes are all disposed on a surface A of the supradermal part.

It may be understood that the embodiment shown in FIG. 3A is merely an example for describing the following: All electrodes in the microneedle sensor 303 may be disposed on a same surface of the microneedle sensor 303. In some other embodiments, electrodes in the microneedle sensor 303 may alternatively be disposed on different surfaces of the microneedle sensor 303. For example, the working electrode 3031 is disposed on the surface A, and the reference electrode 3032 and the counter electrode 3033 are disposed on the surface B. This is not limited in this application. In addition, in some other embodiments, the microneedle sensor 303 may alternatively be in a form different from that in FIG. 3C, for example, a needle, a cylinder, a triangular prism, or a polyhedron. A specific form of the microneedle sensor 303 is not limited in this application.

In this way, when the subcutaneous part of the microneedle sensor 303 is implanted into the subcutaneous tissue of the user, the microneedle sensor 303 may obtain the physiological data 1 of the user by using the one or more electrochemical electrodes.

In some other embodiments, the microneedle sensor 303 may alternatively include a plurality of working electrodes. In a possible implementation, two or more working electrodes may share a same reference electrode and/or counter electrode, to form different three-electrode systems (or dual-electrode systems) to measure a same physiological parameter or different physiological parameters, for example, measure blood glucose and blood ketone. In another possible implementation, the microneedle sensor 303 may further include a plurality of reference electrodes and/or counter electrodes. The plurality of working electrodes may form different three-electrode systems (or dual-electrode systems) with different reference electrodes and/or counter electrodes, to measure a same physiological parameter or different physiological parameters.

It should be noted that, for distribution of electrodes in the microneedle sensor 304, refer to distribution of electrodes in the microneedle sensor 303 shown in FIG. 3C. In addition, if the electronic device 200 further includes another microneedle sensor, for distribution of electrodes in the microneedle sensor, refer to the related descriptions in the embodiment shown in FIG. 3C. Details are not described in this application again.

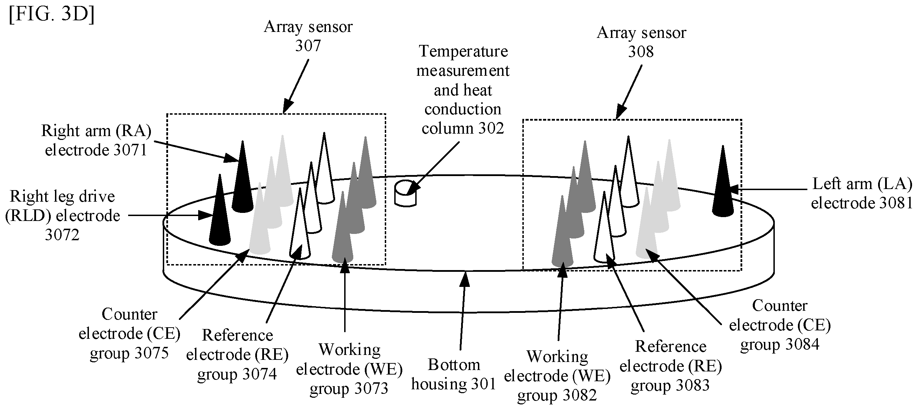

FIG. 3D is a diagram of a device form of another electronic device 200 according to an embodiment of this application.

As shown in FIG. 3D, the electronic device 200 may include a bottom housing 301 and a plurality of array sensors. Each array sensor may include one electrode group. For specific content of the electrode group, refer to the related descriptions in the embodiment shown in FIG. 3A. Details are not described herein again. Optionally, the electronic device 200 may further include a temperature measurement and heat conduction column 302. For specific content of the temperature measurement and heat conduction column 302, refer to the related descriptions in the embodiments shown in FIG. 3A and FIG. 3B. Details are not described herein again. In some embodiments, the plurality of array sensors may be disposed on the bottom housing 301. Optionally, the temperature measurement and heat conduction column 302 may be disposed on the bottom housing 301. In this embodiment of this application, the sensor may be an element configured to obtain a specific signal (for example, a current signal, a voltage signal, or an optical signal). In some embodiments, the array sensor may alternatively be a plurality of electrodes arranged in an array.

For example, the plurality of array sensors may include an array sensor 307 and an array sensor 308. The array sensor 307 may include an electrode group 1, and the array sensor 308 may include an electrode group 2.

The electrode group 1 may include a plurality of electrochemical electrodes, for example, a working electrode group 3073 and a counter electrode group 3075. Optionally, the electrode group 1 may further include a reference electrode group 3074. Further, optionally, the electrode group 1 may include one or more electrocardiography electrodes, for example, a right leg drive (RLD) electrode 3072 and a right arm (RA) electrode 3071. Each electrode group may include one or more electrochemical electrodes of a same type. In some embodiments, the working electrode group 3073 and the counter electrode group 3075 in the electrode group 1 may form one or more dual-electrode systems, and each dual-electrode system may form a loop, to ensure generation and conduction of a reaction current. The electrode group 1 may measure one or more physiological parameters by using the dual-electrode system. In some other embodiments, the working electrode group 3073, the reference electrode group 3074, and the counter electrode group 3075 in the electrode group 1 may form one or more three-electrode systems, and each three-electrode system may form a loop, to ensure generation and conduction of a reaction current. It should be noted that, in some embodiments, a plurality of dual-electrode systems (or three-electrode systems) may share a counter electrode and/or a reference electrode. The electrode group 1 may measure a physiological parameter 1 by using the dual-electrode system and/or the three-electrode system.

The electrode group 2 may include a plurality of electrochemical electrodes, for example, a working electrode group 3082 and a counter electrode group 3084. Optionally, the electrode group 2 may further include a reference electrode group 3083. Further, optionally, the electrode group 2 may include one or more electrocardiography electrodes, for example, a left arm (LA) electrode 3081. Each electrode group may include one or more electrochemical electrodes of a same type. In some embodiments, the working electrode group 3082 and the counter electrode group 3084 in the electrode group 2 may form one or more dual-electrode systems, and each dual-electrode system may form a loop, to ensure generation and conduction of a reaction current. The electrode group 2 may measure one or more physiological parameters by using the dual-electrode system. In some other embodiments, the working electrode group 3082, the reference electrode group 3083, and the counter electrode group 3084 in the electrode group 2 may form one or more three-electrode systems, and each three-electrode system may form a loop, to ensure generation and conduction of a reaction current. It should be noted that, in some embodiments, a plurality of dual-electrode systems (or three-electrode systems) may share a counter electrode and/or a reference electrode. The electrode group 2 may measure a physiological parameter 2 by using the dual-electrode system and/or the three-electrode system.

It may be understood that the foregoing embodiment is merely an example for describing the following: The electrode group 1 and the electrode group 2 each may include one or more electrocardiography electrodes. In embodiments of this application, it only needs to be ensured that the LA electrode and the RA electrode are located in different electrode groups, and the RLD electrode may be located in any electrode group. A specific correspondence between the electrocardiography electrode and the electrode group is not limited in this application.

In some other embodiments, separate electrocardiography electrodes, for example, the left arm (LA) electrode 3081, the right leg drive (RLD) electrode 3072, and the right arm (RA) electrode 3071, may be disposed in the electrode group 1 and the electrode group 2. In this case, the electrocardiography signal may be measured by using the electrocardiography electrode, and the electrocardiography electrode is only configured to measure the electrocardiography signal.

In some embodiments, the electrochemical electrode in the electrode group 1 and the electrochemical electrode in the electrode group 2 may be used as electrocardiography electrodes to measure the electrocardiography signal. Any electrode in the electrode group 1 and any electrode in the electrode group 2 may be respectively used as an LA electrode and an RA electrode. An RLD electrode may be an electrode in the electrode group 1 or an electrode in the electrode group 2, and the RLD electrode is connected to a right leg drive circuit. The right leg drive circuit is configured to cancel a common-mode signal. For example, one or more reference electrodes in the reference electrode group 3074 in the electrode group 1 may be used as the LA electrode, one or more working electrodes in the working electrode group 3082 in the electrode group 2 may be used as the RA electrode, and one or more counter electrodes in the counter electrode group 3084 may be used as the RLD electrode. It may be understood that this embodiment herein is merely an example. In embodiments of this application, another electrode in the electrode group 1 and another electrode in the electrode group 2 may alternatively be selected as the electrocardiography electrodes to measure the electrocardiography signal. This is not limited in this application. In the foregoing case, a part of electrochemical electrodes in the electrode group 1 and the electrode group 2 may measure both a physiological parameter of the user and the electrocardiography signal of the user.

In some other embodiments, separate electrocardiography electrodes may be disposed in the electrode group 1 and the electrode group 2, and a part of electrochemical electrodes in the electrode group 1 and the electrode group 2 may also be used as electrocardiography electrodes. For example, the RLD electrode may be disposed in the electrode group 1, and one electrochemical electrode may be selected from each of the electrode group 1 and the electrode group 2 as the LA electrode and the RA electrode. In this way, the electrocardiography signal may alternatively be measured by using the RLD electrode and the plurality of electrochemical electrodes. It may be understood that this is merely an example for description. A part of electrodes in the electrocardiography electrodes may be the electrochemical electrodes in the electrode group 1 and the electrode group 2. In embodiments of this application, another electrochemical electrode may be used as the electrocardiography electrode to measure the electrocardiography signal. This is not limited in this application.

For specific functions of the working electrode, the reference electrode, and the counter electrode in the array sensor 307 and the array sensor 308, refer to the function descriptions of the electrodes in the embodiment shown in FIG. 3B. Details are not described herein again.

It should be noted that a distance between the array sensor 307 and the array sensor 308 is greater than or equal to a preset distance (for example, 3 centimeters). In this way, it can be ensured that the electrocardiography signal of the user can be obtained by using the two array sensors.

It may be understood that the embodiment shown in FIG. 3D is merely an example. In embodiments of this application, the electronic device 200 may alternatively have a device form different from that in the foregoing embodiment, for example, include more array sensors than those in the foregoing embodiment, or include more or fewer electrodes (including the electrocardiography electrode and the electrochemical electrode) in the array sensor than those in the foregoing embodiment, or include electrodes different from those in the foregoing embodiment, or include more or fewer elements than those in the foregoing embodiment, or include elements different from those in the foregoing embodiment. This is not limited in this application.

It should be noted that the embodiments shown in FIG. 3A and FIG. 3D are merely two examples. In embodiments of this application, the electronic device 200 may alternatively include one or more array sensors and one or more microneedle sensors. The microneedle sensor may include a plurality of electrodes, and the array sensor may also include a plurality of electrodes. The electronic device 200 may determine a physiological parameter by using the array sensor, determine another physiological parameter by using the microneedle sensor, and obtain an electrocardiography signal by using the microneedle sensor and the array sensor. This is not limited in this application.

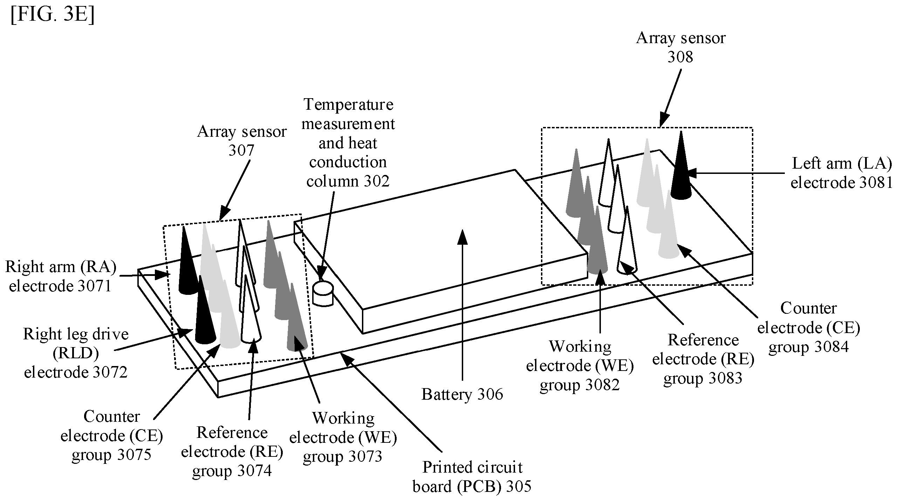

FIG. 3E is a diagram of an internal structure of the electronic device 200 according to an embodiment of this application.

As shown in FIG. 3E, the electronic device 200 may include the array sensor 307, the array sensor 308, a printed circuit board (printed circuit board, PCB) 305, and a battery 306. Optionally, the electronic device 200 may further include a temperature measurement and heat conduction column 302.

The temperature measurement and heat conduction column 302 may be connected to the PCB 305, to transmit a measured temperature to the PCB 305. For other specific content of the temperature measurement and heat conduction column 302, refer to the related descriptions in the embodiment shown in FIG. 3D. Details are not described herein again.

For composition of electrodes in the array sensor 307 (namely, composition of electrodes in the electrode group 1) and composition of electrodes in the array sensor 308 (namely, composition of electrodes in the electrode group 2), refer to the related descriptions in the embodiment shown in FIG. 3D. Details are not described herein again. It should be noted that the array sensor 307 and the array sensor 308 may be disposed on the PCB 305, or may be connected to the PCB.

In some embodiments, the PCB 305 may obtain the electrocardiography signal of the user through the array sensor 307 and the array sensor 308, and may further obtain physiological data 1 of the user through the array sensor 307. The physiological data 1 is used to determine the physiological parameter 1. The PCB 305 may further obtain physiological data 2 of the user through the array sensor 308. The physiological data 2 may be used to determine the physiological parameter 2. The PCB 305 may include a plurality of circuit modules. For internal circuit composition of the plurality of circuit modules and a connection relationship between the circuit modules, refer to related content in the following embodiments shown in FIG. 4A to FIG. 4J. Details are not described herein. It should be noted that the physiological parameter 1 may include one or more physiological parameters. In some embodiments, a plurality of working electrodes in the array sensor 307 may be configured to obtain physiological data of different physiological parameters. In this case, a plurality of different physiological parameters, for example, blood glucose and blood ketone, may be determined based on the physiological data 1. This is not limited in this application. It may be understood that the physiological parameter 2 may also include one or more physiological parameters.

The battery 306 may be configured to supply power to a plurality of modules in the electronic device 200, for example, supply power to the PCB 305. In some embodiments, the battery 306 may be a lithium battery. In some other embodiments, the battery 306 may be a battery made of another material. This is not limited in this application.

It may be understood that the embodiment shown in FIG. 3E is merely an example. In some embodiments, the electronic device 200 may further include more array sensors than those in the foregoing embodiment, and the array sensor 307 and/or the array sensor 308 may also include more or fewer electrodes (including an electrocardiography electrode and an electrochemical electrode) than those in the foregoing embodiment. This is not limited in this application.

The following describes a connection relationship between circuit modules in the PCB 305 according to embodiments of this application.

FIG 4A is a diagram of a connection relationship between circuit modules in a PCB 305 according to an embodiment of this application.

As shown in FIG. 4A, the PCB 305 may include a microcontroller unit (microcontroller unit, MCU) 401, an analog-to-digital converter (analog-to-digital converter, ADC) 402, an electrocardiography circuit module 403, one or more electrochemical circuit modules (for example, an electrochemical circuit module 404 and an electrochemical circuit module 405), one or more electrode interfaces (for example, an electrode interface 406 and an electrode interface 407), and the like. Optionally, the PCB 305 may further include a temperature module 408, and the like. In some embodiments, the ADC 402 may alternatively be integrated into the MCU 401.

The electrocardiography circuit module 403 may be connected to an electrocardiography electrode. When electrocardiography is measured by using a bipolar lead system, the electrocardiography electrode may include an LA electrode, an RA electrode, and an RLD electrode. The LA electrode and the RA electrode may measure an electrocardiography signal 1, and transmit the electrocardiography signal 1 to the electrocardiography circuit module 403. The electrocardiography circuit module 403 may perform amplification and filtering on the electrocardiography signal 1 to obtain an electrocardiography signal 2, and send the electrocardiography signal 2 to the ADC 402. In some embodiments, the electrocardiography circuit module 403 may include an amplification circuit 4031, a filter circuit 4032, and a right leg drive (RLD) circuit 4033. The amplification circuit 4031 may be connected to the LA electrode and the RA electrode, and the RLD circuit 4033 may be connected to the RLD electrode. The amplification circuit 4031 may receive the electrocardiography signal 1 and amplify the electrocardiography signal 1. The RLD circuit 4033 may cancel a common-mode signal. The filter circuit 4032 may perform filtering on an amplified electrocardiography signal 1 to obtain the electrocardiography signal 2.

The one or more electrochemical circuit modules may include the electrochemical circuit module 404 and the electrochemical circuit module 405. Each electrochemical circuit may measure a physiological parameter, for example, blood glucose, blood ketone, uric acid, or blood lactic acid. The electrochemical circuit module 404 and the electrochemical circuit module 405 may measure a same physiological parameter or different physiological parameters.

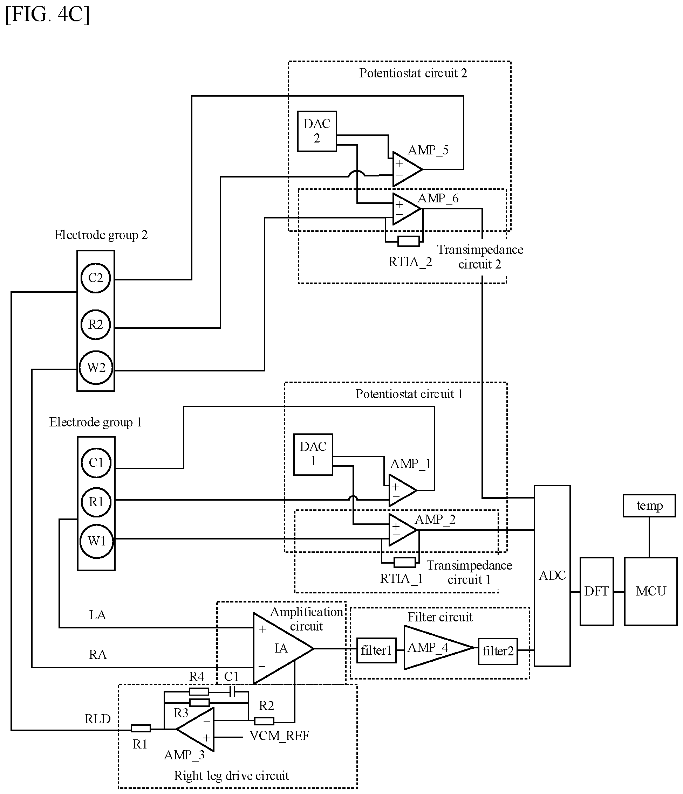

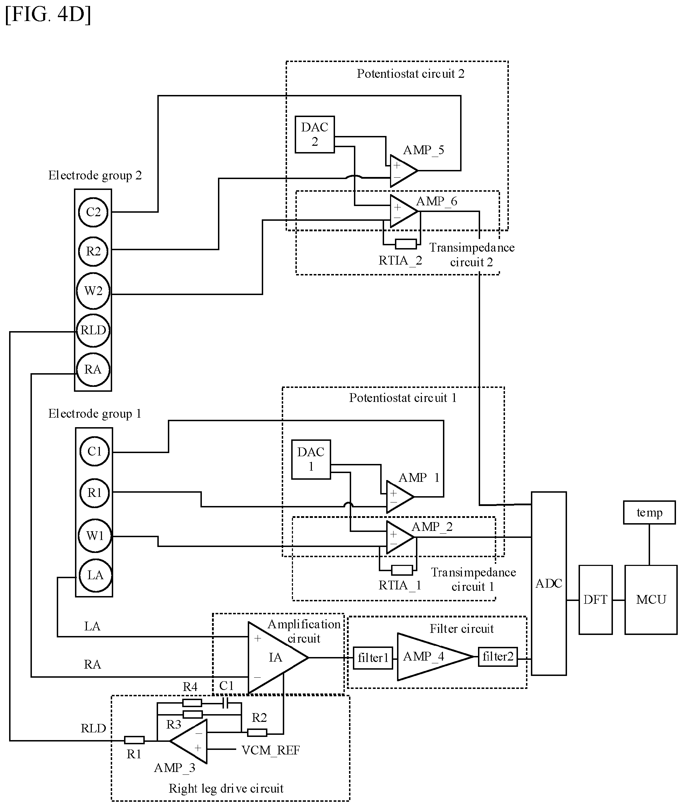

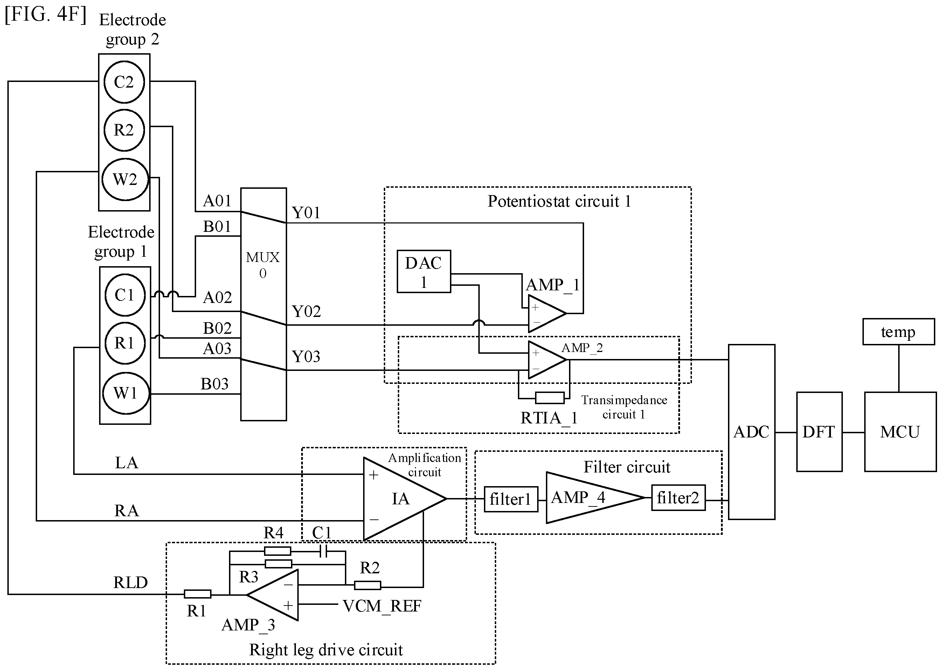

The electrochemical circuit module 404 may be connected to one or more electrochemical electrodes. When the physiological parameter is measured by using a three-electrode system, the electrochemical circuit module 404 may be connected to one or more working electrodes, one or more reference electrodes, and one or more counter electrodes. The one or more electrochemical electrodes may react with a target substance 1 to generate a current signal 1, and conduct the current signal 1 to the electrochemical circuit module 404. The electrochemical circuit module 404 may perform amplification or the like on the current signal 1 to obtain a current signal 2, and send the current signal 2 to the ADC 402. In some embodiments, the electrochemical circuit module 404 may include a potentiostat circuit 4041 and a transimpedance circuit 4042. The potentiostat circuit 4041 may control a voltage difference between the reference electrode and the working electrode. The transimpedance circuit 4042 may amplify the current signal 1 to obtain the current signal 2. In some embodiments, there may be an overlapping part between the potentiostat circuit 4041 and the transimpedance circuit 4042. For example, there is one or more elements shared by the potentiostat circuit 4041 and the transimpedance circuit 4042. For a specific example, refer to related content in the following embodiments shown in FIG. 4C, FIG. 4D, and FIG. 4F to FIG. 4J. Details are not described herein.

The electrochemical circuit module 405 may be connected to one or more electrochemical electrodes. When the physiological parameter is measured by using a three-electrode system, the electrochemical circuit module 405 may be connected to one or more working electrodes, one or more reference electrodes, and one or more counter electrodes. The one or more electrochemical electrodes may react with a target substance 2 to generate a current signal 3, and conduct the current signal 3 to the electrochemical circuit module 405. The electrochemical circuit module 405 may perform amplification or the like on the current signal 3 to obtain a current signal 4, and send the current signal 4 to the ADC 402. In some embodiments, the electrochemical circuit module 405 may include a potentiostat circuit 4051 and a transimpedance circuit 4052. The potentiostat circuit 4051 may control a voltage difference between the reference electrode and the working electrode. The transimpedance circuit 4052 may amplify the current signal 3 to obtain the current signal 4. In some embodiments, there may be an overlapping part between the potentiostat circuit 4051 and the transimpedance circuit 4052. For example, there is one or more elements shared by the potentiostat circuit 4051 and the transimpedance circuit 4052. For a specific example, refer to related content in the following embodiments shown in FIG. 4C, FIG. 4D, and FIG. 4F to FIG. 4J. Details are not described herein.

The one or more electrode interfaces may include the electrode interface 406 and the electrode interface 407. The electrode interface may be connected to the electrochemical circuit module and one or more electrochemical electrodes, and may be further connected to the electrocardiography circuit module 403 and the electrocardiography electrode.

In some embodiments, the electrode interface 406 may be connected to the electrochemical circuit module 404 and one or more electrochemical electrodes. The electrode interface 406 may be further connected to the electrocardiography circuit module 403 and the LA electrode. The electrode interface 407 may be connected to the electrochemical circuit module 405 and one or more electrochemical electrodes, and the electrode interface 407 may be further connected to the electrocardiography circuit module 403, the RA electrode, and the RLD electrode.

For example, if the electronic device 200 is the electronic device 200 shown in FIG. 3A and FIG. 3B, the electrochemical electrode connected to the electrode interface 406 may include the working electrode 3031, the reference electrode 3032, and the counter electrode 3033 in the embodiment shown in FIG. 3B, the LA electrode connected to the electrode interface 406 may be the reference electrode 3032, the electrochemical electrode connected to the electrode interface 407 may include the working electrode 3041, the reference electrode 3042, and the counter electrode 3043 in the embodiment shown in FIG. 3B, the RA electrode connected to the electrode interface 407 may be the working electrode 3041, and the RLD electrode may be the counter electrode 3043.

For another example, if the electronic device 200 is the electronic device 200 shown in FIG. 3D and FIG. 3E, the electrochemical electrode connected to the electrode interface 406 may include the working electrode group 3082, the reference electrode group 3083, and the counter electrode group 3084 in the embodiment shown in FIG. 3D, the LA electrode connected to the electrode interface 406 may be the LA electrode 3081, the electrochemical electrode connected to the electrode interface 407 may include the working electrode group 3073, the reference electrode group 3074, and the counter electrode group 3075 in the embodiment shown in FIG. 3D, the RA electrode connected to the electrode interface 407 may be the RA electrode 3071, and the RLD electrode may be the RLD electrode 3072. It may be understood that the foregoing two embodiments are merely examples. In embodiments of this application, a connection relationship between an electrode interface and an electrode (including an electrochemical electrode and an electrocardiography electrode) may be a connection relationship different from that in the foregoing embodiments. This is not limited in this application.

The ADC 402 may receive a current signal (for example, the current signal 2 or the current signal 4) sent by the one or more electrochemical circuit modules. The ADC 402 may further receive an analog electrocardiography signal 2 sent by the electrocardiography circuit module 403. The ADC 402 may perform analog-to-digital conversion on a received analog signal (for example, the current signal 2, the current signal 4, or the electrocardiography signal 2), to obtain a corresponding digital signal. For example, the ADC 402 may perform analog-to-digital conversion on the current signal 2 to obtain a current signal 5. The ADC 402 may perform analog-to-digital conversion on the current signal 4 to obtain a current signal 6. The ADC 402 may also perform analog-to-digital conversion on the electrocardiography signal 2 to obtain an electrocardiography signal 3. The current signal 5, the current signal 6, and the electrocardiography signal 3 are all digital signals.

The MCU 401 may receive the digital signals (for example, the current signal 5, the current signal 6, and the electrocardiography signal 3) sent by the ADC 402, and may further receive a temperature signal sent by the temperature module 408. The MCU 401 may determine an electrocardiogram of a user based on the electrocardiography signal 3 sent by the ADC 402. The MCU 401 may determine a physiological parameter 1 of the user based on the current signal 5 sent by the ADC 02, and the MCU 401 may also determine a physiological parameter 2 of the user based on the current signal 6. Optionally, the MCU 401 may further determine a body temperature of the user based on the temperature signal.

The temperature module 408 may detect the temperature signal, and send the temperature signal to the MCU 401.

In some embodiments, the PCB 305 may further include a charging circuit module 409. The charging circuit module 409 may supply power to another module in the PCB 305.

The charging circuit module 409 may include a charging management chip 4092 and a voltage regulator circuit 4094. Optionally, the charging circuit module 409 may further include a battery 4093 and a charging interface 4091. In some embodiments, the battery 4093 may not be disposed in the PCB 305, but is connected to the charging circuit module 409 in the PCB 305.

The charging interface 4091 may receive a charging input (for example, a charging input of a wired charger or a wireless charging input). The charging interface 4091 may charge the battery 4093 through the charging management chip 4092, or may transmit electric energy to the voltage regulator circuit 4094 through the charging management chip 4092.

The voltage regulator circuit 4094 may provide electric energy with a stable voltage for another circuit module in the PCB 305. The voltage regulator circuit 4094 may receive electric energy transmitted by the charging management chip 4092, or may receive electric energy transmitted by the battery 4093.

It may be understood that the embodiment shown in FIG. 4A is merely an example. In embodiments of this application, the PCB 305 may further include more or fewer circuit modules (for example, an electrochemical circuit module and an electrode interface) than those in the foregoing embodiment, or include modules different from those in the foregoing embodiment, and a connection relationship between the circuit modules in the PCB 305 may alternatively be a connection relationship different from that in the foregoing embodiment. This is not limited in this application.

In some embodiments, a same electrochemical circuit module in the PCB 305 may be configured to measure a plurality of physiological parameters, and the PCB 305 may control, by using one or more multiplexers, the physiological parameters measured by the electrochemical circuit module.

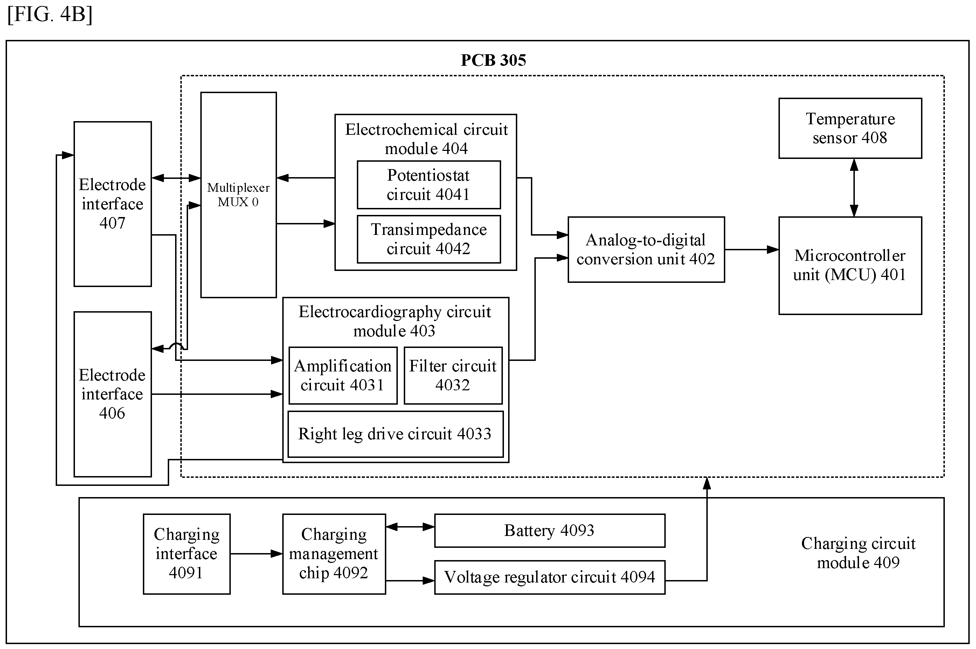

For example, FIG 4B is a diagram of a connection relationship between circuit modules in another PCB 305 according to an embodiment of this application.

As shown in FIG. 4B, the PCB 305 may include a microcontroller unit (microcontroller unit, MCU), an analog-to-digital converter (analog-to-digital converter, ADC) 402, an electrocardiography circuit module 403, a multiplexer MUX 0, one or more electrochemical circuit modules (for example, an electrochemical circuit module 404), one or more electrode interfaces (for example, an electrode interface 406 and an electrode interface 407), and the like. Optionally, the PCB 305 may further include but is not limited to any one or more of the following: a temperature module 408, a charging circuit module 409, and the like.

An input terminal of the multiplexer MUX 0 may be connected to the electrode interface 406 and the electrode interface 407. An output terminal of the multiplexer MUX 0 may be connected to the electrochemical circuit module 404. The multiplexer MUX 0 may be connected to the electrode interface 406 or the electrode interface 407.

The electrochemical circuit module 404 may be connected to a plurality of electrochemical electrodes through the multiplexer MUX 0.

When the MUX 0 is connected to the electrode interface 406, the electrochemical circuit module 404 may be connected to one or more electrochemical electrodes through the MUX 0 and the electrode interface 406. When a physiological parameter is measured by using a three-electrode system, the one or more electrochemical electrodes may include one or more working electrodes, one or more reference electrodes, and one or more counter electrodes. The one or more electrochemical electrodes may react with a target substance 1 to generate a current signal 1, and conduct the current signal 1 to the electrochemical circuit module 404. The electrochemical circuit module 404 may perform amplification or the like on the current signal 1 to obtain a current signal 2, and send the current signal 2 to the ADC 402.

When the MUX 0 is connected to the electrode interface 407, the electrochemical circuit module 404 may be connected to one or more electrochemical electrodes through the MUX 0 and the electrode interface 407. When a physiological parameter is measured by using a three-electrode system, the one or more electrochemical electrodes may include one or more working electrodes, one or more reference electrodes, and one or more counter electrodes. The one or more electrochemical electrodes may react with a target substance 2 to generate a current signal 3, and conduct the current signal 3 to the electrochemical circuit module 404. The electrochemical circuit module 404 may perform amplification or the like on the current signal 3 to obtain a current signal 4, and send the current signal 4 to the ADC 402.

In some embodiments, the electrochemical circuit module 404 may include a potentiostat circuit 4041 and a transimpedance circuit 4042. The potentiostat circuit 4041 may control a voltage difference between the reference electrode and the working electrode. The transimpedance circuit 4042 may amplify a current signal, for example, amplify the current signal 1 to obtain the current signal 2, or amplify the current signal 3 to obtain the current signal 4. In some embodiments, there may be an overlapping part between the potentiostat circuit 4041 and the transimpedance circuit 4042. For example, there is one or more elements shared by the potentiostat circuit 4041 and the transimpedance circuit 4042. For a specific example, refer to related content in the following embodiments shown in FIG. 4C, FIG. 4D, and FIG. 4F to FIG. 4J. Details are not described herein.

The one or more electrode interfaces may include the electrode interface 406 and the electrode interface 407. The electrode interface may be connected to the electrochemical circuit module and one or more electrochemical electrodes, and may be further connected to the electrocardiography circuit module 403 and the electrocardiography electrode. In some embodiments, the electrode interface 406 may be connected to the multiplexer MUX 0 and one or more electrochemical electrodes. The electrode interface 406 may be further connected to the electrocardiography circuit module 403 and the LA electrode. The electrode interface 407 may be connected to the multiplexer MUX 0 and one or more electrochemical electrodes, and the electrode interface 407 may be further connected to the electrocardiography circuit module 403, an RA electrode, and an RLD electrode. For a connection relationship between the electrode interface 406, the electrode interface 407, and each electrode, refer to the related descriptions in the embodiment shown in FIG. 4A. Details are not described herein again.

In addition, for specific content of the MCU 401, the ADC 402, the electrocardiography circuit module 403, the temperature module 408, and the charging circuit module 409, refer to the related descriptions in the embodiment shown in FIG. 4A. Details are not described herein again.

It may be understood that the embodiment shown in FIG. 4B is merely an example. In embodiments of this application, the PCB 305 may further include more or fewer circuit modules (for example, an electrochemical circuit module and an electrode interface) than those in the foregoing embodiment, or include modules different from those in the foregoing embodiment, and a connection relationship between the circuit modules in the PCB 305 may alternatively be a connection relationship different from that in the foregoing embodiment. This is not limited in this application. In addition, in some embodiments, a single electrochemical module may be further configured to measure two or more physiological parameters. This is not limited in this application either.

In some other embodiments, more or fewer multiplexers may be disposed in the PCB 305, or multiplexers may be disposed at positions different from those in the embodiment shown in FIG. 4B. These multiplexers may be configured to control the PCB 305 to measure a current signal of a specified physiological parameter/an electrocardiography signal at a same moment, or measure an electrocardiography signal and a current signal of one or more physiological parameters at a same moment.

The following uses an example in which both an electrode group 1 and an electrode group 2 can form a three-electrode system to describe a plurality of measurement circuits according to embodiments of this application.

FIG. 4C is a circuit diagram of a measurement circuit according to an embodiment of this application.

As shown in FIG. 4C, the measurement circuit may include a potentiostat circuit 1, a transimpedance circuit 1, a potentiostat circuit 2, a transimpedance circuit 2, a right leg drive circuit, an amplification circuit, a filter circuit, an analog-to-digital conversion module ADC, an MCU, and the like. Optionally, the measurement circuit may further include any one or more of the following: a discrete Fourier transform module DFT, a temperature module temp, and the like. In addition, the measurement circuit may further include a plurality of electrode groups, for example, an electrode group 1 and an electrode group 2. For example, the electrode group 1 may include a working electrode W1, a reference electrode R1, and a counter electrode C1; and the electrode group 2 may include a working electrode W2, a reference electrode R2, and a counter electrode C2.

The following separately describes elements in the circuit modules and a connection manner of the elements.

The potentiostat circuit 1 may include a digital-to-analog conversion module DAC 1, an operational amplifier AMP_1, and an operational amplifier AMP_2. Each operational amplifier may include a non-inverting input terminal, an inverting input terminal, and an output terminal. The DAC 1 may generate a stable voltage signal. The DAC 1 may be connected to a non-inverting input terminal of the operational amplifier AMP_1, and may be further connected to a non-inverting input terminal of the operational amplifier AMP_2, to provide a same voltage input for the operational amplifier AMP_1 and the operational amplifier AMP_2. An output terminal of the operational amplifier AMP_1 may be connected to the counter electrode C1 in the electrode group 1, and an inverting input terminal of the operational amplifier AMP_1 may be connected to the reference electrode R1 in the electrode group 1. An inverting input terminal of the operational amplifier AMP_2 may be connected to the working electrode W1 in the electrode group 1, and an output terminal of the operational amplifier AMP_2 may be connected to the ADC. In this way, a same voltage is applied to the non-inverting input terminal of the operational amplifier AMP_1 and the non-inverting input terminal of the operational amplifier AMP_2, so that a voltage difference between the reference electrode R1 and the working electrode W1 can be controlled, and a voltage on the working electrode W1 is approximately equal to a voltage on the reference electrode R1.

The transimpedance circuit 1 may include the operational amplifier AMP_2 and a resistor RTIA_1. Two terminals of the resistor RTIA_1 are respectively connected to the inverting input terminal and the output terminal of the operational amplifier AMP_2. In this way, when a stable voltage is input to the non-inverting input terminal of the operational amplifier AMP_2, the transimpedance circuit 1 may amplify a current signal input to the inverting input terminal of the operational amplifier AMP_2. Because the inverting input terminal of the operational amplifier AMP_2 is connected to the working electrode W1 in the electrode group 1, and the output terminal of the operational amplifier AMP_2 is connected to the ADC, the transimpedance circuit 1 may amplify a current signal conducted by the working electrode W1, and transmit an amplified current signal to the ADC.

The potentiostat circuit 2 may include a digital-to-analog conversion module DAC 2, an operational amplifier AMP_5, and an operational amplifier AMP_6. The DAC 2 may generate a stable voltage signal. The DAC 2 may be connected to a non-inverting input terminal of the operational amplifier AMP_5, and may be further connected to a non-inverting input terminal of the operational amplifier AMP_6, to provide a same voltage input for the operational amplifier AMP_5 and the operational amplifier AMP_6. An output terminal of the operational amplifier AMP_5 may be connected to the counter electrode C2 in the electrode group 2, and an inverting input terminal of the operational amplifier AMP_5 may be connected to the reference electrode R2 in the electrode group 2. An inverting input terminal of the operational amplifier AMP_6 may be connected to the working electrode W2 in the electrode group 2, and an output terminal of the operational amplifier AMP_6 may be connected to the ADC. In this way, a same voltage is applied to the non-inverting input terminal of the operational amplifier AMP_5 and the non-inverting input terminal of the operational amplifier AMP_6, so that a voltage difference between the reference electrode R2 and the working electrode W2 can be controlled, and a voltage on the working electrode W2 is approximately equal to a voltage on the reference electrode R2. It should be noted that, in some embodiments, the potentiostat circuit 2 may alternatively share a digital-to-analog conversion module DAC 1 with the potentiostat circuit 1. In this case, the digital-to-analog conversion module DAC 2 in the potentiostat circuit 2 may alternatively be replaced with the digital-to-analog conversion module DAC 1. This is not limited in this application.

The transimpedance circuit 2 may include the operational amplifier AMP_6 and a resistor RTIA_2. Two terminals of the resistor RTIA_2 are respectively connected to the inverting input terminal and the output terminal of the operational amplifier AMP_6. In this way, when a stable voltage is input to the non-inverting input terminal of the operational amplifier AMP_6, the transimpedance circuit 2 may amplify a current signal input to the inverting input terminal of the operational amplifier AMP_6. Because the inverting input terminal of the operational amplifier AMP_6 is connected to the working electrode W2 in the electrode group 2, and the output terminal of the operational amplifier AMP_6 is connected to the ADC, the transimpedance circuit 2 may amplify a current signal conducted by the working electrode W2, and transmit an amplified current signal to the ADC.

An amplification module may include an instrument amplifier IA, and the instrument amplifier IA may be an element integrated with a plurality of operational amplifiers. The instrument amplifier IA may include a non-inverting input terminal, an inverting input terminal, and an output terminal. The instrument amplifier IA may perform a differential operation on signals at the non-inverting input terminal and the inverting input terminal, to amplify a differential-mode signal between the non-inverting input terminal and the inverting input terminal. In addition, the instrument amplifier IA only follows a common-mode signal, thereby increasing a ratio of an amplitude of the differential-mode signal to an amplitude of the common-mode signal, and suppressing the common-mode signal. The non-inverting input terminal of the instrument amplifier IA may be connected to the reference electrode R1 in the electrode group 1, and the inverting input terminal may be connected to the working electrode W2 in the electrode group 2. In this case, the reference electrode R1 may be considered as an LA electrode, and the working electrode W2 may be considered as an RA electrode. In this way, the instrument amplifier IA may amplify a potential difference between the LA electrode and the RA electrode, and transmit an amplified electrocardiography signal to the filter circuit.

In some embodiments, the right leg drive circuit may include an operational amplifier AMP_3, a plurality of resistors, and a capacitor C1. The plurality of resistors may include a resistor R1, a resistor R2, a resistor R3, and a resistor R4. A non-inverting input terminal of the operational amplifier AMP_3 may be connected to a voltage source VCM_REF, and the voltage source VCM_REF may apply a stable voltage to the operational amplifier AMP_3. An inverting input terminal of the operational amplifier AMP_3 may be connected to the instrument amplifier IA through the resistor R2, to receive a common-mode signal output by the instrument amplifier IA. In addition, an output terminal of the operational amplifier AMP_3 may be connected to the counter electrode C2 in the electrode group 2 through the resistor R1. In this case, the counter electrode C2 may be considered as an RLD electrode. Two terminals of the resistor R3 may be respectively connected to the output terminal and the inverting input terminal of the operational amplifier AMP_3. Two terminals of the resistor R4 are respectively connected to the capacitor C1 and the output terminal of the operational amplifier AMP_3. Two terminals of the capacitor C1 are respectively connected to the resistor R4 and the inverting input terminal of the operational amplifier AMP_3. That is, the resistor R4 and the capacitor C1 are connected in series, and a branch formed by connecting the resistor R4 and the capacitor C1 in series is connected in parallel to the resistor R3. The right leg drive circuit may apply a phase-inverted signal of the common-mode signal by using the RLD electrode, to cancel the common-mode signal.

The filter circuit may include a plurality of filters, for example, a filter filter1 and a filter filter2. Optionally, the filter circuit may further include an operational amplifier AMP_4. In some embodiments, the filter filter1 may be connected to an output terminal of the instrument amplifier IA and an input terminal of the operational amplifier AMP_4, and the filter filter2 may be connected to an output terminal of the operational amplifier AMP_4 and an input terminal of the ADC. The filter circuit may receive the amplified electrocardiography signal sent by the amplification circuit, perform one or more operations such as filtering and amplification on the amplified electrocardiography signal, and send a processed electrocardiography signal to the ADC.

The DFT module is an optional module. The DFT module may perform DFT processing on a digital signal transmitted by the ADC, and then transmit a processed digital signal to the MCU.

For specific functions of the ADC, the MCU, and the temperature module temp, refer to the related descriptions in the embodiment shown in FIG. 4A or FIG. 4B. Details are not described herein again.

It should be noted that, in the embodiment shown in FIG. 4C, the electrode group 1 and the electrode group 2 may be the electrode group 1 and the electrode group 2 in the embodiment shown in FIG. 3A and FIG. 3B or FIG. 3D and FIG. 3E.

It may be understood that the embodiment shown in FIG. 4C is merely an example. In embodiments of this application, the measurement circuit may further include more electrode groups, potentiostat circuits, and transimpedance circuits, to measure more physiological parameters. This is not limited in this application. In addition, each circuit module of the measurement circuit may further include more or fewer elements than those in the foregoing embodiment, or include elements different from those in the foregoing embodiment. This is not limited in this application.

FIG. 4D is a circuit diagram of another measurement circuit according to an embodiment of this application.

As shown in FIG. 4D, the measurement circuit may include a potentiostat circuit 1, a transimpedance circuit 1, a potentiostat circuit 2, a transimpedance circuit 2, a right leg drive circuit, an amplification circuit, a filter circuit, an analog-to-digital conversion module ADC, an MCU, and the like. Optionally, the measurement circuit may further include any one or more of the following: a discrete Fourier transform module DFT, a temperature module temp, and the like. In addition, the measurement circuit may further include a plurality of electrode groups, for example, an electrode group 1 and an electrode group 2. The electrode group 1 may include one or more working electrodes W1, one or more reference electrodes R1, and one or more counter electrodes C1. In addition, the electrode group 1 may further include an LA electrode. The electrode group 2 may include one or more working electrodes W2, one or more reference electrodes R2, and one or more counter electrodes C2. In addition, the electrode group 2 may further include an RA electrode and an RLD electrode.

The following separately describes connection relationships between electrodes in the electrode group 1 and the electrode group 2 and other circuit elements.

In the electrode group 1, the one or more counter electrodes C1 may be connected to an output terminal of an operational amplifier AMP _1; the one or more reference electrodes R1 may be connected to an inverting input terminal of the operational amplifier AMP_1; the one or more working electrodes W1 may be connected to an inverting input terminal of an operational amplifier AMP_2; and the LA electrode may be connected to a non-inverting input terminal of an instrument amplifier IA. The operational amplifier AMP_1 and the operational amplifier AMP_2 belong to the potentiostat circuit 1, and the instrument amplifier IA belongs to the amplification circuit.

In the electrode group 2, the one or more counter electrodes C2 may be connected to an output terminal of an operational amplifier AMP_5; the one or more reference electrodes R2 may be connected to an inverting input terminal of the operational amplifier AMP_5; the one or more working electrodes W2 may be connected to an inverting input terminal of an operational amplifier AMP_6; the RA electrode may be connected to an inverting input terminal of the instrument amplifier IA; and the RLD electrode may be connected to an output terminal of an operational amplifier AMP_3 through a resistor R1. The operational amplifier AMP_5 and the operational amplifier AMP_6 belong to the potentiostat circuit 2, the instrument amplifier IA belongs to the amplification circuit, and the operational amplifier AMP_3 and the resistor R1 belong to the right leg drive circuit.

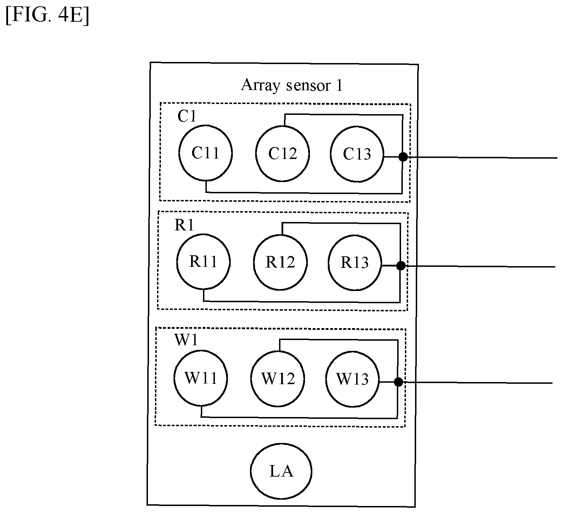

It should be noted that in the electrode group 1 or the electrode group 2, the one or more electrodes with a same function may be connected to an external element in parallel. For a specific connection manner, refer to related content in the embodiment shown in FIG. 4E below. Details are not described herein.

In addition, for specific content of the potentiostat circuit 1, the transimpedance circuit 1, the potentiostat circuit 2, the transimpedance circuit 2, the right leg drive circuit, the amplification circuit, the filter circuit, the MCU, the ADC, the DFT, and the temperature module temp, refer to related content in the embodiment shown in FIG. 4C. Details are not described herein again.

In the embodiment shown in FIG. 4D, the electrode group 1 and the electrode group 2 may be the electrode group 1 and the electrode group 2 in the embodiment shown in FIG. 3A and FIG. 3B or FIG. 3D and FIG. 3E.

It may be understood that the embodiment shown in FIG. 4D is merely an example. In embodiments of this application, the measurement circuit may further include more electrode groups, potentiostat circuits, and transimpedance circuits, to measure more physiological parameters. This is not limited in this application. In addition, each circuit module of the measurement circuit may further include more or fewer elements than those in the foregoing embodiment, or include elements different from those in the foregoing embodiment. This is not limited in this application.

The measurement circuit shown in FIG. 4C or FIG. 4D may be used to simultaneously measure a plurality of physiological parameters and an electrocardiography signal of a user.

FIG. 4E is a diagram of a manner of connecting one or more electrodes with a same function in an array sensor 1 to another element in a circuit according to an embodiment of this application.

As shown in FIG. 4E, the array sensor 1 may include one or more working electrodes W1, one or more reference electrodes R1, one or more counter electrodes C1, and an LA electrode. The one or more working electrodes W1 may include a working electrode W11, a working electrode W12, and a working electrode W13; the one or more reference electrodes R1 may include a reference electrode R11, a reference electrode R12, and a reference electrode R13; and the one or more counter electrodes C1 may include a counter electrode C11, a counter electrode C12, and a counter electrode C13.

For example, the array sensor 1 includes the electrode group 1 shown in FIG. 4D. The working electrode W11, the working electrode W12, and the working electrode W13 may be connected in parallel to a same port, for example, the inverting input terminal of the operational amplifier AMP_2 in the embodiment shown in FIG. 4D. The reference electrode R11, the reference electrode R12, and the reference electrode R13 may be connected in parallel to a same port, for example, the inverting input terminal of the operational amplifier AMP_1 in the embodiment shown in FIG. 4D. The counter electrode C11, the counter electrode C12, and the counter electrode C13 may be connected in parallel to a same port, for example, the output terminal of the operational amplifier AMP_1 shown in FIG. 4D.

It may be understood that the embodiment shown in FIG. 4E is merely an example for describing the following: Electrodes with a same function in the array sensor may be connected in parallel to a same port of a same element in the measurement circuit. In embodiments of this application, a quantity of electrodes with different functions in the array sensor 1 may be different from that in the foregoing embodiment, and another array sensor or microneedle sensor (for example, a microneedle sensor having a plurality of electrochemical electrodes of a same type) may be connected to an element in the measurement circuit in the manner shown in the foregoing embodiment. This is not limited in this application.

In a possible implementation, a plurality of electrode groups in the measurement circuit may share one electrochemical circuit module (including a potentiostat circuit and a transimpedance circuit). In this case, the measurement circuit may further include a multiplexer, and the multiplexer may be configured to control the electrochemical circuit module to be connected to the electrode group 1 or the electrode group 2.

FIG. 4F is a circuit diagram of another measurement circuit according to an embodiment of this application.

As shown in FIG. 4F, the measurement circuit may include a multiplexer MUX 0, a potentiostat circuit 1, a transimpedance circuit 1, a right leg drive circuit, an amplification circuit, a filter circuit, an analog-to-digital conversion module ADC, an MCU, and the like. Optionally, the measurement circuit may further include any one or more of the following: a discrete Fourier transform module DFT, a temperature module temp, and the like. In addition, the measurement circuit may further include a plurality of electrode groups, for example, an electrode group 1 and an electrode group 2. The electrode group 1 may include a working electrode W1, a reference electrode R1, and a counter electrode C1; and the electrode group 2 may include a working electrode W2, a reference electrode R2, and a counter electrode C2.

The multiplexer MUX 0 may include a plurality of input terminals, for example, an input terminal A01, an input terminal A02, an input terminal A03, an input terminal B01, an input terminal B02, and an input terminal B03. The multiplexer MUX 0 may further include a plurality of output terminals, for example, an output terminal Y01, an output terminal Y02, and an output terminal Y03. Each output terminal of the multiplexer MUX 0 may correspond to a plurality of input terminals. The output terminal Y01 may correspond to the input terminal A01 and the input terminal B01, the output terminal Y02 may correspond to the input terminal A02 and the input terminal B02, and the output terminal Y03 may correspond to the input terminal A03 and the input terminal B03. The MUX 0 may control the output terminal to be connected to one of the corresponding input terminals, for example, control the output terminal Y01 to be connected to the input terminal A01 or the input terminal B01, control the output terminal Y02 to be connected to the input terminal A02 or the input terminal B02, or control the output terminal Y03 to be connected to the input terminal A03 or the input terminal B03.

The output terminal Y01 may be connected to an output terminal of an operational amplifier AMP_1, the output terminal Y02 may be connected to an inverting input terminal of the operational amplifier AMP_1, and the output terminal Y03 may be connected to an inverting input terminal of an operational amplifier AMP_2. The operational amplifier AMP_1 and the operational amplifier AMP_2 belong to the potentiostat circuit 1.

The input terminal A01 may be connected to the counter electrode C2, the input terminal A02 may be connected to the reference electrode R2, and the input terminal A03 may be connected to the working electrode W2. The input terminal B01 may be connected to the counter electrode C1, the input terminal B02 may be connected to the reference electrode R1, and the input terminal B03 may be connected to the working electrode W1.

When the multiplexer MUX 0 selects to connect the input terminal A01, the input terminal A02, and the input terminal A03, the potentiostat circuit 1 and the transimpedance circuit 1 may be connected to an electrochemical electrode in the electrode group 2 through the multiplexer MUX 0. When the multiplexer MUX 0 selects to connect the input terminal B01, the input terminal B02, and the input terminal B03, the potentiostat circuit 1 and the transimpedance circuit 1 may be connected to an electrochemical electrode in the electrode group 1 through the multiplexer MUX 0.

In addition, in the electrode group 2, the counter electrode C2 may be further used as an RLD electrode to be connected to an output terminal of an operational amplifier AMP_3 through a resistor R1, and the working electrode W2 may be further used as an RA electrode to be connected to an inverting input terminal of an instrument amplifier IA. In the electrode group 1, the reference electrode R1 may be further used as an LA electrode to be connected to a non-inverting input terminal of the instrument amplifier IA. The instrument amplifier IA belongs to the amplification circuit, and the resistor R1 and the operational amplifier AMP_3 belong to the right leg drive circuit.

In addition, for composition and connection manners of other elements in circuits such as the potentiostat circuit 1, the transimpedance circuit 1, a potentiostat circuit 2, a transimpedance circuit 2, the right leg drive circuit, the amplification circuit, and the filter circuit, refer to the related descriptions in the embodiment shown in FIG. 4C. Details are not described herein again. In addition, for specific content of the MCU, the ADC, the DFT, and the temperature module temp, refer to related content in the embodiment shown in FIG. 4C. Details are not described herein again.

It may be understood that the embodiment shown in FIG. 4F is merely an example. In embodiments of this application, the measurement circuit may further include more electrode groups, potentiostat circuits, and transimpedance circuits, and the multiplexer may alternatively include more input terminals and output terminals, or more multiplexers may be included, to control measurement of more physiological parameters. This is not limited in this application. In addition, each circuit module of the measurement circuit may further include more or fewer elements than those in the foregoing embodiment, or include elements different from those in the foregoing embodiment. This is not limited in this application.

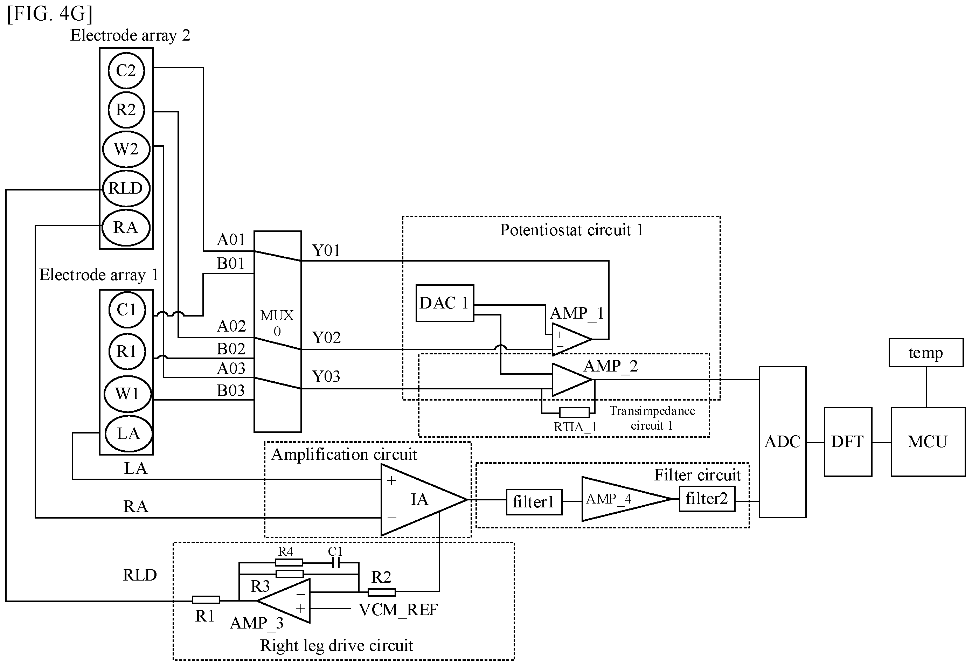

FIG. 4G is a circuit diagram of another measurement circuit according to an embodiment of this application.

As shown in FIG. 4G, the measurement circuit may include a multiplexer MUX 0, a potentiostat circuit 1, a transimpedance circuit 1, a right leg drive circuit, an amplification circuit, a filter circuit, an analog-to-digital conversion module ADC, an MCU, and the like. Optionally, the measurement circuit may further include any one or more of the following: a discrete Fourier transform module DFT, a temperature module temp, and the like. In addition, the measurement circuit may further include a plurality of electrode groups, for example, an electrode group 1 and an electrode group 2. The electrode group 1 may include one or more working electrodes W1, one or more reference electrodes R1, and one or more counter electrodes C1, and the electrode group 1 may further include an LA electrode. The electrode group 2 may include one or more working electrodes W2, one or more reference electrodes R2, and one or more counter electrodes C2, and the electrode group 2 may further include an RA electrode and an RLD electrode.

The multiplexer MUX 0 may include a plurality of input terminals and output terminals, and each output terminal may correspond to a plurality of input terminals. For a correspondence between an input terminal and an output terminal in the multiplexer MUX 0, and a connection relationship between the input terminal, the output terminal, and another element or electrode, refer to the related descriptions in the embodiment shown in FIG. 4F. Details are not described herein again. It should be noted that, for a manner of connecting each electrochemical electrode in the electrode group 1 and the electrode group 2 to the multiplexer MUX 0, refer to related content in the embodiments shown in FIG. 4E and FIG. 4F.

When the multiplexer MUX 0 selects to connect the input terminal A01, the input terminal A02, and the input terminal A03, the potentiostat circuit 1 and the transimpedance circuit 1 may be connected to an electrochemical electrode in the electrode group 2 through the multiplexer MUX 0. When the multiplexer MUX 0 selects to connect the input terminal B01, the input terminal B02, and the input terminal B03, the potentiostat circuit 1 and the transimpedance circuit 1 may be connected to an electrochemical electrode in the electrode group 1 through the multiplexer MUX 0.

In addition, in the electrode group 2, an RLD electrode is connected to an output terminal of an operational amplifier AMP_3 through a resistor R1, and an RA electrode may be connected to an inverting input terminal of an instrument amplifier IA. In the electrode group 1, an LA electrode may be connected to a non-inverting input terminal of the instrument amplifier IA. The instrument amplifier IA belongs to the amplification circuit, and the resistor R1 and the operational amplifier AMP_3 belong to the right leg drive circuit.

In addition, for composition and connection manners of other elements in circuits such as the potentiostat circuit 1, the transimpedance circuit 1, a potentiostat circuit 2, a transimpedance circuit 2, the right leg drive circuit, the amplification circuit, and the filter circuit, refer to the related descriptions in the embodiment shown in FIG. 4C. Details are not described herein again. In addition, for specific content of the MCU, the ADC, the DFT, and the temperature module temp, refer to related content in the embodiment shown in FIG. 4C. Details are not described herein again.

It may be understood that the embodiment shown in FIG. 4G is merely an example. In embodiments of this application, the measurement circuit may further include more electrode groups, potentiostat circuits, and transimpedance circuits, and the multiplexer may alternatively include more input terminals and output terminals, or more multiplexers may be included, to control measurement of more physiological parameters. This is not limited in this application. In addition, each circuit module of the measurement circuit may further include more or fewer elements than those in the foregoing embodiment, or include elements different from those in the foregoing embodiment. This is not limited in this application.

The measurement circuit shown in FIG. 4F or FIG. 4G may be used to measure an electrocardiography signal while measuring another physiological signal by controlling a multiplexer.

In another possible implementation, an electrochemical circuit module and an electrocardiography circuit module in the measurement circuit may share one or more elements (for example, an operational amplifier and a filter). The measurement circuit may further include a plurality of multiplexers, and the multiplexers may be configured to control the one or more elements to measure the electrocardiography signal or another physiological parameter.

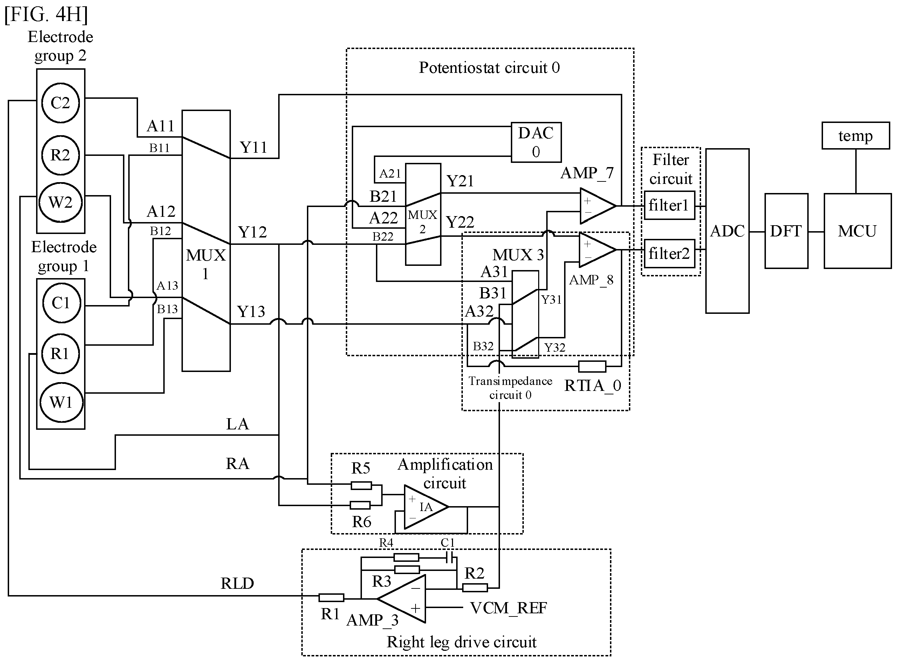

FIG. 4H is a circuit diagram of a measurement circuit according to an embodiment of this application.

As shown in FIG. 4H, the measurement circuit may include a plurality of multiplexers, a potentiostat circuit 0, a transimpedance circuit 0, a right leg drive circuit, an amplification circuit, a filter circuit, an analog-to-digital conversion module ADC, an MCU, and the like. Optionally, the measurement circuit may further include any one or more of the following: a discrete Fourier transform module DFT, a temperature module temp, and the like. In addition, the measurement circuit may further include a plurality of electrode groups, for example, an electrode group 1 and an electrode group 2. The electrode group 1 may include a working electrode W1, a reference electrode R1, and a counter electrode C1; and the electrode group 2 may include a working electrode W2, a reference electrode R2, and a counter electrode C2.

The plurality of multiplexers may include a multiplexer MUX 1, a multiplexer MUX 2, and a multiplexer MUX 3. The multiplexer MUX 1 may be configured to control a microneedle sensor connected to the potentiostat circuit 0 and the transimpedance circuit 0. The multiplexer MUX 2 and the multiplexer MUX 3 may be configured to control the measurement circuit to measure an electrocardiography signal or measure another physiological parameter.

The following separately describes ports of the plurality of multiplexers and connection manners of the ports.

The multiplexer MUX 1 may include a plurality of input terminals, for example, an input terminal A11, an input terminal A12, an input terminal A13, an input terminal B11, an input terminal B12, and an input terminal B13. The multiplexer MUX 1 may further include a plurality of output terminals, for example, an output terminal Y11, an output terminal Y12, and an output terminal Y13. Each output terminal of the multiplexer MUX 1 may correspond to a plurality of input terminals, and the MUX 1 may control an output terminal to be connected to one of the input terminals corresponding to the output terminal. The output terminal Y11 may correspond to the input terminal A11 and the input terminal B11, the output terminal Y12 may correspond to the input terminal A12 and the input terminal B12, and the output terminal Y13 may correspond to the input terminal A13 and the input terminal B13.

The output terminal Y11 may be connected to an output terminal of an operational amplifier AMP_7, the output terminal Y12 may be connected to an input terminal B22 of the multiplexer MUX 2, the output terminal Y12 may be further connected to an input terminal A31 of the multiplexer MUX 3, the output terminal Y12 may be further connected to a non-inverting input terminal of an instrument amplifier IA through a resistor R6, the output terminal Y12 may be further connected to an LA electrode (for example, the reference electrode R1 in the electrode group 1), and the output terminal Y13 may be connected to an input terminal A32 of the multiplexer MUX 3.

The input terminal A11 may be connected to the counter electrode C2, the input terminal A12 may be connected to the reference electrode R2, and the input terminal A13 may be connected to the working electrode W2. The input terminal B11 may be connected to the counter electrode C1, the input terminal B12 may be connected to the reference electrode R1, and the input terminal B13 may be connected to the working electrode W1.

When the multiplexer MUX 1 selects to connect the input terminal A11, the input terminal A12, and the input terminal A13, the potentiostat circuit 0 and the transimpedance circuit 0 may be connected to an electrochemical electrode in the electrode group 2 through the multiplexer MUX 1. When the multiplexer MUX 1 selects to connect the input terminal B11, the input terminal B12, and the input terminal B13, the potentiostat circuit 0 and the transimpedance circuit 0 may be connected to an electrochemical electrode in the electrode group 1 through the multiplexer MUX 1.

The multiplexer MUX 2 may include a plurality of input terminals, for example, an input terminal A21, an input terminal A22, an input terminal B21, and an input terminal B22. The multiplexer MUX 2 may further include a plurality of output terminals, for example, an output terminal Y21 and an output terminal Y22. Each output terminal of the multiplexer MUX 2 may correspond to a plurality of input terminals, and the MUX 2 may control an output terminal to be connected to one of the input terminals corresponding to the output terminal. The output terminal Y21 may correspond to the input terminal A21 and the input terminal B21, and the output terminal Y22 may correspond to the input terminal A22 and the input terminal B22.

The output terminal Y21 may be connected to a non-inverting input terminal of the operational amplifier AMP_7, and the output terminal Y22 may be connected to a non-inverting input terminal of an operational amplifier AMP_8.

The input terminal A21 may be connected to a digital-to-analog conversion module DAC 0, and the input terminal A22 may be connected to the digital-to-analog conversion module DAC 0. The input terminal B21 may be connected to the working electrode W2 in the electrode group 2, and the input terminal B21 may be further connected to the non-inverting input terminal of the instrument amplifier IA through a resistor R5. The input terminal B22 may be connected to the LA electrode (namely, the reference electrode R1 in the electrode group 1), and the input terminal B22 may be further connected to the output terminal Y12 of the multiplexer MUX 1 and the input terminal A31 of the multiplexer MUX 3. The input terminal B22 may be further connected to the non-inverting input terminal of the instrument amplifier IA through the resistor R6.

The multiplexer MUX 3 may include a plurality of input terminals, for example, the input terminal A31, the input terminal A32, an input terminal B31, and an input terminal B32. The multiplexer MUX 3 may further include a plurality of output terminals, for example, an output terminal Y31 and an output terminal Y32. Each output terminal of the multiplexer MUX 3 may correspond to a plurality of input terminals, and the MUX 3 may control an output terminal to be connected to one of the input terminals corresponding to the output terminal. The output terminal Y31 may correspond to the input terminal A31 and the input terminal B31, and the output terminal Y32 may correspond to the input terminal A32 and the input terminal B32.

The output terminal Y31 may be connected to an inverting input terminal of the operational amplifier AMP_7, and the output terminal Y32 may be connected to an inverting input terminal of the operational amplifier AMP_8. The operational amplifier AMP_7 and the operational amplifier AMP_8 belong to the potentiostat circuit 0.

The input terminal A31 may be connected to the output terminal Y12 of the multiplexer MUX 1 and the input terminal B22 of the multiplexer MUX 2. The input terminal A32 may be connected to the output terminal Y13 of the multiplexer MUX 1, and the input terminal A32 may be further connected to an output terminal of the operational amplifier AMP_8 through a resistor RTIA_0. The input terminal B31 may be connected to an output terminal of the instrument amplifier IA, and the input terminal B31 may be further connected to an inverting input terminal of an operational amplifier AMP_3 through a resistor R2. The input terminal B32 may be connected to the input terminal B31, that is, inputs of the input terminal B31 and the input terminal B32 are the same.

When the multiplexer MUX 2 selects to connect the input terminal A21 and the input terminal A22, and the multiplexer MUX 3 selects to connect the input terminal A31 and the input terminal A32, the measurement circuit may measure a physiological parameter (for example, blood glucose or blood ketone) of a user. When the MUX 2 selects to connect the input terminal B21 and the input terminal B22, and the multiplexer MUX 3 selects to connect the input terminal B31 and the input terminal B32, the measurement circuit may measure an electrocardiography signal.

The potentiostat circuit 0 may include the digital-to-analog conversion module DAC 0, the operational amplifier AMP_7, the operational amplifier AMP_8, the multiplexer MUX 2, and the multiplexer MUX 3. For a connection manner of the elements, refer to the related descriptions of the multiplexer MUX 2 and the multiplexer MUX 3. For function descriptions of other elements, refer to the related descriptions in the foregoing embodiments. Details are not described herein again. In addition, an output terminal of the operational amplifier AMP_7 may be further connected to a filter filter1, and the output terminal of the operational amplifier AMP_8 may be further connected to a filter filter2.

The transimpedance circuit 0 may include the operational amplifier AMP_8, the resistor RTIA_0, and the multiplexer MUX 3. For a connection manner of the elements, refer to the related descriptions of the multiplexer MUX 2 and the multiplexer MUX 3. For function descriptions of other elements, refer to the related descriptions in the foregoing embodiments. Details are not described herein again.

The amplification circuit may include the instrument amplifier IA, and may further include the resistor R5 and the resistor R6. The resistor R6 may be connected to the LA electrode (namely, the reference electrode R1) and the non-inverting input terminal of the instrument amplifier IA, and the resistor R5 may be connected to the RA electrode (namely, the working electrode W2) and the non-inverting input terminal of the instrument amplifier IA. The inverting input terminal of the instrument amplifier IA may be connected to the output terminal of the instrument amplifier IA. For another connection relationship of the instrument amplifier IA, refer to related descriptions of other elements. Details are not described herein again.

For composition of elements in the right leg drive circuit and a connection relationship between the elements, refer to the related descriptions in the embodiment shown in FIG. 4C. In addition, the inverting input terminal of the operational amplifier AMP_3 may be further connected to the output terminal of the instrument amplifier IA, and the input terminal B31 and the input terminal B32 of the multiplexer MUX 3 through the resistor R2.

The filter circuit may include the filter filter1 and the filter filter2. The filter circuit may be configured to perform filtering on signals output by the operational amplifier AMP_7 and the operational amplifier AMP_8.

For specific function descriptions of each module in the measurement circuit, refer to the related descriptions in the embodiment shown in FIG. 4C. Details are not described herein again.

It may be understood that the embodiment shown in FIG. 4H is merely an example. In embodiments of this application, the measurement circuit may further include more electrode groups, potentiostat circuits, and transimpedance circuits, and the multiplexer may alternatively include more input terminals and output terminals, or more multiplexers may be included, to control measurement of more physiological parameters. This is not limited in this application. In addition, each circuit module of the measurement circuit may further include more or fewer elements than those in the foregoing embodiment, or include elements different from those in the foregoing embodiment. This is not limited in this application.

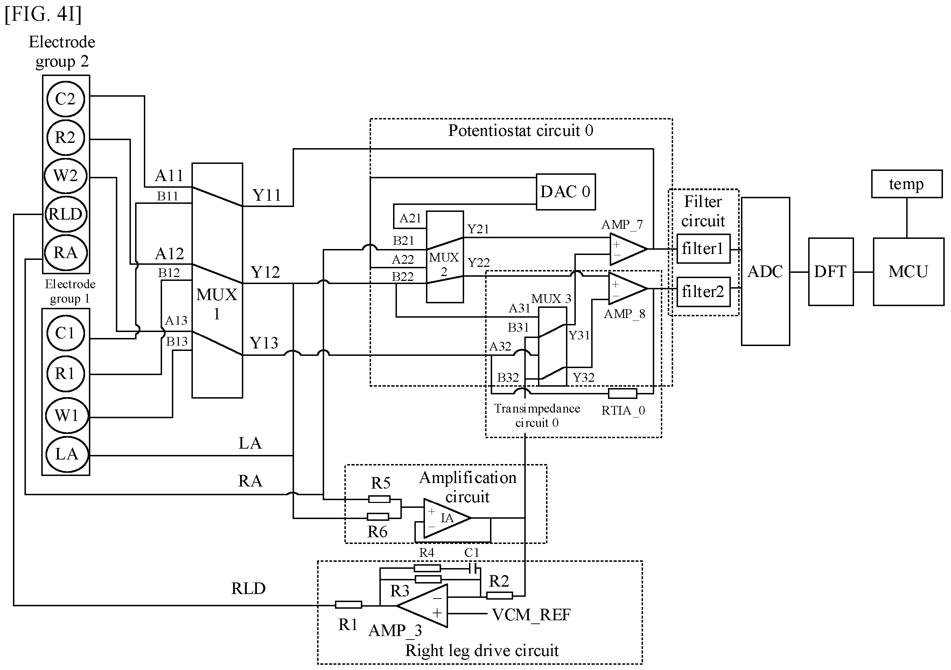

FIG. 4I is a circuit diagram of another measurement circuit according to an embodiment of this application.

As shown in FIG. 4I, the measurement circuit may include a plurality of multiplexers, a potentiostat circuit 0, a transimpedance circuit 0, a right leg drive circuit, an amplification circuit, a filter circuit, an analog-to-digital conversion module ADC, an MCU, and the like. Optionally, the measurement circuit may further include any one or more of the following: a discrete Fourier transform module DFT, a temperature module temp, and the like. In addition, the measurement circuit may further include a plurality of electrode groups, for example, an electrode group 1 and an electrode group 2. The electrode group 1 may include one or more working electrodes W1, one or more reference electrodes R1, and one or more counter electrodes C1, and the electrode group 1 may further include an LA electrode. The electrode group 2 may include one or more working electrodes W2, one or more reference electrodes R2, and one or more counter electrodes C2, and the electrode group 2 may further include an RA electrode and an RLD electrode.

The plurality of multiplexers may include a multiplexer MUX 1, a multiplexer MUX 2, and a multiplexer MUX 3. The multiplexer MUX 1 may be configured to control the potentiostat circuit 0 and the transimpedance circuit 0 to connect the electrode group 1 or the electrode group 2. The multiplexer MUX 2 and the multiplexer MUX 3 may be configured to control the measurement circuit to measure an electrocardiography signal or measure another physiological parameter.

The following describes connection relationships between electrodes in the electrode group 1 and the electrode group 2 and the measurement circuit.

In the electrode group 1, the one or more working electrodes W1 may be connected to an input terminal B13 of the multiplexer MUX 1, the one or more reference electrodes R1 may be connected to an input terminal B12 of the multiplexer MUX 1, the one or more counter electrodes C1 may be connected to an input terminal B11 of the multiplexer MUX 1, the LA electrode may be connected to a non-inverting input terminal of an instrument amplifier IA through a resistor R6, and the LA electrode may be further connected to an output terminal Y12 of the multiplexer MUX 1.

In the electrode group 2, the one or more counter electrodes W2 may be connected to an input terminal A13 of the multiplexer MUX 1, the one or more reference electrodes R2 may be connected to an input terminal A12 of the multiplexer MUX 1, the one or more counter electrodes C2 may be connected to an input terminal A11 of the multiplexer MUX 1, the RA electrode may be connected to the non-inverting input terminal of the instrument amplifier IA through a resistor R5, and the RA electrode may be further connected to an input terminal B21 of the multiplexer MUX 2. The RLD electrode may be connected to an output terminal of an operational amplifier AMP_3 through a resistor R1.

When the multiplexer MUX 1 selects to connect the input terminal A11, the input terminal A12, and the input terminal A13, the potentiostat circuit 0 and the transimpedance circuit 0 may be connected to an electrochemical electrode in the electrode group 2 through the multiplexer MUX 1. When the multiplexer MUX 1 selects to connect the input terminal B11, the input terminal B12, and the input terminal B13, the potentiostat circuit 0 and the transimpedance circuit 0 may be connected to an electrochemical electrode in the electrode group 1 through the multiplexer MUX 1.

When the multiplexer MUX 2 selects to connect an input terminal A21 and an input terminal A22, and the multiplexer MUX 3 selects to connect an input terminal A31 and an input terminal A32, the measurement circuit may measure a physiological parameter (for example, blood glucose or blood ketone) of a user. When the MUX 2 selects to connect an input terminal B21 and an input terminal B22, and the multiplexer MUX 3 selects to connect an input terminal B31, an input terminal B32, and an input terminal B33, the measurement circuit may measure an electrocardiography signal.

For specific function descriptions of each module in the measurement circuit, refer to the related descriptions in the embodiment shown in FIG. 4H. Details are not described herein again.

It may be understood that the embodiment shown in FIG. 4I is merely an example. In embodiments of this application, the measurement circuit may further include more electrode groups, potentiostat circuits, and transimpedance circuits, and the multiplexer may alternatively include more input terminals and output terminals, or more multiplexers may be included, to control measurement of more physiological parameters. This is not limited in this application. In addition, each circuit module of the measurement circuit may further include more or fewer elements than those in the foregoing embodiment, or include elements different from those in the foregoing embodiment. This is not limited in this application.

The measurement circuit shown in FIG. 4H or FIG. 4I may be used to: measure an electrocardiography signal by controlling a multiplexer, or measure a physiological parameter by using an electrochemical electrode.

In another possible implementation, the measurement circuit may include three or more electrochemical circuit modules, and a plurality of working electrodes in an electrode group may be separately connected to different electrochemical circuit modules, to measure different physiological parameters. In this way, the measurement circuit may be used to simultaneously measure three or more physiological parameters and an electrocardiography signal.

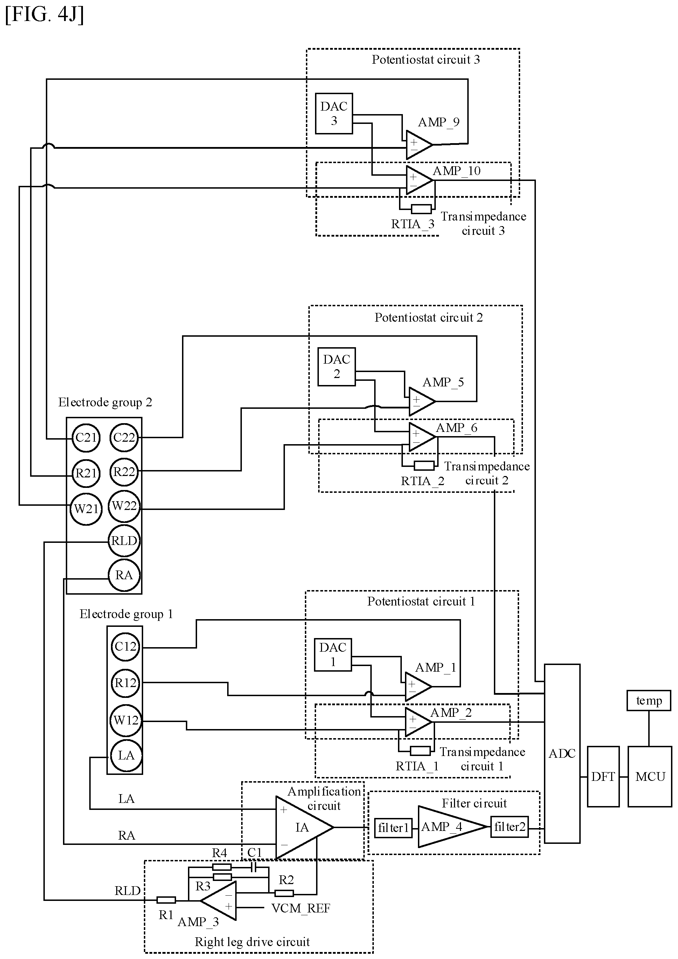

For example, as shown in FIG. 4J, a measurement circuit may include a potentiostat circuit 1, a transimpedance circuit 1, a potentiostat circuit 2, a transimpedance circuit 2, a potentiostat circuit 3, a transimpedance circuit 3, a right leg drive circuit, an amplification circuit, a filter circuit, an analog-to-digital conversion module ADC, an MCU, and the like. Optionally, the measurement circuit may further include any one or more of the following: a discrete Fourier transform module DFT, a temperature module temp, and the like. In addition, the measurement circuit may further include a plurality of electrode groups, for example, an electrode group 1 and an electrode group 2. The electrode group 1 may include one or more working electrodes W1, one or more reference electrodes R1, and one or more counter electrodes C1. In addition, the electrode group 1 may further include an LA electrode. The electrode group 2 may include a working electrode W21, a working electrode W22, a reference electrode R21, a reference electrode R22, a counter electrode C21, and a counter electrode C22. In addition, the electrode group 2 may further include an RA electrode and an RLD electrode.

For composition of internal elements in circuits such as the potentiostat circuit 1, the transimpedance circuit 1, the potentiostat circuit 2, the transimpedance circuit 2, the right leg drive circuit, the amplification circuit, and the filter circuit and a connection relationship between the elements, refer to the related descriptions in the embodiment shown in FIG. 4D. Details are not described herein again. For function descriptions of the analog-to-digital conversion module ADC, the discrete Fourier transform module DFT, and the MCU, refer to the related descriptions in the embodiment shown in FIG. 4D.

The potentiostat circuit 3 may include an operational amplifier AMP_9, an operational amplifier AMP_10, and a digital-to-analog conversion module DAC 3. In some embodiments, the digital-to-analog conversion module DAC 3 may alternatively be replaced with a digital-to-analog conversion module DAC 1 in the potentiostat circuit 1, or replaced with a digital-to-analog conversion module DAC 2 in the potentiostat circuit 2. This is not limited in this application. The DAC 3 may generate a stable voltage signal. The DAC 3 may be connected to a non-inverting input terminal of the operational amplifier AMP_9, and may be further connected to a non-inverting input terminal of the operational amplifier AMP_10, to provide a same voltage input for the operational amplifier AMP_9 and the operational amplifier AMP_10. An output terminal of the operational amplifier AMP_9 may be connected to the counter electrode C21 in the electrode group 2, and an inverting input terminal of the operational amplifier AMP_9 may be connected to the reference electrode R21 in the electrode group 2. An inverting input terminal of the operational amplifier AMP_10 may be connected to the working electrode W21 in the electrode group 2, and an output terminal of the operational amplifier AMP_10 may be connected to the ADC. In this way, a same voltage is applied to the non-inverting input terminal of the operational amplifier AMP_9 and the non-inverting input terminal of the operational amplifier AMP_10, so that a voltage difference between the reference electrode R21 and the working electrode W21 can be controlled, and a voltage on the working electrode W21 is approximately equal to a voltage on the reference electrode R21.

The transimpedance circuit 3 may include the operational amplifier AMP_10 and a resistor RTIA_3. Two terminals of the resistor RTIA_3 may be respectively connected to the inverting input terminal and the output terminal of the operational amplifier AMP_10. The inverting input terminal of the operational amplifier AMP_10 may be further connected to the working electrode W21 in the electrode group 2.

For composition of elements in the potentiostat circuit 2 and the transimpedance circuit 2 and a connection relationship between the elements, refer to the related descriptions in the embodiment shown in FIG. 4D.

A connection relationship between the potentiostat circuit 2 and a plurality of electrodes in the electrode group 2 is as follows: An output terminal of an operational amplifier AMP_5 may be connected to the counter electrode C22 in the electrode group 2, and an inverting input terminal of the operational amplifier AMP_5 may be connected to the reference electrode R22 in the electrode group 2. An inverting input terminal of the operational amplifier AMP_6 may be connected to the working electrode W22 in the electrode group 2, and an output terminal of the operational amplifier AMP_6 may be connected to the ADC. In this way, a same voltage is applied to the non-inverting input terminal of the operational amplifier AMP_5 and the non-inverting input terminal of the operational amplifier AMP_6, so that a voltage difference between the reference electrode R22 and the working electrode W22 can be controlled, and a voltage on the working electrode W22 is approximately equal to a voltage on the reference electrode R22.

A connection relationship between the transimpedance circuit 2 and the plurality of electrodes in the electrode group 2 is as follows: The inverting input terminal of the operational amplifier AMP_6 may be further connected to the working electrode W22 in the electrode group 2.

For composition of elements of another module in the measurement circuit and a connection relationship between the elements, refer to related content in the embodiment shown in FIG. 4D. Details are not described herein again.

It may be understood that the embodiment shown in FIG. 4J is merely an example for description. Different working electrodes in an electrode group may be connected to different electrochemical circuit modules. In embodiments of this application, the measurement circuit may further include more or fewer electrochemical circuit modules than those in the foregoing embodiment, and a plurality of working electrodes in the electrode group 2 (or the electrode group 1) may alternatively be separately connected to different electrochemical circuit modules to measure different physiological parameters. This is not limited in this application.

In some other embodiments, a same reference electrode and/or counter electrode may be connected to different electrochemical circuit modules (for example, different potentiostat circuits) to measure a plurality of different physiological parameters. This is not limited in this application either.

It may be understood that the embodiments shown in FIG. 4C to FIG. 4J are merely some examples. In embodiments of this application, the measurement circuit may further include more or fewer circuit modules and elements than those in the foregoing embodiments, or include circuit modules and elements different from those in the foregoing embodiments, or a connection manner of the elements in the measurement circuit may be different from that in the foregoing embodiments. This is not limited in this application. For example, one or more circuit switches may be further disposed in the measurement circuit (or in the circuit module of the measurement circuit) shown in any one of the foregoing embodiments. The one or more circuit switches may also be used by the measurement circuit to measure one or more physiological parameters (or electrocardiography signals). This is not limited in this application.

It should be noted that the electrode group 1 and the electrode group 2 in the embodiments shown in FIG. 4C to FIG. 4J may be disposed in a microneedle sensor, or may be disposed in an array sensor. In addition, the electrode group 1 and the electrode group 2 in the embodiments shown in FIG. 4C to FIG. 4J may alternatively use a dual-electrode system. In the dual-electrode system, a connection manner of the counter electrode and the working electrode may be the same as that in the foregoing embodiments, and the counter electrode may also be used as a reference electrode to be connected to a port connected to the reference electrode in the foregoing embodiments. This is not limited in this application.

The following describes a measurement method according to an embodiment of this application.

FIG. 5A is a schematic flowchart of a measurement method according to an embodiment of this application.

As shown in FIG. 5A, a specific procedure of the measurement method may include the following steps.

S501: An electronic device 200 determines that a monitoring condition 1 is satisfied, and determines a physiological parameter 1.

The monitoring condition 1 may include but is not limited to any one or more of the following: Information 1 that is sent by another electronic device and that is used to indicate the electronic device 200 to detect the physiological parameter 1 is received; it is detected that a user wears the electronic device 200 (that is, an electrode of the electronic device 200 is implanted into a subcutaneous tissue of the user) or the like; and it is detected that the electrocardiography signal is abnormal.

For example, the electronic device 200 may determine, based on whether a temperature detected by a temperature sensor is within a preset body temperature range, whether the electronic device 200 is worn by the user. It may be understood that this embodiment is merely an example. In embodiments of this application, the electronic device 200 may alternatively determine, in another manner, whether the electronic device 200 is worn. This is not limited in this application.

The physiological parameter 1 may include but is not limited to any one or more of the following: blood glucose, blood ketone, uric acid, blood lactic acid, and the like.

In a possible implementation, when determining that the monitoring condition 1 is satisfied, the electronic device 200 may measure the physiological parameter 1, and stop monitoring after determining the physiological parameter 1. In another possible implementation, when determining that the monitoring condition 1 is satisfied, the electronic device 200 may periodically detect the physiological parameter 1, for example, determine a value of the physiological parameter 1 at a current moment at an interval of 10 minutes (or 20 minutes, 30 minutes, or the like). It should be noted that, in this case, different physiological parameters may correspond to different monitoring cycles, or may correspond to a same monitoring cycle. This is not limited in this application.

The electronic device 200 may obtain, by using an electrochemical electrode corresponding to the physiological parameter 1, a reaction current generated when the electrochemical electrode reacts with a target substance 1, and determine the physiological parameter 1 based on a magnitude of the reaction current.

For example, if the physiological parameter 1 is blood glucose, and the electrochemical electrode for measuring the blood glucose includes the working electrode 3031, the reference electrode 3032, and the counter electrode 3033 in the microneedle sensor 303 shown in FIG. 3B, when the microneedle sensor 303 is implanted into the subcutaneous tissue, the working electrode 3031 may react with glucose to generate a reaction current. The electronic device 200 may obtain a magnitude of the reaction current, and determine a value of the blood glucose based on the magnitude of the reaction current by using a blood glucose calculation model stored in the electronic device 200.

For another example, if the physiological parameter 1 is blood glucose, and the electrochemical electrode for measuring the blood glucose includes the working electrode group 3073, the reference electrode group 3074, and the counter electrode group 3075 in the array sensor 307 shown in FIG. 3D, when the array sensor 307 is implanted into the subcutaneous tissue, the working electrode group 3073 may react with glucose to generate a reaction current. The electronic device 200 may obtain a magnitude of the reaction current, and determine a value of the blood glucose based on the magnitude of the reaction current by using a blood glucose calculation model stored in the electronic device 200.

It may be understood that this embodiment is merely two examples. In embodiments of this application, the physiological parameter 1 may alternatively be another physiological parameter different from blood glucose, or may include a plurality of physiological parameters. This is not limited in this application.

Optionally, the electronic device 200 may further measure a body temperature of the user. In this case, further optionally, the electronic device 200 may further calibrate the value of the physiological parameter 1 based on the body temperature of the user.

S502: The electronic device 200 outputs the physiological parameter 1.

In some embodiments, the electronic device 200 may include (or be connected to) an audio module and/or a display. In this case, the electronic device 200 may output the physiological parameter 1 through the audio module in a voice broadcast manner, or may output the physiological parameter 1 through the display in a display manner.

In some other embodiments, that the electronic device 200 outputs the physiological parameter 1 may alternatively be sending output information 1 to another electronic device (for example, an electronic device 100). The output information 1 may include the physiological parameter 1, and the output information 1 may be used to indicate a receive end to output the physiological parameter 1.

In some embodiments, when outputting the physiological parameter 1 of the user (or after outputting the physiological parameter 1), the electronic device 200 may further output any one or more of the following content: an evaluation result, a value range, a user status, a historical curve, a reference suggestion, and the like. For specific descriptions and determining manners of the content, refer to related descriptions in step S605 shown in FIG. 6 below. Details are not described herein.

S503: The electronic device 200 determines that a monitoring condition 2 is satisfied, and obtains an electrocardiography signal.

It should be noted that there is no limitation on an execution sequence of step S503 and step S501. The electronic device 200 may simultaneously perform step S501 and step S503, or may first perform one of the steps, and then perform the other step. This is not limited in this application.

The monitoring condition 2 may include but is not limited to any one or more of the following: Information 2 that is sent by another electronic device and that is used to indicate the electronic device 200 to detect the electrocardiography signal is received; it is detected that a living being wears the electronic device 200 (that is, an electrode of the electronic device 200 is implanted into a subcutaneous tissue); it is detected that the physiological parameter 1 is abnormal; and the like.

That the physiological parameter 1 is abnormal means that a value of the physiological parameter 1 is not within a preset value range of the physiological parameter 1, where the value range may be prestored in the electronic device 200. For example, the electronic device 200 may start to obtain the electrocardiography signal when detecting that the blood glucose of the user exceeds a preset blood glucose value range. It may be understood that this embodiment is merely an example for describing the following: Whether to obtain the electrocardiography signal may be determined based on a value of a physiological parameter. In embodiments of this application, the physiological parameter 1 may alternatively be another physiological parameter different from blood glucose, or may include a plurality of physiological parameters. This is not limited in this application.

It should be noted that, in a process of obtaining the electrocardiography signal, an electrocardiography electrode configured to measure the electrocardiography signal may be implanted into the subcutaneous tissue. In some embodiments, the electrocardiography electrode may be an electrochemical electrode that is in the electronic device 200 and that is configured to measure another physiological parameter.

For example, if the electronic device 200 is the electronic device 200 in the embodiment shown in FIG. 3B, the electrocardiography electrode may be a part of electrochemical electrodes in the microneedle sensor 303 and the microneedle sensor 304. For example, an LA electrode may be the reference electrode 3032, an RA electrode may be the working electrode 3041, and an RLD electrode may be the counter electrode 3043. For another example, if the electronic device 200 is the electronic device 200 in the embodiment shown in FIG. 3D, the electrocardiography electrode may be the electrocardiography electrode in the array sensor 307 and the array sensor 308. For example, an LA electrode may be the LA electrode 3081, an RA electrode may be the RA electrode 3071, and an RLD electrode may be the RLD electrode 3072. It may be understood that this embodiment is merely two examples. In embodiments of this application, when the electrocardiography electrode and the electrochemical electrode are a same electrode, a correspondence between the electrocardiography electrode and the electrochemical electrode may alternatively be a relationship different from that in the foregoing embodiment. This is not limited in this application.

In a bipolar lead system, the electrocardiography signal may be a voltage difference between the LA electrode and the RA electrode in a preset time period (for example, 1 minute or 3 minutes). When the electronic device 200 measures the electrocardiography signal by using another lead system, the electrocardiography signal may alternatively be a voltage difference between other electrodes in a preset time period. This is not limited in this application.

For example, FIG. 5B is a diagram of a waveform of an electrocardiography signal according to an embodiment of this application.

As shown in FIG. 5B, a two-dimensional coordinate system may include a horizontal axis and a vertical axis. The horizontal axis may represent time, and the vertical axis may represent a voltage. The waveform of the electrocardiography signal may be a curve Q in the two-dimensional coordinate system. It can be learned from the curve Q that the waveform of the electrocardiography signal periodically fluctuates as a heart periodically pumps blood.

It may be understood that the embodiment shown in FIG. 5B is merely an example. In embodiments of this application, the waveform of the electrocardiography signal may alternatively be a waveform different from that in the foregoing embodiment. This is not limited in this application.

S504: The electronic device 200 outputs the electrocardiography signal.

In some embodiments, the electronic device 200 may include (or be connected to) an audio module and/or a display. In this case, the electronic device 200 may output the electrocardiography signal through the audio module in a voice broadcast manner, or may output the electrocardiography signal through the display in a displaying manner.

In some other embodiments, that the electronic device 200 outputs the electrocardiography signal may alternatively be sending output information 2 to another electronic device (for example, the electronic device 100). The output information 2 may include the electrocardiography signal, and the output information 2 may be used by the receive end to output the electrocardiography signal.

In some embodiments, the electronic device 200 (or the electronic device 100) may output the electrocardiography signal in one or more different forms, for example, output the electrocardiography signal in a form of waveform diagram (output an electrocardiogram) or in a form of electrocardiography indicator.

In some embodiments, the electronic device 200 (or the electronic device 100) may determine an electrocardiogram based on the electrocardiography signal, and display the electrocardiogram through the display. The electrocardiogram may be a waveform diagram of the electrocardiography signal, and the electrocardiogram indicates a relationship between an amplitude of the electrocardiography signal and time.

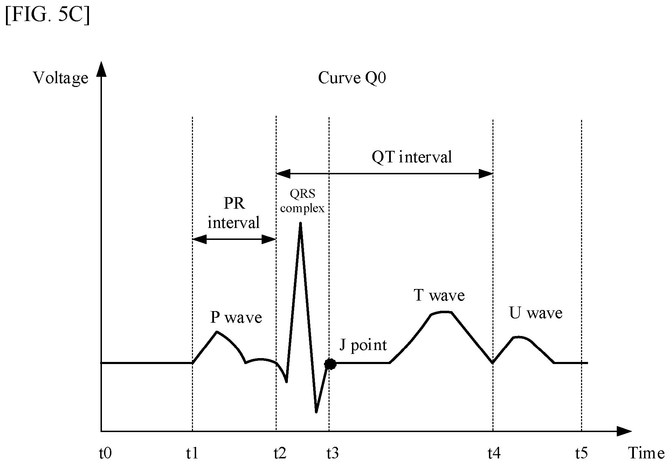

The electrocardiogram may include a plurality of different waves, for example, a P wave, a QRS complex, a T wave, and a U wave. The following describes the waves in the electrocardiogram with reference to the electrocardiogram shown in FIG. 5C.

As shown in FIG. 5C, a two-dimensional coordinate system may include a horizontal axis and a vertical axis. The horizontal axis may represent time, and the vertical axis may represent a voltage. A waveform of an electrocardiography signal in a single cardiac cycle may be a curve Q0 in the two-dimensional coordinate system.

As shown in FIG. 5C, in the curve Q0, a curve segment from a moment t1 to a moment t2 may be referred to as a P wave; a curve segment from the moment t2 to a moment t3 may be referred to as a QRS complex; a curve segment from the moment t3 to a moment t4 may be referred to as a T wave; and a curve segment from the moment t4 to a moment t5 may be referred to as a U wave. A point in the curve Q0 at the moment t3 may be referred to as a J point, and the J point indicates an end of the QRS complex. In addition, a time period from the moment t1 to the moment t2 may be referred to as a PR interval, and a time period from the moment t2 to the moment t4 may be referred to as a QT interval.

For example, a change of an amplitude of the curve Q0 in each time period is as follows: From a moment t0 to the moment t1, the amplitude of the curve Q0 is stable; from the moment t1 to the moment t2, the amplitude of the curve Q0 first increases to a first wave crest, then decreases, then becomes stable, and tends to decrease at the moment t2; from the moment t2 to the moment t3, the amplitude of the curve Q0 first decreases to a first wave trough, then increases, decreases after reaching a second wave crest, and increases again after reaching a second wave trough; from the moment t3 to the moment t4, the amplitude of the curve Q0 gradually increases after being stable for a period of time, and gradually decreases to a third wave trough after reaching a third wave crest; and from the moment t4 to the moment t5, the amplitude of the curve Q0 increases again from the third wave trough to a fourth wave crest, decreases again, and then becomes stable. An amplitude of the second wave crest is far greater than amplitudes of other wave crests.

It may be understood that the embodiment shown in FIG. 5C is merely an example. In embodiments of this application, the electrocardiogram may alternatively include more cycles than those in the foregoing embodiment, and a waveform in each cycle may alternatively be different from that in the foregoing embodiment. For example, in some cycles, the P wave may alternatively include two wave crests or the like. This is not limited in this application.

Each wave and interval in the electrocardiogram may indicate a health status of the heart of the user. The P wave indicates a depolarization process of two atriums. A sinus node is located at a junction of a right atrium and a superior vena cava. Therefore, excitation of the sinus node is first conducted to the right atrium and then to a left atrium through an interatrial bundle, to form the P wave in the electrocardiogram. The P wave indicates excitation of the atriums. The first half indicates excitation of the right atrium, and the second half indicates excitation of the left atrium. When the atriums are enlarged and conduction between the two atriums is abnormal, the P wave is a peaked or double-peaked P wave. The PR interval indicates time from a start of atrial depolarization to a start of ventricular depolarization, and mainly reflects time for the excitation to be conducted through an atrioventricular junction. A conduction velocity of an atrioventricular node is slow, so that a PR segment in the electrocardiogram is formed. When atrial-to-ventricular conduction is blocked, the PR interval is prolonged or a ventricular wave disappears after the P wave. The QRS complex indicates a ventricular depolarization process. When conduction block of left and right bundle branches of a heart, ventricular enlargement or hypertrophy, or the like occurs, the QRS complex is widened, deformed, and prolonged in duration. The J point indicates that all ventricular myocytes are depolarized. The T wave indicates a repolarization process of two ventricles. A change of the T wave in the electrocardiogram is affected by a plurality of factors. For example, the T wave may be flat and inverted when myocardial ischemia occurs, and the T wave may be peaked when hyperkalemia, a hyperacute period of acute myocardial infarction, or the like occurs. The U wave may be a waveform after the T wave, and is currently considered to be related to repolarization of the ventricles. The QT interval indicates time required for an entire ventricular depolarization and repolarization process. Prolongation of the QT interval is usually associated with malignant arrhythmia.

The electronic device 200 may determine one or more electrocardiography indicators based on the electrocardiography signal, and the electronic device 200 may further output the one or more electrocardiography indicators when outputting the electrocardiography signal (or after outputting the electrocardiography signal). The electrocardiography indicator may include but is not limited to any one or more of the following: a heart rate, an amplitude, a PR interval, a QT interval, and a duration of each wave (for example, P wave duration). The heart rate may be determined based on a quantity of cardiac cycles of the electrocardiography signal per minute. The PR interval, the QT interval, the P wave duration, and the like may be determined based on the electrocardiogram (or the electrocardiography signal).

FIG. 6 is a schematic flowchart of another measurement method according to an embodiment of this application.

As shown in FIG. 6, a specific procedure of the measurement method may include the following steps.

S601: An electronic device 100 determines that a monitoring condition 3 is satisfied, and sends information 3 to an electronic device 200, where the information 3 is used to request the electronic device 200 to send physiological data 1 to the electronic device 100.

In some embodiments, the monitoring condition 3 may include but is not limited to any one or more of the following: An operation of enabling a physiological monitoring function by a user is received, a start instruction 1 sent by another electronic device is received, it is detected that a physiological status of the user is abnormal (for example, a heart rate is not within a preset heart rate range), it is detected that a psychological status of the user is abnormal (for example, the user is frightened), it is detected that the user is in an exercise state, it is detected that the user has insomnia, it is detected that a body posture of the user is abnormal (for example, the user falls down), it is detected that exercise equipment of the user is abnormal, it is detected that a position of the user is within a preset area range (for example, the user is in a high-altitude area), and the like.

The physiological data 1 may be used to determine a physiological parameter 1. The physiological parameter 1 may include but is not limited to any one or more of the following: blood glucose, blood ketone, blood lactic acid, uric acid, and the like.

The physiological data 1 may include but is not limited to any one or more of the following: current data, a body temperature, a measured value of the physiological parameter 1, a calibrated value of the physiological parameter 1, and the like. The current data indicates a magnitude of a reaction current generated when an electrochemical electrode in the electronic device 200 reacts with a target substance 1. The body temperature may be a body temperature of the user (or another living being) wearing the electronic device 200. The measured value of the physiological parameter 1 may be a value that is of the physiological parameter 1 and that is determined by the electronic device 200 based on the current data by using a calculation model of the physiological parameter 1. The calibrated value of the physiological parameter 1 may be a value that is of the physiological parameter 1 and that is determined by the electronic device 200 based on the current data and the body temperature, namely, a value of the physiological parameter 1 after calibration based on the body temperature.

The following describes a specific manner in which the electronic device 100 determines whether the monitoring condition 3 is satisfied.

In some embodiments, the electronic device 100 may obtain user information, and determine, based on the user information, whether the electronic device 100 satisfies the monitoring condition 3. The user information may include but is not limited to any one or more of the following: physiological information, psychological information, exercise information, exercise equipment information, posture information, position information, interaction information, and the like. The physiological information may indicate the physiological status of the user. The physiological information may include but is not limited to any one or more of the following: the heart rate, the body temperature, blood pressure, disease information, and the like. The psychological information may indicate the psychological status of the user. The psychological information may include but is not limited to any one or more of the following: a stress value, a low mood, a stable mood, a high mood, a mood of being frightened, and the like. The exercise information may indicate the exercise state of the user. The exercise information may include but is not limited to any one or more of the following: swimming, diving, cycling, running, mountain climbing, rope jumping, yoga, and the like. The exercise equipment information may indicate a status of the exercise equipment. The exercise equipment information may include but is not limited to any one or more of the following: an oxygen balance in an oxygen cylinder, a weight of a smart backpack, traveling resistance of a bicycle, and the like. The posture information may indicate the body posture of the user. The posture information may include but is not limited to any one or more of the following: falling down, stepping on the air, being static, and the like. The position information may indicate the position of the user. The position information may include but is not limited to any one or more of the following: a geographical position of the user, latitude and longitude information of the position of the user, altitude information of the position of the user, depth information of the position of the user, and the like. The interaction information may include an interaction operation between the user and the electronic device 100, for example, an operation of enabling the physiological monitoring function by the user is received.

It should be noted that, in this embodiment of this application, a manner in which the electronic device 100 obtains the user information may include but is not limited to the following manners: The electronic device 100 detects the user information, the electronic device 100 receives the user information sent by another electronic device, or the electronic device 100 receives and obtains the user information (for example, disease information) in response to an operation of entering the user information by the user.

The following describes some manners in which the electronic device 100 detects the user information according to this embodiment of this application.

For example, the electronic device 100 may detect the exercise information and the posture information of the user by using a component like a gyroscope sensor or an acceleration sensor. The electronic device 100 may detect the physiological information such as the heart rate and the blood pressure of the user by using a component like a PPG module. The electronic device 100 may further collect a facial expression of the user by using a camera, and determine an emotion status of the user by using an algorithm model like image analysis or facial expression analysis. The electronic device 100 may determine the psychological information such as the stress value of the user based on the physiological information. The electronic device 100 may further detect the interaction information of the user by using a touch sensor. The electronic device 100 may detect the position information of the user by using a position sensor (for example, a global positioning chip). The electronic device 100 may further detect barometric pressure of an environment in which the user is located by using a barometric pressure sensor, and determine the position information such as an altitude of the position of the user based on a barometric pressure value, and the like.

It may be understood that this embodiment is merely some examples. In embodiments of this application, the electronic device 100 may include more or fewer components that those in the foregoing embodiment, or include components different from those in the foregoing embodiment, and the electronic device 100 may collect the user information by using a sensor or another component different from those in the foregoing embodiment. This is not limited in this application.

The following describes some manners in which the electronic device 100 determines, based on the user information, whether the monitoring condition 3 is satisfied according to this embodiment of this application.

If the electronic device 100 determines that any physiological information is abnormal, the electronic device 100 determines that a monitoring condition is satisfied. For example, that the physiological information is abnormal may include but is not limited to any one or more of the following: The heart rate is not within the preset heart rate range, the blood pressure is not within a preset blood pressure range, the body temperature is not within a preset body temperature range, and the like.

For another example, the electronic device 100 may determine whether the physiological information satisfies any one of the following: The heart rate is not within the preset heart rate range, the blood pressure is not within a preset blood pressure range, the body temperature is not within a preset body temperature range, and the like. If the physiological information satisfies any one of the foregoing, the electronic device 100 may determine that the physiological status of the user is abnormal, that is, determine that the monitoring condition is satisfied.

If the electronic device 100 determines that the exercise information satisfies a preset exercise state, the electronic device 100 determines that the monitoring condition is satisfied. For example, the preset exercise state may include but is not limited to any one or more of the following: a diving state, a mountain climbing state, a cycling state, a yoga state, a swimming state, a running state, and the like.

If the electronic device 100 determines, based on the psychological information, that the psychological status of the user is abnormal, the electronic device 100 determines that the monitoring condition is satisfied. For example, that the psychological status is abnormal includes but is not limited to any one or more of the following: the user is frightened, the user is in a low mood, the user is in a high mood, and the like.

If the electronic device 100 determines, based on the position information, that the user is in a preset area, the electronic device 100 determines that the monitoring condition is satisfied. The preset area may include but is not limited to any one or more of the following: a high-altitude area, a deep-water area, and the like.

If the electronic device 100 determines, based on the posture information, that the body posture of the user is abnormal, the electronic device 100 determines that the monitoring condition is satisfied. For example, that the body posture of the user is abnormal includes but is not limited to any one or more of the following: The user falls down, the user steps on the air, and the like.

If the electronic device 100 determines, based on the exercise equipment information, that the exercise equipment of the user is abnormal, the electronic device 100 determines that the monitoring condition is satisfied. For example, that the exercise equipment of the user is abnormal may include but is not limited to any one or more of the following: The oxygen balance in the oxygen cylinder is less than a preset oxygen amount, the traveling resistance of the bicycle is greater than preset resistance, the weight of the smart backpack is greater than a preset weight, and the like. It may be understood that the foregoing embodiment is merely an example for describing a plurality of manners of determining whether the monitoring condition 3 is satisfied based on the user information. In embodiments of this application, the electronic device 100 may alternatively determine, based on a plurality of types of user information, whether the monitoring condition 3 is satisfied, or the electronic device 100 may determine, based on other information in the user information, whether the monitoring condition 3 is satisfied, and the monitoring condition 3 may alternatively include more or fewer conditions than those in the foregoing embodiment, or include conditions different from those in the foregoing embodiment. This is not limited in this application.

S602: The electronic device 200 obtains the physiological data 1.

In some embodiments, the electronic device 200 may start to obtain the physiological data 1 in response to the information 3.

In some other embodiments, the electronic device 200 may alternatively periodically obtain the physiological data 1 before receiving the information 3.

For a manner in which the electronic device 200 obtains the physiological data 1, refer to related content of step S501 shown in FIG. 5A. Details are not described herein again.

S603: The electronic device 200 sends the physiological data 1 to the electronic device 100.

In some embodiments, the electronic device 200 may send the physiological data 1 to the electronic device 100 in response to the information 3.

In some other embodiments, the electronic device 200 periodically sends the physiological data 1 to the electronic device 100 before receiving the information 3. In this case, step S602 may not be performed. In addition, the monitoring condition 3 may be used to trigger the electronic device 100 to perform step S605.

S604: The electronic device 100 determines the physiological parameter 1 based on the physiological data 1.

In some embodiments, if the physiological data 1 includes the measured value or the calibrated value of the physiological parameter 1, the electronic device 100 may use the measured value or the calibrated value of the physiological parameter 1 as the value of the physiological parameter 1.

In some embodiments, if the physiological parameter 1 includes the measured value of the physiological parameter 1 and the body temperature, the electronic device 100 may calibrate the measured value of the physiological parameter 1 based on the body temperature, and determine a calibrated value as the value of the physiological parameter 1.

In some embodiments, if the physiological data 1 includes the current data, the electronic device 100 may determine the value of the physiological parameter 1 based on the current data. Optionally, if the physiological data 1 further includes the body temperature, the electronic device 100 may alternatively determine the value of the physiological parameter 1 based on the current data and the body temperature.

S605: The electronic device 100 outputs the physiological parameter 1.

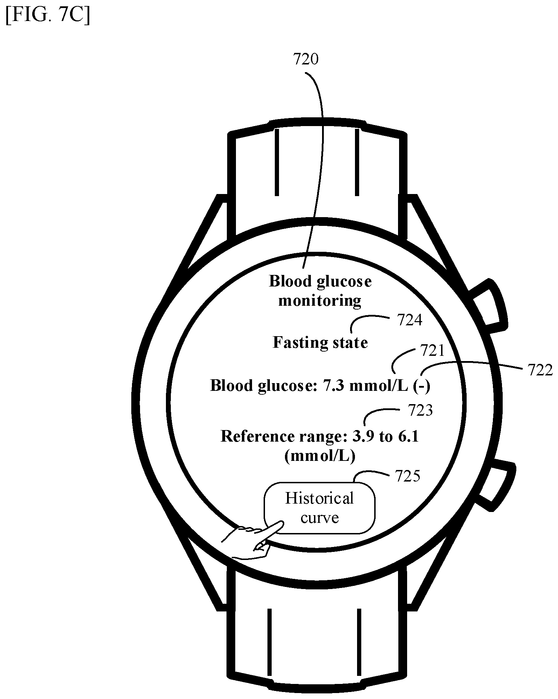

In some embodiments, after determining the physiological parameter 1 of the user, the electronic device 100 may output the physiological parameter 1. A manner in which the electronic device 100 outputs the physiological parameter 1 may include but is not limited to any one or more of the following: display displaying, voice broadcast, vibration, indicator blinking, and the like. For a diagram of an interface for outputting the physiological parameter 1 by the electronic device 100, refer to related descriptions in the embodiments shown in FIG. 7C to FIG. 7F below. Details are not described herein.

In some embodiments, when outputting the physiological parameter 1 of the user (or after outputting the physiological parameter 1), the electronic device 100 may further output any one or more of the following content: an evaluation result, a value range, a user status, a historical curve, a reference suggestion, and the like. The evaluation result indicates whether the physiological parameter of the user is normal. The value range is a normal range of the physiological parameter of the user. The user status is a current status of the user, for example, a fasting state, a non-fasting state, an exercise state, a high-altitude state, or a sleep state. The historical curve indicates a relationship between the physiological parameter of the user and time in a past period (for example, 30 minutes, 3 hours, or 24 hours). The reference suggestion may indicate the user to maintain or restore the physiological parameter to the normal range.

In some other embodiments, after determining the physiological parameter 1 of the user, the electronic device 100 may alternatively send an output instruction to another electronic device. The output instruction may include the physiological parameter 1 of the user, and the output instruction may indicate the electronic device to output the physiological parameter 1 of the user. Optionally, the output instruction may further include but is not limited to one or more of the following: the user status, the value range, the evaluation result, the historical curve, the reference suggestion, and the like. The electronic device 100 may output any one or more of the foregoing content based on the output instruction.

The following separately describes manners of determining the evaluation result, the value range, the user status, the historical curve, the reference suggestion, and the like.

The following describes a manner of determining the user status.

In some embodiments, the electronic device 100 may determine the user status based on the user information. For specific content of the user information, refer to the related descriptions in step S501. Details are not described herein again. For example, the electronic device 100 may receive and respond to an operation of setting the user status by the user, to determine the user status. For another example, the electronic device 100 may determine, based on the exercise information of the user, whether the user is in the exercise state. For another example, the electronic device 100 may determine, based on the position information of the user, whether the user is in the high-altitude state or the like. It may be understood that this embodiment is merely an example for describing the following: The electronic device 100 may determine the user status based on the user information. In embodiments of this application, the electronic device 100 may alternatively determine the user status based on other information in the user information. This is not limited in this application.

The following describes a manner of determining the value range.

The value range may be a value range of a physiological parameter stored in the electronic device 100. For example, Table 1 shows value ranges of physiological parameters stored in an electronic device 100 according to an embodiment of this application.

**Table 1**

| Physiological parameter | Value range unit: mmol/L (mmol/L) |
|---|---|
| Blood glucose | [3.9, 6.1] |
| Blood ketone | [0.05, 0.3] |
| Uric acid | [0.18, 0.42] |

As shown in Table 1, the electronic device 100 may store value ranges of one or more physiological parameters. For example, the value range of the blood glucose may be [3.9, 6.1], the value range of the blood ketone may be [0.05, 0.3], and the value range of the uric acid may be [0.18, 0.42]. Units of the foregoing value ranges are all mmol/L (mmol/L).

It may be understood that the embodiment shown in Table 1 is merely an example. In embodiments of this application, the electronic device 100 may further store value ranges of more or fewer physiological parameters than those shown in the embodiment in Table 1, or store value ranges of physiological parameters different from those in the foregoing embodiment, and the value ranges of the physiological parameters may alternatively be different from the foregoing value ranges. This is not limited in this application.

In some other embodiments, the electronic device 100 may alternatively store value ranges of physiological parameters in different user statuses. For example, Table 2 shows value ranges of physiological parameters in different user statuses stored in another electronic device 100 according to an embodiment of this application.

**Table 2**

| Physiological parameter | User status | Value range unit: mmol/L (mmol/L) |
|---|---|---|
| Blood glucose | Fasting state | [3.9, 6.1] |
| Blood glucose | Non-fasting state | [3.9, 8.99] |
| Blood ketone | Fasting state | [0.05, 0.3] |
| Blood ketone | Non-fasting state | [0.05, 0.5] |
| Uric acid | Fasting state | [0.18, 0.42] |
| Uric acid | Non-fasting state | [0.18, 0.5] |

As shown in Table 2, the electronic device 100 may store value ranges of one or more physiological parameters in different user statuses. For example, in the fasting state, the value range of the blood glucose may be [3.9, 6.1]; in the non-fasting state, the value range of the blood glucose may be [3.9, 8.99]; in the fasting state, the value range of the blood ketone may be [0.05, 0.3]; in the non-fasting state, the value range of the blood ketone may be [0.05, 0.5]; in the fasting state, the value range of the uric acid may be [0.18, 0.42]; and in the non-fasting state, the value range of the uric acid may be [0.18, 0.5]. Units of the foregoing value ranges are all mmol/L (mmol/L).

It may be understood that the embodiment shown in Table 2 is merely an example. In embodiments of this application, the electronic device 100 may further store value ranges of more or fewer physiological parameters than those shown in the embodiment in Table 2, or store value ranges of physiological parameters different from those in the foregoing embodiment, and the value ranges of the physiological parameters may alternatively be different from the foregoing value ranges. This is not limited in this application. In addition, the user status may alternatively include more or fewer user statuses than those in the foregoing embodiment, or include user statuses different from those in the foregoing embodiment. In addition, in some other embodiments, the electronic device 100 may further store a correspondence between a value range and a factor like a gender, an age, or a disease of the user. This is not limited in this application.

The electronic device 100 may determine the value range based on any one or more factors such as the user status, the user age, or the user gender.

The following describes a manner of determining the evaluation result.

In a possible implementation, the electronic device 100 may store the value range of the physiological parameter of the user, and determine the evaluation result based on the physiological parameter 1 of the user and the value range of the corresponding physiological parameter. In another possible implementation, the electronic device 100 may alternatively determine, based on a correspondence between a value range and one or more factors such as a gender, an age, and a user status of the user, a value range that is of a physiological parameter of the user and within which the user currently falls, and determine the evaluation result based on a calibrated value of the physiological parameter.

In some embodiments, the evaluation result may include normal and abnormal. When the physiological parameter 1 is within the value range, the electronic device 100 may determine that the evaluation result is that the physiological parameter is normal. When the physiological parameter 1 is not within the value range, the electronic device 100 may determine that the evaluation result is that the physiological parameter is abnormal. Optionally, when the evaluation result is abnormal, the evaluation result may be further subdivided into any one or more of the following: high, low, excessively high, excessively low, and the like. This is not limited in this application.

In some embodiments, if it is determined that the evaluation result is abnormal (or it is determined that the evaluation result is excessively high, excessively low, or the like in abnormal), the electronic device 100 may output a warning. A manner of outputting the warning may include but is not limited to any one or more of the following: display displaying, voice broadcast, vibration, indicator blinking, and the like.

The following describes a manner of determining the historical curve.

In some embodiments, the electronic device 100 may determine a historical curve of a physiological parameter based on a currently measured calibrated value of the physiological parameter. In some other embodiments, the electronic device 100 may alternatively determine a historical curve of a physiological parameter based on a currently measured calibrated value of the physiological parameter and a previously measured calibrated value of the physiological parameter. The electronic device 100 may store a measurement moment of each calibrated value of the physiological parameter, and the electronic device 100 may determine the historical curve of the physiological parameter based on different calibrated values corresponding to different measurement moments.

The following describes a manner of determining the reference suggestion.

In a possible implementation, the electronic device 100 may determine the reference suggestion based on the evaluation result. For example, if the evaluation result is normal, the reference suggestion may be suggesting the user to keep a current living habit, or suggesting the user to exercise more, keep a good living schedule, or the like. If the evaluation result is abnormal, the reference suggestion may be suggesting the user to change a bad living habit, reduce intake of greasy food, or the like.

In another possible implementation, the electronic device 100 may further determine the reference suggestion based on the user information. For example, when the electronic device 100 determines, based on the user information, that the user is in a high-altitude area, the reference suggestion may include "Oxygen is thin in the current area, and intense exercise needs to be reduced". For another example, when the electronic device 100 determines, based on the user information, that duration in which the user is in the fasting state is greater than specific duration (for example, 4 hours), the reference suggestion may include "You are in a hunger state currently. Eat as soon as possible", or the like. It may be understood that this embodiment is merely an example for describing The electronic device 100 may determine the reference suggestion based on the user information. In embodiments of this application, the electronic device 100 may further determine, based on the user information, content different from the reference suggestion. This is not limited in this application.

S606: The electronic device 100 determines that a monitoring condition 4 is satisfied, and sends information 4 to the electronic device 200, where the information 4 is used to request the electronic device 200 to send an electrocardiography signal to the electronic device 100.

In some embodiments, the monitoring condition 4 may include but is not limited to any one or more of the following: An operation of enabling an electrocardiography function by the user is received, a start instruction 2 sent by another electronic device is received, it is detected that the physiological status of the user is abnormal (for example, the blood glucose is not within a preset blood glucose value range), it is detected that the psychological status of the user is abnormal (for example, the user is frightened), it is detected that the user is in the exercise state, it is detected that the user has insomnia, it is detected that the body posture of the user is abnormal (for example, the user falls down), it is detected that the exercise equipment of the user is abnormal, it is detected that the position of the user is within the preset area range (for example, the user is in the high-altitude area), and the like.

For a determining manner of each condition in the monitoring condition 4, refer to related content in step S601. Details are not described herein again.

In some other embodiments, the monitoring condition 4 may be the same as the monitoring condition 3. To be specific, when the electronic device 100 determines that the monitoring condition 3 is satisfied, the electronic device 100 may perform step S602 and step S608.

S607: The electronic device 200 obtains the electrocardiography signal.

In some embodiments, the electronic device 200 may start to obtain the electrocardiography signal in response to the information 4.

In some other embodiments, the electronic device 200 may alternatively periodically obtain the electrocardiography signal before receiving the information 4.

For a manner in which the electronic device 200 obtains the electrocardiography signal, refer to related content of step S503 shown in FIG. 5A. Details are not described herein again.

S608: The electronic device 200 sends the electrocardiography signal to the electronic device 100.

In some embodiments, the electronic device 200 may send the electrocardiography signal to the electronic device 100 in response to the information 4.

In some other embodiments, the electronic device 200 periodically sends the electrocardiography signal to the electronic device 100 before receiving the information 4. In this case, step S608 may not be performed. In addition, in this case, the monitoring condition 4 may be used to trigger the electronic device 100 to perform step S609.

S609: The electronic device 100 outputs the electrocardiography signal.

The electronic device 100 may output the electrocardiography signal in one or more manners such as display displaying, audio broadcast, vibration, and indicator blinking.

The electronic device 100 may further output the electrocardiography signal in the electrocardiogram. Optionally, after outputting the electrocardiogram (or when outputting the electrocardiogram), the electronic device 100 may further output one or more of the following content: one or more electrocardiography indicators, an evaluation result of the electrocardiography indicator, an evaluation result of the electrocardiography function, a value range of the electrocardiography indicator, the user status, and the like. The value range of the electrocardiography indicator is a normal range of the electrocardiography indicator. The user status is the current status of the user, for example, a resting state, the exercise state, the high-altitude state, or the sleep state. The evaluation result of the electrocardiography indicator indicates whether the electrocardiography indicator is within a preset value range of the electrocardiography indicator. The evaluation result of the electrocardiography function indicates whether a heart function of the user is normal.

For specific content and a determining manner of the electrocardiography indicator, refer to the related descriptions in step S504 shown in FIG. 5A. The value range of the electrocardiography indicator may be prestored in the electronic device 100. The evaluation result of the electrocardiography indicator may be determined based on the electrocardiography indicator and the value range of the electrocardiography indicator. For a specific manner, refer to the related descriptions in step S605. The evaluation result of the electrocardiography function may be determined based on evaluation results of one or more electrocardiography indicators. For a manner of determining the user status, refer to the related descriptions in step S605. Details are not described herein again.

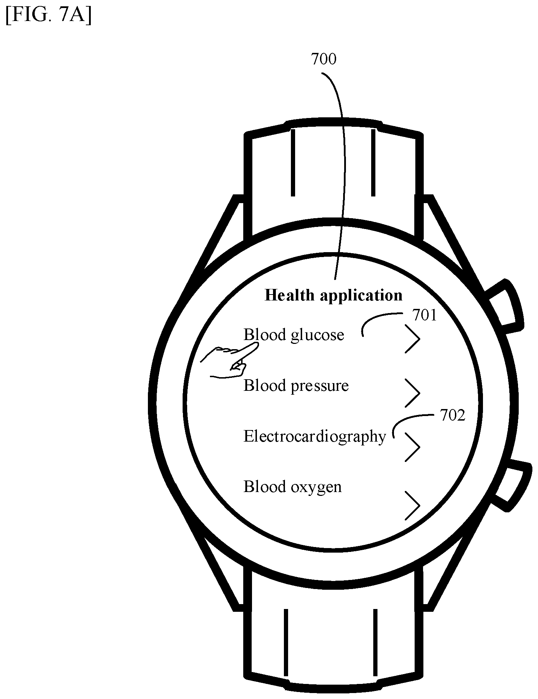

In some application scenarios, the physiological parameter 1 may be blood glucose. For a diagram of an output interface of blood glucose, refer to FIG. 7A to FIG. 7F.

For example, as shown in FIG. 7A, the electronic device 100 displays a health application interface 700. The health application interface 700 may include one or more entries, for example, a blood glucose entry 701, a blood pressure entry, an electrocardiography entry 702, and a blood oxygen entry. Each entry may be used to trigger the electronic device 100 to display a corresponding physiological parameter monitoring interface.

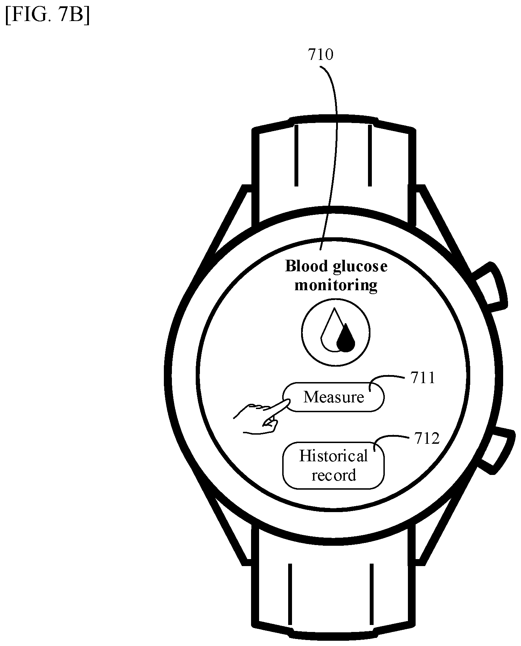

The electronic device 100 may receive a tap operation performed by a user on the blood glucose entry 701, and display, in response to the tap operation, a blood glucose monitoring interface 710 shown in FIG. 7B.

As shown in FIG. 7B, the blood glucose monitoring interface 710 may include a measure control 711 and a historical record control 712. The measure control 711 may be used to trigger the electronic device 100 to determine and output blood glucose of the user, and the historical record control 712 is used to trigger the electronic device 100 to display a previous blood glucose measurement record (including measurement time and blood glucose of the user).

After the electronic device 100 receives and responds to a tap operation performed by the user on the measure control 711, and determines the blood glucose of the user based on physiological data sent by the electronic device 200, the electronic device 100 may display an output interface 720 shown in FIG. 7C.

As shown in FIG. 7C, the output interface 720 may include blood glucose 721, and the blood glucose 721 indicates a blood glucose value of the user. Optionally, the output interface 720 may further include but is not limited to any one or more of the following: an evaluation result 722, a value range 723, a user status 724, and a historical curve control 725. The evaluation result 722 may indicate whether the blood glucose of the user is normal. It can be learned from FIG. 7C that the evaluation result 722 is a horizontal line, which may indicate that the blood glucose value of the user is within a preset value range, in other words, the blood glucose of the user is normal. In some embodiments, if the blood glucose value of the user is greater than the preset value range, the evaluation result may alternatively be an upward arrow; or if the blood glucose value of the user is less than the preset value range, the evaluation result may alternatively be a downward arrow. It may be understood that a manner of displaying the evaluation result 722 shown in FIG. 7C is merely an example. In embodiments of this application, the evaluation result may alternatively be indicated by using different symbols, texts, or the like. This is not limited in this application. The value range 723 indicates a reference range of the blood glucose of the user, namely, a normal value range of the blood glucose of the user in a current user status. The user status 724 may indicate the current user status, for example, a fasting state. In some embodiments, the value range 723 may vary with the user status. The historical curve control 725 may be used to trigger the electronic device 100 to display a historical curve of the blood glucose. The historical curve indicates a relationship between a blood glucose value of the user and time in a past period.

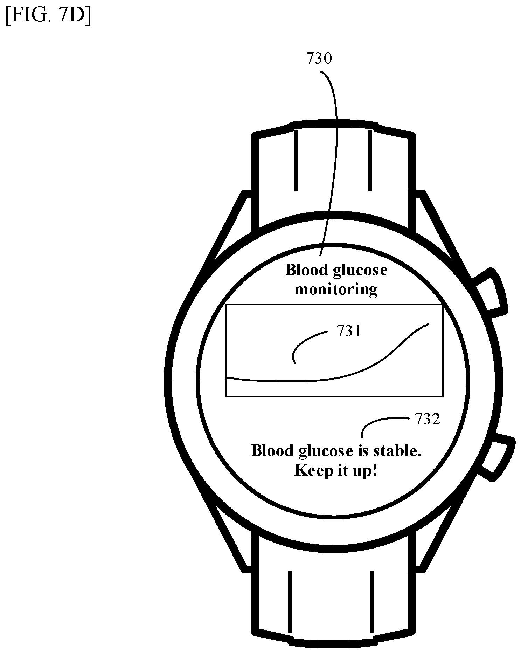

The electronic device 100 may receive a tap operation performed by the user on the historical curve control 725, and display, in response to the tap operation, a historical curve interface 730 shown in FIG. 7D. Alternatively, the electronic device 100 may receive a swipe-up operation performed by the user on the output interface 720, and display, on the output interface 720 in response to the swipe-up operation, content such as a historical curve shown in FIG. 7D.

As shown in FIG. 7D, the historical curve interface 730 may include a historical curve 731, and optionally, may further include a reference suggestion 732. The historical curve 731 may indicate a relationship between a blood glucose value of the user and time in a past period. The reference suggestion 732 may indicate the user to maintain or restore the blood glucose to a normal range. For example, the reference suggestion 732 may include text "Blood glucose is stable. Keep it up!" It may be understood that the reference suggestion 732 shown in FIG. 7D is merely an example. In embodiments of this application, the reference suggestion 732 may alternatively have an output form different from that in the foregoing embodiment, or may include more or less content than that in the foregoing embodiment, or include content different from that in the foregoing embodiment. This is not limited in this application.

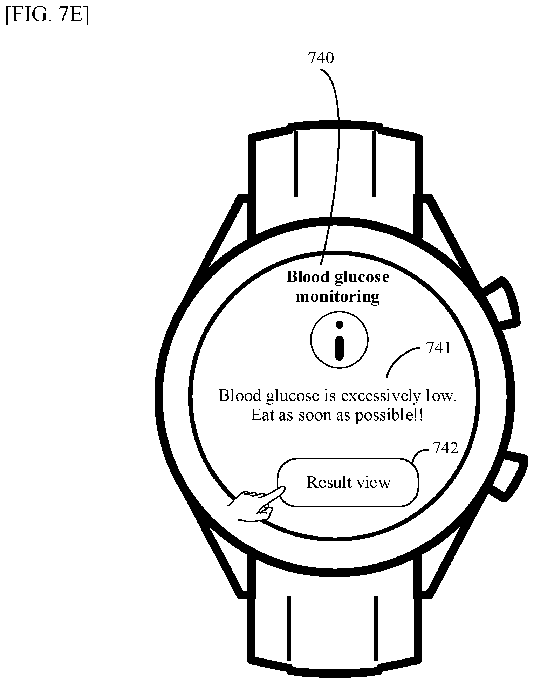

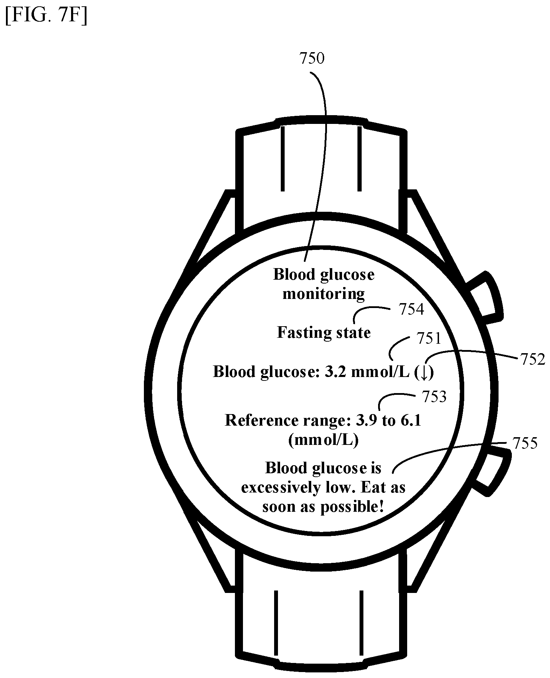

In some other embodiments, the electronic device 100 may receive and respond to the tap operation performed by the user on the measure control 711, and after determining the blood glucose (namely, the blood glucose value) of the user based on the physiological data, if the blood glucose of the user is abnormal, the electronic device 100 may optionally display a warning interface 740 shown in FIG. 7E, or display an output interface 750 shown in FIG. 7F.

As shown in FIG. 7E, the warning interface 740 may include a warning 741, and optionally, may further include a result view control 742. The warning 741 may be used to inform the user that the current blood glucose is abnormal. For example, the warning 741 may include text "Blood glucose is excessively low. Eat as soon as possible!!" The result view control 742 may be used to trigger the electronic device 100 to display the blood glucose value.

The electronic device 100 may receive a tap operation performed by the user on the result view control 742, and display, in response to the tap operation, the output interface 750 shown in FIG. 7F. In some embodiments, the electronic device 100 may alternatively display the output interface 750 shown in FIG. 7F when detecting that display duration of the warning interface 740 is greater than preset duration (for example, 10 seconds or 15 seconds).

As shown in FIG. 7F, the output interface 750 may include blood glucose 751, and the blood glucose 751 may be a blood glucose value of the user. Optionally, the output interface 750 may further include but is not limited to any one or more of the following: an evaluation result 752, a value range 753, a user status 754, a reference suggestion 755, and the like. The evaluation result 752 may indicate whether the blood glucose of the user is normal. It can be learned from FIG. 7F that the evaluation result 752 is a downward arrow, and may indicate that the blood glucose value of the user is less than a preset value range, that is, the blood glucose of the user is abnormal. The value range 753 indicates a reference range of the blood glucose of the user, namely, a value range of the blood glucose of the user in a healthy state. The user status 754 may indicate the current user status, for example, a fasting state. In some embodiments, the value range 753 may vary with the user status. The output interface 750 may further include the reference suggestion 755. For example, the reference suggestion 755 may include text "Blood glucose is excessively low. Eat as soon as possible!" It may be understood that, in some other embodiments, a historical blood glucose curve (or a historical curve control) or the like may be further displayed in the output interface 750. The historical curve indicates a relationship between a blood glucose value of the user and time in a past period. This is not limited in this application.

It may be understood that the embodiment shown in FIG. 7A to FIG. 7F is merely two examples. In embodiments of this application, the output physiological parameter may alternatively be another physiological parameter (for example, blood ketone or uric acid), a device for outputting the physiological parameter may alternatively be the electronic device 200 or another electronic device, and content displayed in the output interface may further include more or less content than that in the foregoing embodiment, or include content different from that in the foregoing embodiment. This is not limited in this application.

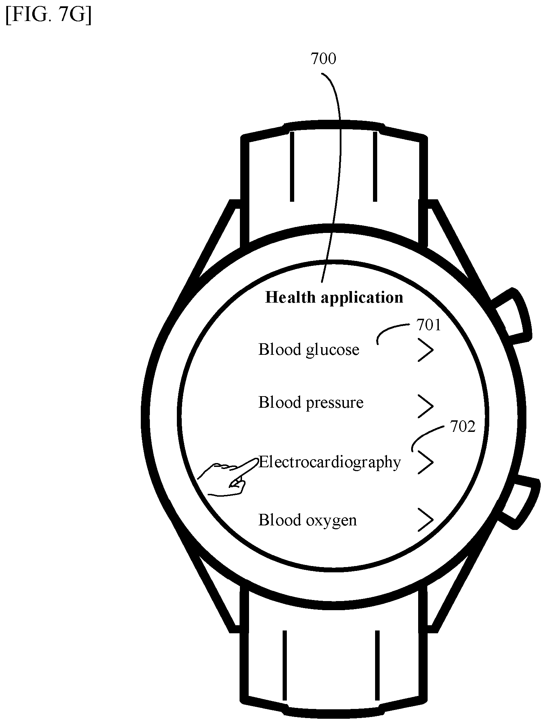

FIG. 7G to FIG. 7J are diagrams of a group of output interfaces of electrocardiography signals according to an embodiment of this application.

For example, as shown in FIG. 7G, the electronic device 100 may display the health application interface 700. The health application interface 700 may include one or more entries, for example, the blood glucose entry 701, the blood pressure entry, the electrocardiography entry 702, and the blood oxygen entry. Each entry may be used to trigger the electronic device 100 to display a corresponding physiological parameter monitoring interface.

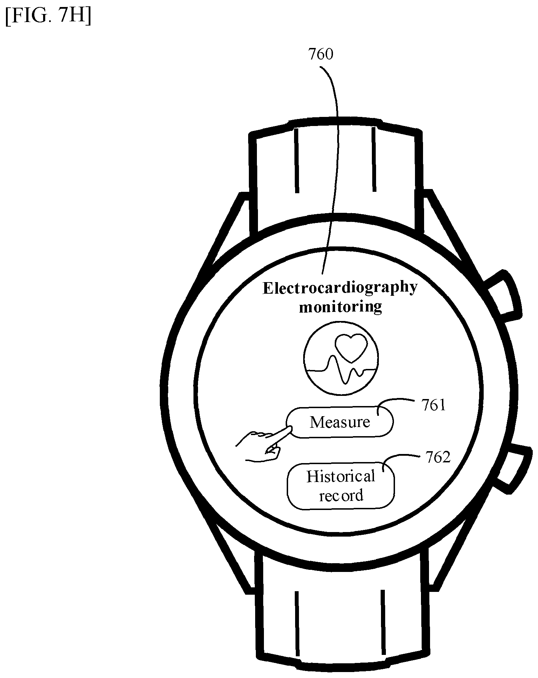

The electronic device 100 may receive a tap operation performed by the user on the electrocardiography entry 702, and display, in response to the tap operation, an electrocardiography monitoring interface 760 shown in FIG. 7H.

As shown in FIG. 7H, the electrocardiography monitoring interface 760 may include a measure control 761 and a historical record control 762. The measure control 761 may be used to trigger the electronic device 100 to determine and output an electrocardiography signal of the user, and the historical record control 762 is used to trigger the electronic device 100 to display a previous electrocardiography signal measurement record.

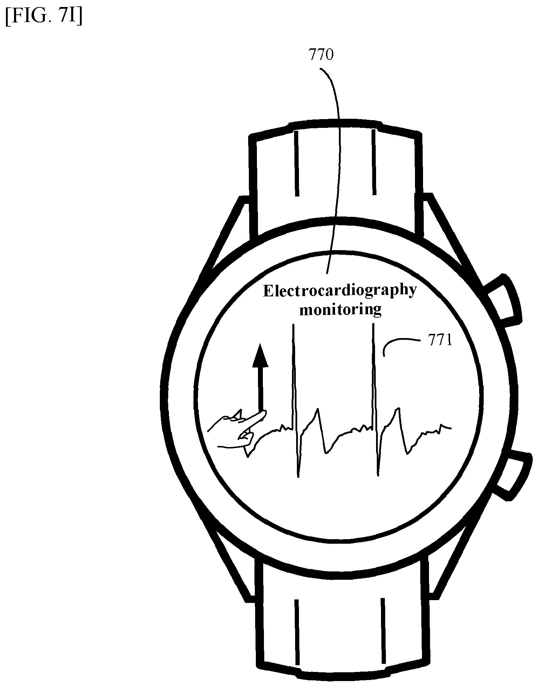

After the electronic device 100 receives and responds to a tap operation performed by the user on the measure control 761, and determines an electrocardiogram of the user based on the electrocardiography signal sent by the electronic device 200, the electronic device 100 may display an output interface 770 shown in FIG. 7I.

As shown in FIG. 7I, the output interface 770 may include an electrocardiogram 771, and the electrocardiogram 771 may be a waveform diagram of the electrocardiography signal.

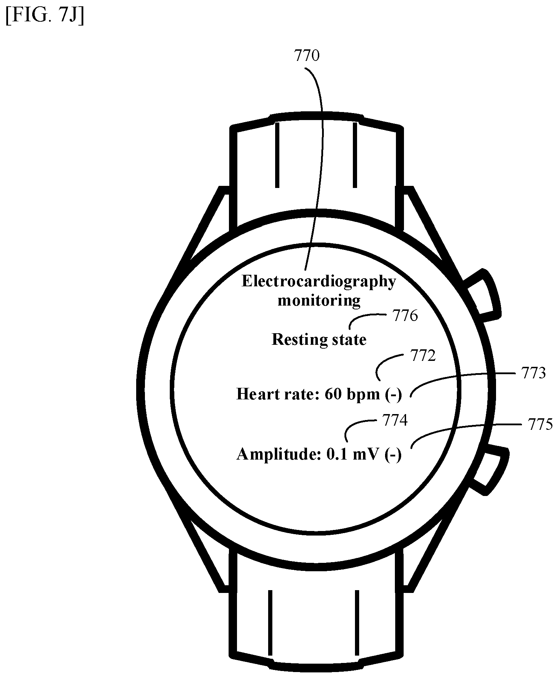

In some embodiments, the electronic device 100 may further receive and respond to a swipe-up operation performed by the user on the output interface 770. As shown in FIG. 7J, the electronic device 100 may display, in the output interface 770, any one or more of the following content: a heart rate 772, a heart rate evaluation result 773, an amplitude 774, an amplitude evaluation result 775, a user status 776, and the like. The heart rate 772 indicates a heart rate of the user. The heart rate evaluation result 773 indicates whether the heart rate of the user is normal. The amplitude 774 indicates an amplitude of the electrocardiography signal. The amplitude evaluation result 775 indicates whether the amplitude of the electrocardiography signal is normal. The user status 776 indicates a current user status, for example, a resting state (or an exercise state). The user status may affect the electrocardiography signal. It can be learned from FIG. 7J that both the heart rate evaluation result 773 and the amplitude evaluation result 775 are horizontal lines, which may indicate that the electrocardiogram of the user is within a preset value range, in other words, the electrocardiography of the user is normal. For other specific content of the heart rate evaluation result 773 and the amplitude evaluation result 775, refer to related content of the evaluation result 722 in the embodiment shown in FIG. 7C. Details are not described herein again.

It may be understood that the embodiment shown in FIG. 7G to FIG. 7J is merely an example. In embodiments of this application, the output interface of the electrocardiography signal may further include more or less content than that in the foregoing embodiment, or include content different from that in the foregoing embodiment, for example, include electrocardiography indicators such as P wave duration and a QT interval. This is not limited in this application.

In some application scenarios, when detecting that a value of the physiological parameter 1 is abnormal, the electronic device 100 may alternatively prompt the user to perform electrocardiography monitoring.

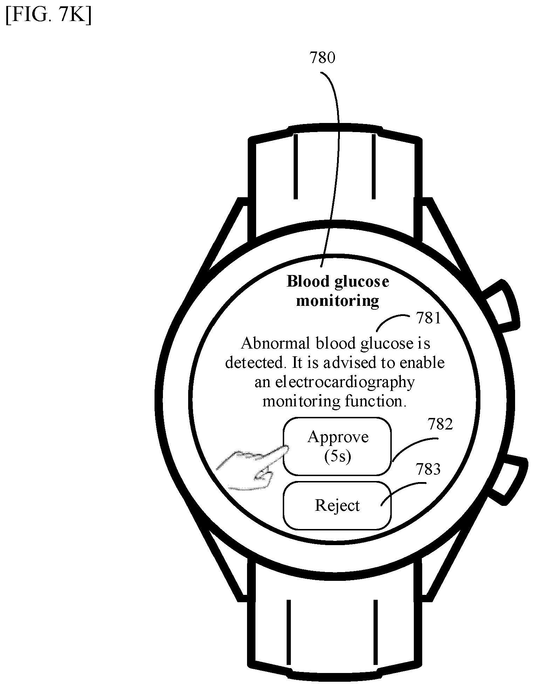

For example, the physiological parameter 1 is blood glucose. FIG. 7K shows a prompt interface 780 of the electronic device 100 when the blood glucose is abnormal.

As shown in FIG. 7K, the prompt interface 780 may include a prompt 781, an approve control 782, and a reject control 783. The prompt 781 may be used to prompt the user to perform electrocardiography monitoring. The prompt 781 may include text, for example, "Abnormal blood glucose is detected. It is advised to enable an electrocardiography monitoring function". The approve control 782 may be used to trigger the electronic device 100 to send information 4 to the electronic device 200. The information 4 is used to request the electronic device 200 to send the electrocardiography signal to the electronic device 100. Optionally, a countdown (for example, 5 seconds or 3 seconds) may be further displayed on the approve control 782. When the countdown ends and the electronic device 100 does not receive an operation of the user, the electronic device 100 may send the information 4 to the electronic device 200. The reject control 783 may be used to trigger the electronic device 100 to stop displaying the prompt interface 780, and optionally, may further trigger the electronic device 100 to output content such as a calibrated value of the blood glucose.

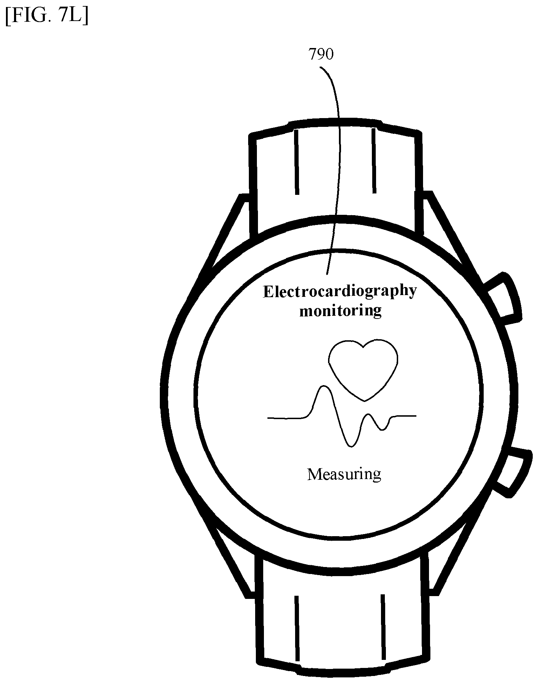

The electronic device 100 may receive a tap operation performed by the user on the approve control 782, and display, in response to the tap operation, an electrocardiography monitoring interface 790 shown in FIG. 7L. The electrocardiography monitoring interface 790 is used to inform the user that the electronic device 100 is obtaining the electrocardiography signal. In some embodiments, after receiving the electrocardiography signal sent by the electronic device 200, the electronic device 100 may display the output interface 770 shown in FIG. 7I.

It may be understood that FIG. 7K and FIG. 7L are merely examples for description. When the value of the physiological parameter 1 is abnormal, the user may be prompted to enable electrocardiography monitoring. In embodiments of this application, the electronic device 100 may alternatively directly redirect to an electrocardiography monitoring interface when detecting that the value of the physiological parameter 1 (for example, blood glucose or blood ketone) is abnormal, and display the electrocardiography signal after obtaining the electrocardiography signal. This is not limited in this application.

In some other application scenarios, the electronic device 100 may have a Health Glance function. When detecting that the user enables the Health Glance function, the electronic device 100 may output one or more physiological parameters and an electrocardiography signal after determining the one or more physiological parameters and the electrocardiography signal.

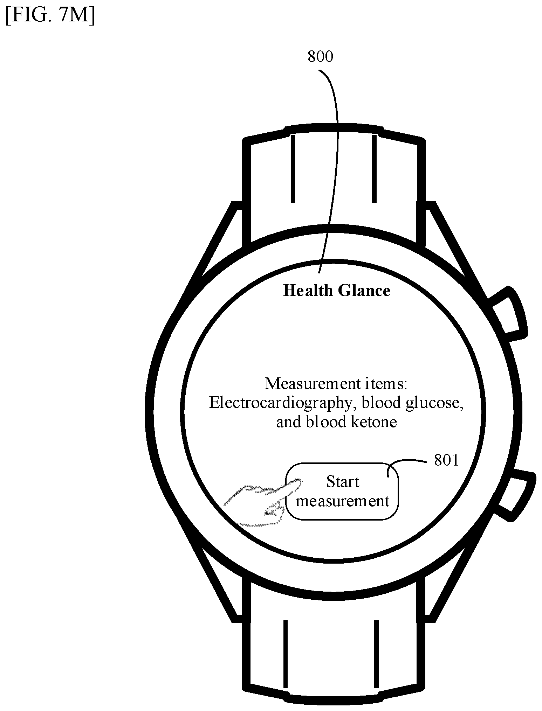

For example, as shown in FIG. 7M, the electronic device 100 may display a Health Glance interface 800. The Health Glance interface 800 may include a measurement control 801, and the measurement control 801 may be used to trigger the electronic device 100 to output one or more physiological parameters and an electrocardiography signal. Optionally, a measurement item may be further displayed in the Health Glance interface 800, and the measurement item may be used to notify the user of an item that can be monitored by using the Health Glance function. For example, in the embodiment shown in FIG. 7M, measurement items may include electrocardio, blood glucose, and blood ketone. After the Health Glance function is enabled, the electronic device 100 may output the electrocardio, blood glucose, blood ketone, and the like.

Optionally, before displaying the Health Glance interface 800, the electronic device 100 may further display an interface for the user to select a measurement item. The electronic device 100 may receive an operation of selecting a measurement item by the user, and display, in response to the operation, the Health Glance interface 800 shown in FIG. 7M.

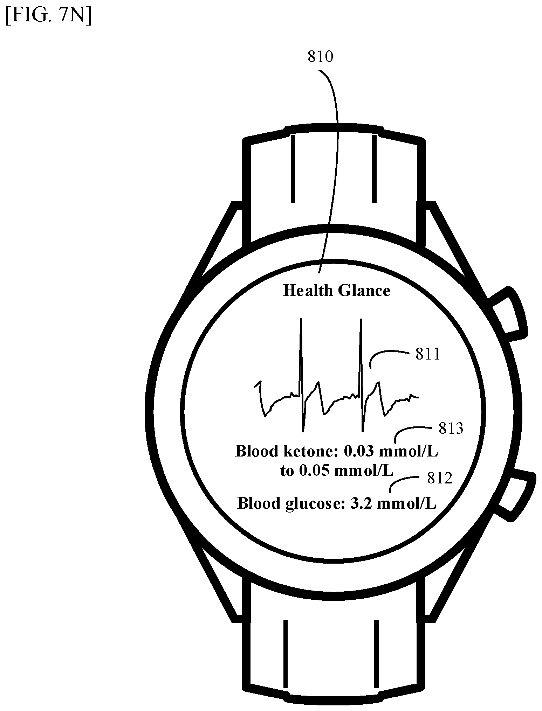

After the electronic device 100 receives and responds to a tap operation performed by the user on the measurement control 801, and determines blood glucose, blood ketone, and the electrocardiography signal, the electronic device 100 may display an output interface 810 shown in FIG. 7N. The output interface 810 may include an electrocardiogram 811, and may further include a blood glucose value 812 and a blood ketone value 813. Optionally, the output interface 810 may further include but is not limited to any one or more of the following: an electrocardiography indicator, a value range of the blood glucose, a value range of the blood ketone, an evaluation result of the blood glucose, an evaluation result of the blood ketone, and the like.

It may be understood that the embodiment shown in FIG. 7M and FIG. 7N is merely an example for description. The electronic device 100 may output the one or more physiological parameters and the electrocardiography signal at the same time. In embodiments of this application, monitoring items of the Health Glance function may alternatively be different from those in the foregoing embodiment, and the electronic device 100 may alternatively output a plurality of physiological parameters at the same time. This is not limited in this application.

The following describes functional modules of the electronic device 200 according to embodiments of this application.

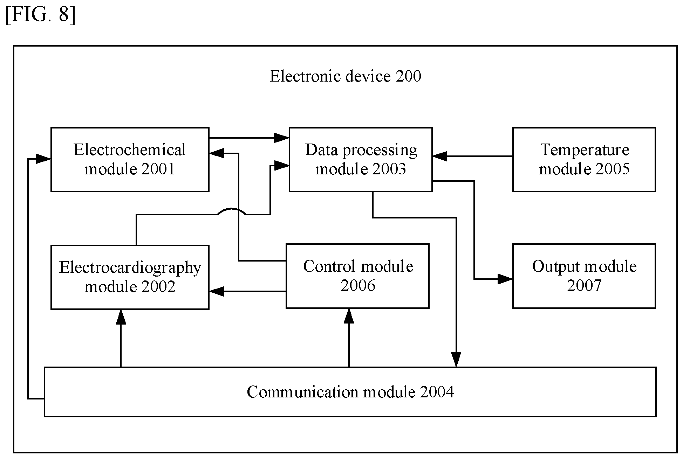

FIG. 8 is a diagram of functional modules of a measurement system 10 according to an embodiment of this application.

As shown in FIG. 8, the electronic device 200 may include an electrochemical module 2001, an electrocardiography module 2002, a data processing module 2003, a communication module 2004, and the like, and optionally, may further include any one or more of the following: a temperature module 2005, a control module 2006, an output module 2007, and the like.

The electrochemical module 2001 may obtain current data of one or more physiological parameters (for example, blood glucose, blood ketone, blood lactic acid, and uric acid). In some embodiments, the electrochemical module 2001 may receive a message N1 sent by the control module 2006 or the communication module 2004, and start to obtain, in response to the message N1, current data of one or more physiological parameters specified in the message N1. The electrochemical module 2001 may send the current data to the data processing module 2003.

The electrocardiography module 2002 may obtain an electrocardiography signal, and send the electrocardiography signal to the data processing module 2003. In some embodiments, the electrocardiography signal may alternatively receive a message N2 sent by the control module 2006 or the communication module 2004, and obtain the electrocardiography signal in response to the message N2.

The data processing module 2003 may determine the one or more physiological parameters based on the current data sent by the electrochemical module 2001. In some embodiments, the data processing module 2003 may further determine an electrocardiography indicator and/or an electrocardiogram based on the electrocardiography signal sent by the electrocardiography module 2002. In some embodiments, the data processing module 2003 may further calibrate a value of a physiological parameter based on a body temperature sent by the temperature module 2005. The data processing module 2003 may send any one or more of the one or more physiological parameters, the electrocardiography indicator, the electrocardiogram, and the like to the output module 2007 or the communication module 2004.

The communication module 2004 may receive data (for example, the any one or more of the one or more physiological parameters, the electrocardiography indicator, and the electrocardiogram) sent by the data processing module 2003, and send the received data to an electronic device 100. In some embodiments, the communication module 2004 may receive and respond to information 1 sent by another electronic device (for example, the electronic device 100), and send the message N1 to the electrochemical module 2001. The message N1 is used to indicate the electrochemical module 2001 to start to obtain the current data of the one or more physiological parameters specified in the message N1. The communication module 2004 may further receive and respond to information 2 sent by the another electronic device (for example, the electronic device 100), and send the message N2 to the electrocardiography module 2002. The message N2 is used to indicate the electrocardiography module 2002 to start to obtain the electrocardiography signal. In some other embodiments, the communication module 2004 may further receive the information 1 sent by the another electronic device (for example, the electronic device 100), and send the information 1 to the control module 2006. The communication module 2004 may further receive the information 2 sent by the another electronic device (for example, the electronic device 100), and send the information 2 to the control module 2006.

The control module 2006 may further determine whether a monitoring condition 1 or a monitoring condition 2 is satisfied. When the monitoring condition 1 is satisfied, the control module 2006 may send the message N1 to the electrochemical module 2001. The message N1 is used to indicate the electrochemical module 2001 to start to obtain the current data of the one or more physiological parameters specified in the message N1. When the monitoring condition 2 is satisfied, the control module 2006 may send the message N2 to the electrochemical module 2001. The message N2 is used to indicate the electrocardiography module 2002 to start to obtain the electrocardiography signal. In some embodiments, the monitoring condition 1 may include that the information 1 sent by the communication module 2004 is received, and the monitoring condition 2 may include that the information 2 sent by the communication module 2004 is received.

The temperature module 2005 may measure a body temperature of a user or a living being, and send the body temperature to the data processing module 2003.

The output module 2007 may receive the data (for example, the any one or more of the one or more physiological parameters, the electrocardiography indicator, and the electrocardiogram) sent by the data processing module 2003, and output the received data in any one or more manners such as voice broadcast, display displaying, vibration, and indicator blinking.

It may be understood that the embodiment shown in FIG. 8 is merely an example. In embodiments of this application, the electronic device 200 may further include more or fewer functional modules than those in the foregoing embodiment, or include functional modules different from those in the foregoing embodiment, or combine the plurality of functional modules into one functional module, or split any one of the functional modules into a plurality of functional modules. This is not limited in this application.

The following describes functional modules of the measurement system 10 according to embodiments of this application.

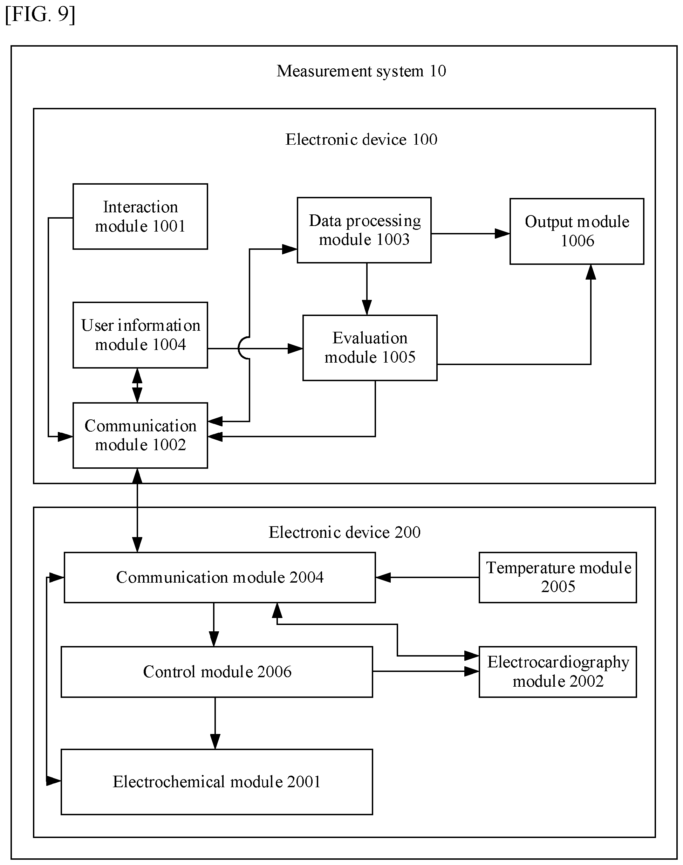

FIG. 9 is a diagram of functional modules of a measurement system 10 according to an embodiment of this application.

As shown in FIG. 9, the measurement system 10 may include an electronic device 100 and an electronic device 200. The electronic device 100 may include a communication module 1002, a data processing module 1003, an output module 1006, and the like. Optionally, the electronic device 100 may further include but is not limited to any one or more of the following: an interaction module 1001, a user information module 1004, and an evaluation module 1005. The electronic device 200 may include an electrochemical module 2001, an electrocardiography module 2002, and a communication module 2004. Optionally, the electronic device 200 may further include any one or more of the following: a temperature module 2005, a control module 2006, and the like.

The interaction module 1001 may receive and respond to an operation of a user, for example, an operation of enabling a physiological monitoring function or an operation of enabling an electrocardiography function. The interaction module 1001 may send a message N3 to the communication module 1002 in response to the operation of enabling the physiological monitoring function by the user. The message N3 may be used to indicate the communication module 1002 to send information 3 to the electronic device 200, and the information 3 is used to request to obtain physiological data of one or more specified physiological parameters.

The communication module 1002 may communicate with another electronic device (for example, the electronic device 200). In some embodiments, the communication module 1002 may receive the message N3 and send the information 3 to the communication module 2004 in the electronic device 200 in response to the message N3. The information 3 is used to request to obtain physiological data. The communication module 1002 may further receive the physiological data sent by the communication module 2004 in the electronic device 200, and send the physiological data to the data processing module 1003. In some embodiments, the communication module 1002 may further receive a physiological parameter (namely, a value of the physiological parameter) sent by the data processing module 1003, receive an evaluation result and/or a reference suggestion sent by the evaluation module 1005, and send any one or more of the physiological parameter, the evaluation result, and the reference suggestion to the another electronic device.

The data processing module 1003 may determine the physiological parameter, for example, blood glucose or blood ketone, based on the physiological data sent by the communication module 1002. After determining the physiological parameter, the data processing module 1003 may send the physiological parameter to the output module 1006 or the communication module 1002. In some embodiments, the data processing module 1003 may further send the physiological parameter to the evaluation module 1005.

The user information module 1004 may obtain user information. In some embodiments, the user information module 1004 may collect user information. In other embodiments, the user information module 1004 may receive user information obtained by the communication module 1002 from the another electronic device. In some embodiments, the user information module 1004 may determine a user status based on the user information, and send the user status to the evaluation module 1005. In some embodiments, the user information module 1004 may further determine whether the user information satisfies a monitoring condition (for example, a monitoring condition 3 or a monitoring condition 4). When the monitoring condition 3 is satisfied, the user information module 1004 may send a message N4 to the communication module 1002. The message N4 may be used to indicate the communication module 1002 to send the information 3 to the electronic device 200. When the monitoring condition 4 is satisfied, the user information module 1004 may send a message N5 to the communication module 1002. The message N5 may be used to indicate the communication module 1002 to send information 4 to the electronic device 200.

The evaluation module 1005 may store a value range of the physiological parameter. The evaluation module 1005 may determine the evaluation result based on the physiological parameter and the value range of the physiological parameter. The evaluation result indicates whether the physiological parameter is normal. In some embodiments, the evaluation module 1005 may further receive the user status sent by the user information module 1004, and determine the evaluation result based on the physiological parameter and a relationship between the user status and the value range of the physiological parameter. In some other embodiments, the evaluation module 1005 may further determine the reference suggestion based on content such as the user status and/or the evaluation result. The reference suggestion is used to indicate the user to maintain (or restore) the physiological parameter to a normal range. The evaluation module 1005 may send the evaluation result and/or the reference suggestion to the output module 1006, or send the evaluation result and/or the reference suggestion to the another electronic device through the communication module 1002.

The output module 1006 may receive and output the physiological parameter sent by the data processing module 1003, may further receive and output the evaluation result and/or the reference suggestion sent by the evaluation module 1005, and the like.

In the electronic device 200, the communication module 2004 may communicate with the electronic device 100. In some embodiments, the communication module 2004 may receive the information 3 sent by the communication module 1002, and send the information 3 to the control module 2006 or send a message N6 to the electrochemical module 2001. The message N6 is used to indicate the electrochemical module 2001 to send current data of one or more specified physiological parameters to the communication module 2004. In some embodiments, the communication module 2004 may receive the information 4 sent by the communication module 1002, and send the information 4 to the control module 2006 or send a message N7 to the electrocardiography module 2002. The message N7 is used to indicate the electrocardiography module 2002 to send an electrocardiography signal to the communication module 2004.

The electrochemical module 2001 may obtain current data of one or more physiological parameters (for example, blood glucose, blood ketone, blood lactic acid, and uric acid). In some embodiments, the electrochemical module 2001 may receive the message N6 sent by the control module 2006 or the communication module 2004, and start to obtain, in response to the message N6, the current data of the one or more physiological parameters specified in the message N6. In some other embodiments, the electrochemical module 2001 may send the current data of the one or more physiological parameters to the communication module 2004 in response to the message N6 sent by the control module 2006 or the communication module 2004.

The electrocardiography module 2002 may obtain the electrocardiography signal. In some embodiments, the electrocardiography signal may alternatively receive the message N7 sent by the control module 2006 or the communication module 2004, and start to obtain the electrocardiography signal in response to the message N7. In some other embodiments, the electrocardiography module 2002 may send the electrocardiography signal to the communication module 2004 in response to the message N7 sent by the control module 2006 or the communication module 2004.

The control module 2006 may further determine whether a monitoring condition 1 or a monitoring condition 2 is satisfied. When the monitoring condition 1 is satisfied, the control module 2006 may send a message N1 to the electrochemical module 2001. The message N1 is used to indicate the electrochemical module 2001 to start to obtain current data of one or more physiological parameters specified in the message N1. When the monitoring condition 2 is satisfied, the control module 2006 may send a message N2 to the electrochemical module 2001. The message N2 is used to indicate the electrocardiography module 2002 to start to obtain the electrocardiography signal. In some embodiments, the monitoring condition 1 may include that the information 3 sent by the communication module 2004 is received, and the monitoring condition 2 may include that the information 4 sent by the communication module 2004 is received.

The temperature module 2005 may measure a body temperature of a user or a living being, and send the body temperature to the communication module 2004.

In some other embodiments, the electronic device 200 may further include but is not limited to any one or more of the following: a data processing module, an evaluation module, an output module, and the like. For function descriptions of the one or more modules, refer to function descriptions of related modules in the embodiment shown in FIG. 8 or FIG. 9. Details are not described herein again. It should be noted that if the electronic device 200 includes the data processing module, the physiological data sent by the communication module 2004 to the electronic device 100 may alternatively be a value of the physiological parameter (for example, a measured value of the physiological parameter or a calibrated value of the physiological parameter).

It may be understood that the embodiment shown in FIG. 9 is merely an example. In embodiments of this application, the measurement system 10 may include more or fewer functional modules than those in the foregoing embodiment, or include functional modules different from those in the foregoing embodiment, or combine the plurality of functional modules into one functional module, or split any one of the functional modules into a plurality of functional modules. This is not limited in this application.

It should be noted that the measurement method provided in embodiments of this application may be used to measure not only physiological parameters and an electrocardiography signal of a user, but also physiological parameters and an electrocardiography signal of another living being (for example, a pet, a poultry, a livestock, or an endangered animal). This is not limited in this application.

For ease of subsequent description, the electronic device 100 and the electronic device 200 may be collectively referred to as an apparatus. It should be understood that division into units in the apparatus is merely logical function division. During actual implementation, all or a part of the units may be integrated into one physical entity, or may be physically separated. In addition, the units in the apparatus may be implemented in a form of software invoked by a processor. For example, the apparatus includes a processor, the processor is connected to a memory, the memory stores instructions, and the processor invokes the instructions stored in the memory, to implement any one of the foregoing methods or implement functions of each unit in the apparatus. The processor is, for example, a general-purpose processor like a central processing unit (central processing unit, CPU), or a microprocessor. The memory is a memory inside the apparatus or a memory outside the apparatus. Alternatively, the units in the apparatus may be implemented in a form of hardware circuits, and functions of a part or all of the units may be implemented by designing the hardware circuits. The hardware circuits may be understood as one or more processors. For example, in an implementation, the hardware circuit is an application-specific integrated circuit (application-specific integrated circuit, ASIC), and functions of a part or all of the foregoing units are implemented by designing a logical relationship between elements in the circuits. For another example, in another implementation, the hardware circuits may be implemented by using a programmable logic device (programmable logic device, PLD). A field programmable gate array (field programmable gate array, FPGA) is used as an example. The field programmable gate array may include a large quantity of logic gate circuits. A configuration file is used to configure a connection relationship between the logic gate circuits, to implement functions of a part or all of the foregoing units. All units in the foregoing apparatus may be implemented in a form of software invoked by a processor; or all units may be implemented in a form of hardware circuit; or a part of units may be implemented in a form of software invoked by a processor, and a remaining part may be implemented in a form of hardware circuit.

In embodiments of this application, the processor is a circuit with a data processing capability. In an implementation, the processor may be a circuit with an instruction reading and running capability, for example, a CPU, a microprocessor, a graphics processing unit (graphics processing unit, GPU) (which may be understood as a microprocessor), or a digital signal processor (digital signal processor, DSP). In another implementation, the processor may implement a specific function based on a logical relationship of hardware circuits. The logical relationship of the hardware circuits is fixed or reconfigurable. For example, the processor is a hardware circuit implemented by an ASIC or a PLD, for example, an FPGA. In a reconfigurable hardware circuit, a process in which the processor loads a configuration document to implement hardware circuit configuration may be understood as a process in which the processor loads instructions to implement functions of a part or all of the units. In addition, the processor may alternatively be a hardware circuit designed for artificial intelligence, and may be understood as an ASIC, for example, a neural-network processing unit (Neural-Network Processing Unit, NPU), a tensor processing unit (tensor processing unit, TPU), or a deep learning processing unit (deep learning processing unit, DPU).

It can be learned that each unit in the foregoing apparatus may be one or more processors (or processing circuits) configured to implement the foregoing method, for example, a CPU, a GPU, an NPU, a TPU, a DPU, a microprocessor, a DSP, an ASIC, an FPGA, or a combination of at least two of these processor forms.

In addition, all or a part of the units in the foregoing apparatus may be integrated, or may be implemented independently. In an implementation, these units are integrated together and implemented in a form of a system-on-a-chip (system-on-a-chip, SOC). The SOC may include at least one processor, configured to implement any one of the methods or implement functions of the units in the apparatus. Types of the at least one processor may be different. For example, the at least one processor includes a CPU and an FPGA, a CPU and an artificial intelligence processor, or a CPU and a GPU.

The following describes a possible physical entity structure of an electronic device 300 according to an embodiment of this application.

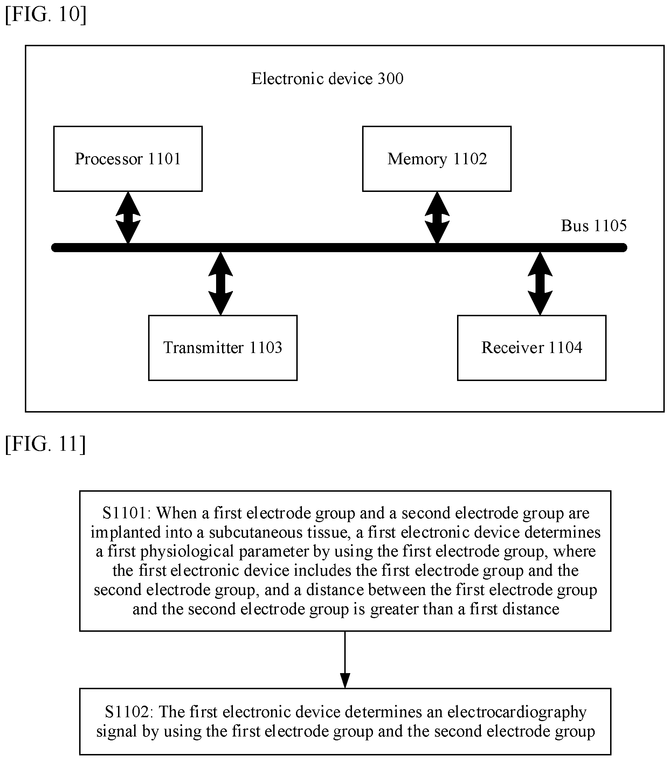

For example, FIG. 10 is a diagram of a physical entity structure of the electronic device 300 according to this embodiment of this application.

As shown in FIG. 10, the electronic device 300 may be any one of the electronic device 100 and the electronic device 200 in the foregoing embodiments. The electronic device 300 may include a processor 1101 and a memory 1102, and optionally, may further include a transmitter 1103 and a receiver 1104. The processor 1101, the memory 1102, the transmitter 1103, and the receiver 1104 may be connected to each other or connected to each other through a bus 1105.

For example, the memory 1102 is configured to store a computer program and data of the electronic device 300. The memory 1102 may include but is not limited to a random access memory (random access memory, RAM), a read-only memory (read-only memory, ROM), an erasable programmable read-only memory (erasable programmable read-only memory, EPROM), a compact disc read-only memory (compact disc read-only memory, CD-ROM), or the like.

Software or program code required for all or a part of functions of the electronic device 300 in the foregoing method embodiment is stored in the memory 1102.

In a possible implementation, if software or program code required for a part of the functions is stored in the memory 1102, in addition to invoking the program code in the memory 1102 to implement the part of the functions, the processor 1101 may cooperate with other components (for example, the transmitter 1103 and the receiver 1104) to jointly complete another function (for example, a data receiving or sending function) described in the method embodiments.

The transmitter 1103 and the receiver 1104 are configured to support the electronic device 300 in communication, for example, receiving or sending data or a signal.

For example, the processor 1101 may be a CPU, a GPU, an NPU, a TPU, a DPU, a microprocessor, a DSP, an ASIC, an FPGA, or a combination of at least two of these processor forms. The processor 1101 may be configured to read a program stored in the memory 1102, to perform an operation performed by the electronic device 300 in any one of the foregoing embodiments.

For specific operations and beneficial effects of the units in the electronic device 300 shown in FIG. 10, refer to corresponding descriptions in the foregoing method embodiments. Details are not described herein again.

It may be understood that the embodiment shown in FIG. 10 is merely an example. In embodiments of this application, the electronic device 300 may further include more or fewer components than those in the foregoing embodiment shown in FIG. 10, or include components different from those in the embodiment shown in FIG. 10. This is not limited in this application.

The following describes a chip system according to an embodiment of this application.

This application further provides a chip system. The chip system includes at least one processor, configured to implement functions related to either the electronic device 100 or the electronic device 200 in any one of the foregoing embodiments.

In a possible design, the chip system further includes a memory, the memory is configured to store program instructions and data, and the memory is located inside or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may also be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in embodiments of this application.

For example, the chip system may be a field programmable gate array (field programmable gate array, FPGA), an application-specific integrated circuit (application-specific integrated circuit, ASIC), a system on chip (system on chip, SoC), a central processing unit (central processing unit, CPU), a network processor (network processor, NP), a digital signal processing circuit (digital signal processor, DSP), a microcontroller (microcontroller unit, MCU), a programmable controller (programmable logic device, PLD), or another integrated chip.

It may be understood that the chip system is merely an example. In embodiments of this application, the chip system may alternatively include more or fewer components than those in the foregoing embodiment, or include components different from those in the foregoing embodiment. This is not limited in this application.

FIG. 11 is a schematic flowchart of a measurement method according to an embodiment of this application.

As shown in FIG. 11, a specific procedure of the measurement method may include the following steps.

S1101: When a first electrode group and a second electrode group are implanted into a subcutaneous tissue, a first electronic device determines a first physiological parameter by using the first electrode group, where the first electronic device includes the first electrode group and the second electrode group, and a distance between the first electrode group and the second electrode group is greater than a first distance.

The first electronic device may be the electronic device 200 in the foregoing embodiment. The first electrode group may be the electrode group 1 in the foregoing embodiment, and the second electrode group may be the electrode group 2 in the foregoing embodiment.

The first distance may be a preset distance, for example, three centimeters. When the distance between the first electrode group and the second electrode group is greater than the first distance, the first electrode group and the second electrode group may measure an electrocardiography signal.

In a possible implementation, the first physiological parameter may include but is not limited to any one or more of the following: blood glucose, blood ketone, uric acid, blood lactic acid, and the like.

For a specific manner in which the first electronic device determines the first physiological parameter by using the first electrode group, refer to the related descriptions in the embodiment shown in FIG. 5A. Details are not described herein again.

In a possible implementation, the first electrode group includes a first working electrode and a first counter electrode, the second electrode group includes a second working electrode, and the first counter electrode forms a loop with the first working electrode. Determining the first physiological parameter by using the first electrode group specifically includes: determining the first physiological parameter by using the first working electrode and the first counter electrode. Determining the electrocardiography signal by using the first electrode group and the second electrode group specifically includes: determining the electrocardiography signal by using the first working electrode and the second working electrode, or determining the electrocardiography signal by using the first counter electrode and the second working electrode.

In this way, the first electrode group may form a dual-electrode system, and the first physiological parameter may be measured by using the dual-electrode system. The first working electrode (or the first counter electrode) in the first electrode group and any electrode in the second electrode group may be respectively used as an LA electrode and an RA electrode, to measure the electrocardiography signal.

In a possible implementation, the first electrode group includes a first working electrode and a first counter electrode, the second electrode group includes a second working electrode, the first counter electrode forms a loop with the first working electrode, and the first working electrode or the first counter electrode is connected to a right leg drive circuit. Determining the first physiological parameter by using the first electrode group specifically includes: determining the first physiological parameter by using the first working electrode and the first counter electrode. Determining the electrocardiography signal by using the first electrode group and the second electrode group specifically includes: determining the electrocardiography signal by using the first working electrode, the first counter electrode, and the second working electrode.

An electrode connected to the right leg drive circuit may be used as a right leg drive RLD electrode. The right leg drive circuit may be configured to cancel a common-mode signal. For specific circuit composition of the right leg drive circuit, refer to related content in the foregoing embodiment shown in FIG. 4C or the like. Details are not described herein again.

In this way, the first electrode group may form a dual-electrode system, and the first physiological parameter may be measured by using the dual-electrode system. The first working electrode and the first counter electrode in the first electrode group and any electrode in the second electrode group may be respectively used as an LA electrode, an RLD electrode, and an RA electrode to measure the electrocardiography signal.

In a possible implementation, the first electrode group includes a first working electrode, a first reference electrode, and a first counter electrode, the first reference electrode is configured to control a voltage of the first working electrode, and the first counter electrode forms a loop with the first working electrode. Determining the first physiological parameter by using the first electrode group specifically includes: generating a first current by using the first working electrode; and determining the first physiological parameter based on the first current.

In this way, the first electrode group may form a three-electrode system, and the first current is generated and conducted by using the three-electrode system.

In a possible implementation, the second electrode group includes a second working electrode, a second reference electrode, and a second counter electrode, the second reference electrode is configured to control a voltage of the second working electrode, and the second counter electrode is configured to form a loop with the second working electrode. The method further includes: generating a second current by using the second working electrode; and determining a second physiological parameter based on the second current.

In this way, the second electrode group may form a three-electrode system, and the second current is generated and conducted by using the three-electrode system.

For example, the embodiments shown in FIG. 4C to FIG. 4J are used as examples. In the foregoing embodiments, the first working electrode may be the working electrode W1, the second working electrode may be the working electrode W2, the first counter electrode may be the counter electrode C1, the second counter electrode may be the counter electrode C2, the first reference electrode may be the reference electrode R1, and the second reference electrode may be the reference electrode R2.

S1102: The first electronic device determines the electrocardiography signal by using the first electrode group and the second electrode group.

In a possible implementation, determining the electrocardiography signal by using the first electrode group and the second electrode group specifically includes: determining the electrocardiography signal by using any electrode in the first electrode group and any electrode in the second electrode group.

One electrode in the first electrode group and one electrode in the second electrode group may be respectively selected as a left arm electrode and a right arm electrode. In this way, the electrocardiography signal may be measured by using the left arm electrode and the right arm electrode.

In a possible implementation, determining the electrocardiography signal by using the first electrode group and the second electrode group specifically includes: determining the electrocardiography signal by using any two electrodes in the first electrode group and any electrode in the second electrode group; or determining the electrocardiography signal by using any electrode in the first electrode group and any two electrodes in the second electrode group.

One electrode in the first electrode group and one electrode in the second electrode group may be respectively selected as a left arm electrode and a right arm electrode. A right leg drive electrode may be an electrode in the first electrode group, or may be an electrode in the second electrode group. In this way, the electrocardiography signal may be measured by using the left arm electrode, the right arm electrode, and the right leg drive electrode. The right leg drive electrode is configured to cancel a common-mode signal by using a right leg drive circuit.

In this way, an electrochemical electrode in the first electrode group may be configured to measure the first physiological parameter, and may also be configured to measure the electrocardiography signal.

In a possible implementation, determining the electrocardiography signal by using the any two electrodes in the first electrode group and the any electrode in the second electrode group specifically includes: determining the electrocardiography signal by using the first counter electrode, the first working electrode, and the second reference electrode when the first counter electrode is connected to the right leg drive circuit.

In a possible implementation, determining the electrocardiography signal by using the any electrode in the first electrode group and the any two electrodes in the second electrode group specifically includes: determining the electrocardiography signal by using the second counter electrode, the second working electrode, and the first reference electrode when the second counter electrode is connected to the right leg drive circuit.

It should be noted that the foregoing two implementations are merely two examples. In this application, another electrode in the first electrode group and another electrode in the second electrode group may be selected to measure the electrocardiography signal. This is not limited in this application.

In a possible implementation, the first electrode group further includes a first electrocardiography electrode and a second electrocardiography electrode, the second electrode group further includes a third electrocardiography electrode, and the second electrocardiography electrode is connected to a right leg drive circuit. Determining the electrocardiography signal by using the first electrode group and the second electrode group specifically includes: determining the electrocardiography signal by using the first electrocardiography electrode, the second electrocardiography electrode, and the third electrocardiography electrode.

In this way, the electrocardiography signal may be measured by using a separately disposed electrocardiography electrode.

According to the measurement method provided in this embodiment of this application, the electrocardiography signal of a user may be measured anytime and anywhere, and physiological parameters such as blood glucose, blood ketone, blood lactic acid, and uric acid may be further measured.

In a possible implementation, the first electronic device further includes a first microneedle sensor and a second microneedle sensor, the first microneedle sensor includes the first electrode group, and the second microneedle sensor includes the second electrode group.

For example, the first microneedle sensor may be the microneedle sensor 303 shown in FIG. 3A and FIG. 3B, and the second microneedle sensor may be the microneedle sensor 304 shown in FIG. 3A and FIG. 3B.

In some embodiments, the microneedle sensor may be a sensor whose form is similar to that of a microneedle, and a plurality of electrodes may be disposed inside the microneedle sensor. In this way, the first electrode group and the second electrode group may be disposed in the microneedle sensor.

In a possible implementation, the first electronic device further includes a first array sensor and a second array sensor, the first array sensor includes the first electrode group, and the second array sensor includes the second electrode group.

In some embodiments, an array sensor may be a sensor including a plurality of electrodes arranged in an array.

For example, the first array sensor may be the array sensor 307 shown in FIG. 3D and FIG. 3E, and the second array sensor may be the array sensor 308 shown in FIG. 3D and FIG. 3E.

In this way, the first electrode group and the second electrode group may be separately disposed in the array sensor in an array.

In a possible implementation, the first electronic device further includes a first microneedle sensor and a second array sensor, the first microneedle sensor includes the first electrode group, and the second array sensor includes the second electrode group.

In this way, the first electrode group may be disposed in the microneedle sensor, and the second electrode group may be disposed in the array sensor in an array.

In a possible implementation, before determining the first physiological parameter by using the first electrode group, the method further includes: determining that a first condition is satisfied, where the first condition includes any one or more of the following: first information sent by a second electronic device is received, where the first information is used to indicate the first electronic device to determine the first physiological parameter; it is detected that the first electrode group and the second electrode group are implanted into the subcutaneous tissue; and it is detected that the electrocardiography signal is abnormal.

In this way, the first condition may be a trigger condition for measuring the first physiological parameter.

The first condition may be the monitoring condition 1 in the embodiment shown in FIG. 5A.

In a possible implementation, before determining the electrocardiography signal by using the first electrode group and the second electrode group, the method further includes: determining that a second condition is satisfied, where the second condition includes any one or more of the following: second information sent by the second electronic device is received, where the second information is used to indicate the first electronic device to determine the electrocardiography signal; it is detected that the first electrode group and the second electrode group are implanted into the subcutaneous tissue; and it is detected that the first physiological parameter is not within a first range.

In this way, the second condition may be a trigger condition for determining the electrocardiography signal.

The second condition may be the monitoring condition 2 in the embodiment shown in FIG. 5A.

In a possible implementation, after determining the first physiological parameter by using the first electrode group, the method further includes: outputting the first physiological parameter, or sending the first physiological parameter to the second electronic device.

In this way, the first physiological parameter may be output, or the first physiological parameter may be output by another electronic device.

In a possible implementation, after determining the electrocardiography signal by using the first electrode group and the second electrode group, the method further includes: outputting the electrocardiography signal, or sending the electrocardiography signal to the second electronic device.

In this way, the electrocardiography signal may be output, or the electrocardiography signal may be output by another electronic device.

The second electronic device may be the electronic device 100 in the foregoing embodiment.

For a specific manner of outputting the first physiological parameter and the electrocardiography signal, refer to related content in the embodiments shown in FIG. 5A and FIG. 7A to FIG. 7N. Details are not described herein again.

The implementations of this application may be randomly combined to achieve different technical effects.

All or a part of the foregoing embodiments may be implemented by using software, hardware, firmware, or any combination thereof. When software is used to implement the embodiments, all or a part of the embodiments may be implemented in a form of computer program product. The computer program product includes one or more computer instructions. When the computer program instructions are loaded and executed on a computer, the procedure or functions according to this application are all or partially generated. The computer may be a general-purpose computer, a dedicated computer, a computer network, or other programmable apparatuses. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

A person of ordinary skill in the art may understand that all or a part of the processes of the methods in the foregoing embodiments may be implemented by a computer program instructing related hardware. The program may be stored in a computer-readable storage medium. When the program is executed, the processes in the foregoing method embodiments are performed. The foregoing storage medium includes any medium that can store program code, for example, a ROM, a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, improvement, or the like made based on the disclosure of the present invention shall fall within the protection scope of the present invention.

## Claims

1. A measurement method, applied to a first electronic device, wherein the first electronic device comprises a first electrode group and a second electrode group, and a distance between the first electrode group and the second electrode group is greater than a first distance; and
when the first electrode group and the second electrode group are implanted into a subcutaneous tissue, the method comprises:
determining a first physiological parameter by using the first electrode group; and
determining an electrocardiography signal by using the first electrode group and the second electrode group.

2. The method according to claim 1, wherein the first electrode group comprises a first working electrode and a first counter electrode, the second electrode group comprises a second working electrode, the first counter electrode forms a loop with the first working electrode, and the first working electrode or the first counter electrode is connected to a right leg drive circuit;
determining the first physiological parameter by using the first electrode group specifically comprises:
determining the first physiological parameter by using the first working electrode and the first counter electrode; and
determining the electrocardiography signal by using the first electrode group and the second electrode group specifically comprises:
determining the electrocardiography signal by using the first working electrode, the first counter electrode, and the second working electrode.

3. The method according to claim 1, wherein the first electrode group comprises a first working electrode, a first reference electrode, and a first counter electrode, the first reference electrode is configured to control a voltage of the first working electrode, and the first counter electrode forms a loop with the first working electrode; and
determining the first physiological parameter by using the first electrode group specifically comprises:
generating a first current by using the first working electrode; and
determining the first physiological parameter based on the first current.

4. The method according to claim 3, wherein the second electrode group comprises a second working electrode, a second reference electrode, and a second counter electrode, the second reference electrode is configured to control a voltage of the second working electrode, and the second counter electrode is configured to form a loop with the second working electrode; and the method further comprises:
generating a second current by using the second working electrode; and
determining the second physiological parameter based on the second current.

5. The method according to claim 4, wherein determining the electrocardiography signal by using the first electrode group and the second electrode group specifically comprises:
determining the electrocardiography signal by using any two electrodes in the first electrode group and any electrode in the second electrode group;
or
determining the electrocardiography signal by using any electrode in the first electrode group and any two electrodes in the second electrode group.

6. The method according to claim 5, wherein determining the electrocardiography signal by using the any two electrodes in the first electrode group and the any electrode in the second electrode group specifically comprises:
determining the electrocardiography signal by using the first counter electrode, the first working electrode, and the second reference electrode when the first counter electrode is connected to a right leg drive circuit.

7. The method according to claim 3 or 4, wherein the first electrode group further comprises a first electrocardiography electrode and a second electrocardiography electrode, the second electrode group further comprises a third electrocardiography electrode, and the second electrocardiography electrode is connected to a right leg drive circuit; and
determining the electrocardiography signal by using the first electrode group and the second electrode group specifically comprises:
determining the electrocardiography signal by using the first electrocardiography electrode, the second electrocardiography electrode, and the third electrocardiography electrode.

8. The method according to any one of claims 1 to 7, wherein the first electronic device further comprises a first microneedle sensor and a second microneedle sensor, the first microneedle sensor comprises the first electrode group, and the second microneedle sensor comprises the second electrode group.

9. The method according to any one of claims 1 to 7, wherein the first electronic device further comprises a first array sensor and a second array sensor, the first array sensor comprises the first electrode group, and the second array sensor comprises the second electrode group.

10. The method according to any one of claims 1 to 9, wherein before determining the first physiological parameter by using the first electrode group, the method further comprises:
determining that a first condition is satisfied, wherein the first condition comprises any one or more of the following: first information sent by a second electronic device is received, wherein the first information is used to indicate the first electronic device to determine the first physiological parameter; it is detected that the first electrode group and the second electrode group are implanted into the subcutaneous tissue; and it is detected that the electrocardiography signal is abnormal.

11. The method according to any one of claims 1 to 10, wherein before determining the electrocardiography signal by using the first electrode group and the second electrode group, the method further comprises:
determining that a second condition is satisfied, wherein the second condition comprises any one or more of the following: second information sent by the second electronic device is received, wherein the second information is used to indicate the first electronic device to determine the electrocardiography signal; it is detected that the first electrode group and the second electrode group are implanted into the subcutaneous tissue; and it is detected that the first physiological parameter is not within a first range.

12. The method according to any one of claims 1 to 11, wherein after determining the first physiological parameter by using the first electrode group, the method further comprises:
outputting the first physiological parameter, or sending the first physiological parameter to the second electronic device.

13. The method according to any one of claims 1 to 12, wherein after determining the electrocardiography signal by using the first electrode group and the second electrode group, the method further comprises:
outputting the electrocardiography signal, or sending the electrocardiography signal to the second electronic device.

14. The method according to any one of claims 1 to 13, wherein the first physiological parameter comprises any one or more of the following: blood glucose, blood ketone, uric acid, and blood lactic acid.

15. An electronic device, wherein the electronic device is a first electronic device, and comprises a first electrode group and a second electrode group, wherein a distance between the first electrode group and the second electrode group is greater than a first distance;
the first electrode group is configured to determine a first physiological parameter when the first electrode group is implanted into a subcutaneous tissue;
the second electrode group is configured to determine a second physiological parameter when the second electrode group is implanted into the subcutaneous tissue; and
the first electrode group and the second electrode group are further configured to determine an electrocardiography signal when the first electrode group and the second electrode group are implanted into the subcutaneous tissue.

16. The electronic device according to claim 15, wherein the first electrode group comprises a first working electrode and a first counter electrode, the second electrode group comprises a second working electrode and a second counter electrode, the first counter electrode forms a loop with the first working electrode, and the second counter electrode forms a loop with the second working electrode;
that the first electrode group is configured to determine the first physiological parameter when the first electrode group is implanted into the subcutaneous tissue specifically comprises:
the first working electrode is configured to determine the first physiological parameter when the first working electrode is implanted into the subcutaneous tissue;
that the second electrode group is configured to determine the second physiological parameter when the second electrode group is implanted into the subcutaneous tissue specifically comprises: the second working electrode is configured to determine the second physiological parameter when the second working electrode is implanted into the subcutaneous tissue; and
that the first electrode group and the second electrode group are further configured to determine the electrocardiography signal when the first electrode group and the second electrode group are implanted into the subcutaneous tissue specifically comprises:
the first working electrode, the first counter electrode, and the second working electrode are further configured to determine the electrocardiography signal when the first working electrode, the first counter electrode, and the second working electrode are implanted into the subcutaneous tissue.

17. The electronic device according to claim 15, wherein the first electronic device further comprises a microcontroller unit MCU, the first electrode group comprises a first working electrode, a first reference electrode, and a first counter electrode, and the second electrode group comprises a second working electrode, a second reference electrode, and a second counter electrode;
that the first electrode group is configured to determine the first physiological parameter when the first electrode group is implanted into the subcutaneous tissue specifically comprises:
the first working electrode is configured to generate a first current when the first working electrode is implanted into the subcutaneous tissue;
the first reference electrode is configured to control a voltage of the first working electrode;
the first counter electrode is configured to form a loop with the first working electrode; and
the MCU is configured to determine the first physiological parameter based on the first current; and
that the second electrode group is configured to determine the second physiological parameter when the second electrode group is implanted into the subcutaneous tissue specifically comprises: the second working electrode is configured to generate a second current when the second working electrode is implanted into the subcutaneous tissue;
the second reference electrode is configured to control a voltage of the second working electrode; the second counter electrode is configured to form a loop with the second working electrode; and the MCU is configured to determine the second physiological parameter based on the second current.

18. The electronic device according to claim 17, wherein that the first electrode group and the second electrode group are further configured to determine the electrocardiography signal when the first electrode group and the second electrode group are implanted into the subcutaneous tissue specifically comprises:
any two electrodes in the first electrode group and any electrode in the second electrode group are configured to determine the electrocardiography signal when the first electrode group and the second electrode group are implanted into the subcutaneous tissue;
or
any electrode in the first electrode group and any two electrodes in the second electrode group are configured to determine the electrocardiography signal when the first electrode group and the second electrode group are implanted into the subcutaneous tissue.

19. The electronic device according to claim 18, wherein that the any two electrodes in the first electrode group and the any electrode in the second electrode group are configured to determine the electrocardiography signal when the first electrode group and the second electrode group are implanted into the subcutaneous tissue specifically comprises:
when the first counter electrode is connected to a right leg drive circuit, the first counter electrode, the first working electrode, and the second reference electrode are configured to determine the electrocardiography signal when the first electrode group and the second electrode group are implanted into the subcutaneous tissue.

20. The electronic device according to claim 17, wherein the first electrode group further comprises a first electrocardiography electrode and a second electrocardiography electrode, the second electrode group further comprises a third electrocardiography electrode, and the second electrocardiography electrode is connected to a right leg drive circuit; and
that the first electrode group and the second electrode group are further configured to determine the electrocardiography signal when the first electrode group and the second electrode group are implanted into the subcutaneous tissue specifically comprises:
the first electrocardiography electrode, the second electrocardiography electrode, and the third electrocardiography electrode are configured to determine the electrocardiography signal when the first electrode group and the second electrode group are implanted into the subcutaneous tissue.

21. The electronic device according to any one of claims 15 to 20, wherein the first electronic device further comprises a first microneedle sensor and a second microneedle sensor, the first microneedle sensor comprises the first electrode group, and the second microneedle sensor comprises the second electrode group.

22. The electronic device according to any one of claims 15 to 20, wherein the first electronic device further comprises a first array sensor and a second array sensor, the first array sensor comprises the first electrode group, and the second array sensor comprises the second electrode group.

23. The electronic device according to any one of claims 15 to 22, wherein the first electronic device further comprises a communication module;
the communication module is configured to send the first physiological parameter to a second electronic device; and
the communication module is further configured to send the electrocardiography signal to the second electronic device.

24. A measurement circuit, comprising a first electrode group, a second electrode group, a first electrochemical circuit module, a second electrochemical circuit module, an electrocardiography circuit module, and a microcontroller unit MCU, wherein
the first electrode group is connected to the first electrochemical circuit module, and the first electrode group is connected to the electrocardiography circuit module;
the second electrode group is connected to the second electrochemical circuit module, and the second electrode group is connected to the electrocardiography circuit module;
the MCU is connected to the first electrochemical circuit module, the second electrochemical circuit module, and the electrocardiography circuit module;
the first electrode group is configured to generate a first current signal;
the first electrode group is further configured to conduct the first current signal to the first electrochemical circuit module;
the first electrochemical circuit module is configured to determine a second current signal based on the first current signal;
the first electrochemical circuit module is further configured to conduct the second current signal to the MCU;
the MCU is configured to determine a first physiological parameter based on the second current signal;
the second electrode group is configured to generate a third current signal;
the second electrode group is further configured to conduct the third current signal to the second electrochemical circuit module;
the second electrochemical circuit module is configured to determine a fourth current signal based on the third current signal;
the second electrochemical circuit module is further configured to conduct the fourth current signal to the MCU;
the MCU is configured to determine a second physiological parameter based on the fourth current signal;
the first electrode group and the second electrode group are configured to obtain a first electrocardiography signal;
the first electrode group and the second electrode group are further configured to conduct the first electrocardiography signal to the electrocardiography circuit module;
the electrocardiography circuit module is configured to determine a second electrocardiography signal based on the first electrocardiography signal; and
the electrocardiography circuit module is further configured to conduct the second electrocardiography signal to the MCU.

25. The circuit according to claim 24, wherein the first electrode group comprises a first working electrode, a first reference electrode, and a first counter electrode; the first electrochemical circuit module comprises a first potentiostat circuit and a first transimpedance circuit; the first potentiostat circuit is configured to control voltages of the first working electrode and the first reference electrode; and the first transimpedance circuit is configured to amplify the first current signal; and
that the first electrode group is connected to the first electrochemical circuit module specifically comprises:
the first working electrode, the first reference electrode, and the first counter electrode are connected to the first potentiostat circuit; and
the first working electrode is connected to the first transimpedance circuit.

26. The circuit according to claim 25, wherein the second electrode group comprises a second working electrode, a second reference electrode, and a second counter electrode; the second electrochemical circuit module comprises a second potentiostat circuit and a second transimpedance circuit; the second potentiostat circuit is configured to control voltages of the second working electrode and the second reference electrode; and the second transimpedance circuit is configured to amplify the third current signal; and
that the second electrode group is connected to the second electrochemical circuit module specifically comprises:
the second working electrode, the second reference electrode, and the second counter electrode are connected to the second potentiostat circuit; and
the second working electrode is connected to the second transimpedance circuit.

27. The circuit according to claim 26, wherein the electrocardiography circuit module comprises a right leg drive circuit, an amplification circuit, and a filter circuit, the amplification circuit is connected to the filter circuit, the right leg drive circuit is configured to cancel a common-mode signal, the amplification circuit is configured to amplify the first electrocardiography signal, and the filter circuit is configured to perform filtering;
that the first electrode group is connected to the electrocardiography circuit module specifically comprises:
the first working electrode is connected to the amplification circuit, and the first counter electrode is connected to the right leg drive circuit;
that the second electrode group is connected to the electrocardiography circuit module specifically comprises:
the second reference electrode is connected to the amplification circuit;
that the first electrode group and the second electrode group are configured to obtain the first electrocardiography signal specifically comprises:
the first working electrode and the second reference electrode are configured to obtain the first electrocardiography signal;
that the first electrode group and the second electrode group are further configured to conduct the first electrocardiography signal to the electrocardiography circuit module specifically comprises:
the first working electrode and the second reference electrode are configured to conduct the first electrocardiography signal to the amplification circuit;
that the electrocardiography circuit module is configured to determine the second electrocardiography signal based on the first electrocardiography signal specifically comprises:
the amplification circuit is configured to: amplify the first electrocardiography signal, and then conduct an amplified first electrocardiography signal to the filter circuit; and
the filter circuit is configured to determine the second electrocardiography signal based on the amplified first electrocardiography signal; and
that the electrocardiography circuit module is further configured to conduct the second electrocardiography signal to the MCU specifically comprises:
the filter circuit is further configured to conduct the second electrocardiography signal to the MCU.

28. The circuit according to claim 26, wherein the first electrode group further comprises a first electrocardiography electrode and a second electrocardiography electrode, the second electrode group further comprises a third electrocardiography electrode, the electrocardiography circuit module comprises a right leg drive circuit, an amplification circuit, and a filter circuit, the amplification circuit is connected to the filter circuit, the right leg drive circuit is configured to cancel a common-mode signal, the amplification circuit is configured to amplify the first electrocardiography signal, and the filter circuit is configured to perform filtering;
that the first electrode group is connected to the electrocardiography circuit module specifically comprises:
the first electrocardiography electrode is connected to the amplification circuit, and the second electrocardiography electrode is connected to the right leg drive circuit;
that the second electrode group is connected to the electrocardiography circuit module specifically comprises:
the third electrocardiography electrode is connected to the amplification circuit;
that the first electrode group and the second electrode group are configured to conduct an electrocardiography signal to the electrocardiography circuit module specifically comprises:
the first electrocardiography electrode and the third electrocardiography electrode are configured to conduct the electrocardiography signal to the amplification circuit;
that the electrocardiography circuit module is configured to determine the second electrocardiography signal based on the first electrocardiography signal specifically comprises:
the amplification circuit is configured to: amplify the first electrocardiography signal, and then conduct an amplified first electrocardiography signal to the filter circuit; and
the filter circuit is configured to determine the second electrocardiography signal based on the amplified first electrocardiography signal; and
that the electrocardiography circuit module is further configured to conduct the second electrocardiography signal to the MCU specifically comprises:
the filter circuit is further configured to conduct the second electrocardiography signal to the MCU.

29. A measurement circuit, comprising a first electrode group, a second electrode group, a first multiplexer, a first electrochemical circuit module, an electrocardiography circuit module, and a microcontroller unit MCU, wherein the first multiplexer comprises a first group of input ports, a second group of input ports, and a first group of output ports, and the first multiplexer is configured to connect the first group of input ports or the second group of input ports;
the first electrode group is connected to the first group of input ports, and the first electrode group is connected to the electrocardiography circuit module;
the second electrode group is connected to the second group of input ports, and the second electrode group is connected to the electrocardiography circuit module;
the first group of output ports are connected to the first electrochemical circuit module;
the MCU is connected to the first electrochemical circuit module and the electrocardiography circuit module;
the first electrode group is configured to generate a first current signal;
the first electrode group is further configured to conduct the first current signal to the first multiplexer;
the first multiplexer is configured to conduct the first current signal to the first electrochemical circuit module when the first group of input ports are connected;
the first electrochemical circuit module is configured to determine a second current signal based on the first current signal;
the first electrochemical circuit module is further configured to send the second current signal to the MCU;
the MCU is configured to determine a first physiological parameter based on the second current signal;
the second electrode group is configured to generate a third current signal;
the second electrode group is further configured to conduct the third current signal to the first multiplexer;
the first multiplexer is configured to conduct the third current signal to the first electrochemical circuit module when the second group of input ports are connected;
the first electrochemical circuit module is further configured to determine a fourth current signal based on the third current signal;
the first electrochemical circuit module is further configured to conduct the fourth current signal to the MCU;
the MCU is configured to determine a second physiological parameter based on the fourth current signal;
the first electrode group and the second electrode group are configured to obtain a first electrocardiography signal;
the first electrode group and the second electrode group are further configured to conduct the first electrocardiography signal to the electrocardiography circuit module;
the electrocardiography circuit module is configured to determine a second electrocardiography signal based on the first electrocardiography signal; and
the electrocardiography circuit module is further configured to conduct the second electrocardiography signal to the MCU.

30. The circuit according to claim 29, wherein the first electrode group comprises a first working electrode, a first reference electrode, and a first counter electrode; the first group of input ports comprise a first input port, a second input port, and a third input port; the second electrode group comprises a second working electrode, a second reference electrode, and a second counter electrode; and the second group of input ports comprise a fourth input port, a fifth input port, and a sixth input port;
the first working electrode is connected to the first input port, the first reference electrode is connected to the second input port, and the first counter electrode is connected to the third input port; and
the second working electrode is connected to the fourth input port, the second reference electrode is connected to the fifth input port, and the second counter electrode is connected to the sixth input port.

31. The circuit according to claim 30, wherein the first group of output ports comprise a first output port, a second output port, and a third output port;
that the first multiplexer is configured to connect the first group of input ports specifically comprises:
the first output port is configured to be connected to the first input port, the second output port is configured to be connected to the second input port, and the third output port is configured to be connected to the third input port; and
that the first multiplexer is configured to connect the second group of input ports specifically comprises:
the first output port is configured to be connected to the fourth input port, the second output port is configured to be connected to the fifth input port, and the third output port is configured to be connected to the sixth input port.

32. The circuit according to claim 31, wherein the first electrochemical circuit module comprises a first potentiostat circuit and a first transimpedance circuit; the first potentiostat circuit is configured to control voltages of the first working electrode and the first reference electrode; and the first transimpedance circuit is configured to amplify the first current signal; and
that the first group of output ports are connected to the first electrochemical circuit module specifically comprises:
the first output port, the second output port, and the third output port are connected to the first potentiostat circuit; and
the first output port is connected to the first transimpedance circuit.

33. A chip system, used in a first electronic device, wherein the chip system comprises a processing circuit and an interface circuit, the interface circuit is configured to receive code instructions and transmit the code instructions to the processing circuit, and the processing circuit is configured to run the code instructions, to enable the chip system to perform the method according to any one of claims 1 to 14.

34. A readable storage medium, comprising instructions, wherein when the instructions are run on a first electronic device, the first electronic device is enabled to perform the method according to any one of claims 1 to 14.

## Amended claims

### Amended claims under Art. 19.1 PCT

1. A measurement method, applied to a first electronic device, wherein the first electronic device comprises a first electrode group and a second electrode group, and a distance between the first electrode group and the second electrode group is greater than a first distance; and when the first electrode group and the second electrode group are implanted into a subcutaneous tissue, the method comprises:
determining an electrocardiography signal by using the first electrode group and the second electrode group.

2. The method according to claim 1, comprising: determining a first physiological parameter by using the first electrode group, and/or determining a second physiological parameter by using the second electrode group, and/or determining a third physiological parameter by using the first electrode group and the second electrode group.

3. The method according to claim 1 or 2, wherein the first electrode group comprises a first working electrode and a first counter electrode, the second electrode group comprises a second working electrode, and the first counter electrode forms a loop with the first working electrode; and
determining the electrocardiography signal by using the first electrode group and the second electrode group specifically comprises:
determining the electrocardiography signal by using the first working electrode, the first counter electrode, and the second working electrode.

4. The method according to claim 1 or 2, wherein the first electrode group comprises a first working electrode, a first reference electrode, and a first counter electrode, and the second electrode group comprises a second working electrode, a second reference electrode, and a second counter electrode; and
determining the electrocardiography signal by using the first electrode group and the second electrode group specifically comprises:
determining the electrocardiography signal by using any two electrodes in the first electrode group and any electrode in the second electrode group; or
determining the electrocardiography signal by using any electrode in the first electrode group and any two electrodes in the second electrode group.

5. The method according to claim 4, wherein determining the electrocardiography signal by using the any two electrodes in the first electrode group and the any electrode in the second electrode group specifically comprises:
determining the electrocardiography signal by using the first counter electrode, the first working electrode, and the second reference electrode when the first counter electrode is connected to a right leg drive circuit.

6. The method according to claim 1 or 2, wherein the first electrode group comprises a first electrocardiography electrode and a second electrocardiography electrode, the second electrode group comprises a third electrocardiography electrode, and the second electrocardiography electrode is connected to a right leg drive circuit; and
determining the electrocardiography signal by using the first electrode group and the second electrode group specifically comprises:
determining the electrocardiography signal by using the first electrocardiography electrode, the second electrocardiography electrode, and the third electrocardiography electrode.

7. The method according to claim 6, wherein the first electrode group comprises a first working electrode and a first counter electrode, and the first counter electrode forms a loop with the first working electrode; and
determining the first physiological parameter by using the first electrode group specifically comprises:
determining the first physiological parameter by using the first working electrode and the first counter electrode;
or
the first electrode group comprises a first working electrode, a first reference electrode, and a first counter electrode, the first reference electrode is configured to control a voltage of the first working electrode, and the first counter electrode forms a loop with the first working electrode; and
determining the first physiological parameter by using the first electrode group specifically comprises:
generating a first current by using the first working electrode; and
determining the first physiological parameter based on the first current.

8. The method according to claim 6 or 7, wherein the second electrode group comprises a second working electrode and a second counter electrode, and the second counter electrode forms a loop with the second working electrode; and
determining the second physiological parameter by using the second electrode group specifically comprises:
determining the second physiological parameter by using the second working electrode and the second counter electrode;
or
the second electrode group comprises a second working electrode, a second reference electrode, and a second counter electrode, the second reference electrode is configured to control a voltage of the second working electrode, and the second counter electrode is configured to form a loop with the second working electrode; and the method further comprises:
generating a second current by using the second working electrode; and
determining the second physiological parameter based on the second current.

9. The method according to any one of claims 1 to 8, wherein the first electronic device further comprises a first microneedle sensor and a second microneedle sensor, the first microneedle sensor comprises the first electrode group, and the second microneedle sensor comprises the second electrode group.

10. The method according to any one of claims 1 to 8, wherein the first electronic device further comprises a first array sensor and a second array sensor, the first array sensor comprises the first electrode group, and the second array sensor comprises the second electrode group.

11. The method according to any one of claims 1 to 10, wherein before determining the first physiological parameter by using the first electrode group, the method further comprises: determining that a first condition is satisfied, wherein the first condition comprises any one or more of the following: first information sent by a second electronic device is received, wherein the first information is used to indicate the first electronic device to determine the first physiological parameter; it is detected that the first electrode group and the second electrode group are implanted into the subcutaneous tissue; and it is detected that the electrocardiography signal is abnormal.

12. The method according to any one of claims 1 to 11, wherein before determining the electrocardiography signal by using the first electrode group and the second electrode group, the method further comprises:
determining that a second condition is satisfied, wherein the second condition comprises any one or more of the following: second information sent by the second electronic device is received, wherein the second information is used to indicate the first electronic device to determine the electrocardiography signal; it is detected that the first electrode group and the second electrode group are implanted into the subcutaneous tissue; and it is detected that the first physiological parameter is not within a first range.

13. The method according to any one of claims 2 to 12, wherein the first physiological parameter comprises any one or more of the following: blood glucose, blood ketone, uric acid, and blood lactic acid; and/or the second physiological parameter comprises any one or more of the following: blood glucose, blood ketone, uric acid, and blood lactic acid.

14. A measurement apparatus, comprising a first electrode group and a second electrode group, wherein a distance between the first electrode group and the second electrode group is greater than a first distance; and
the first electrode group and the second electrode group are further configured to determine an electrocardiography signal when the first electrode group and the second electrode group are implanted into a subcutaneous tissue.

15. The measurement apparatus according to claim 14, wherein the first electrode group comprises a first working electrode and a first counter electrode, the second electrode group comprises a second working electrode, and the first counter electrode forms a loop with the first working electrode; and
that the first electrode group and the second electrode group are configured to determine the electrocardiography signal when the first electrode group and the second electrode group are implanted into the subcutaneous tissue specifically comprises:
the first working electrode, the first counter electrode, and the second working electrode are further configured to determine the electrocardiography signal when the first working electrode, the first counter electrode, and the second working electrode are implanted into the subcutaneous tissue.

16. The measurement apparatus according to claim 14, wherein the first electrode group comprises a first working electrode, a first reference electrode, and a first counter electrode, and the second electrode group comprises a second working electrode, a second reference electrode, and a second counter electrode; and
that the first electrode group and the second electrode group are configured to determine the electrocardiography signal when the first electrode group and the second electrode group are implanted into the subcutaneous tissue specifically comprises:
any two electrodes in the first electrode group and any electrode in the second electrode group are configured to determine the electrocardiography signal when the first electrode group and the second electrode group are implanted into the subcutaneous tissue;
or
any electrode in the first electrode group and any two electrodes in the second electrode group are configured to determine the electrocardiography signal when the first electrode group and the second electrode group are implanted into the subcutaneous tissue.

17. The measurement apparatus according to claim 16, wherein that the any two electrodes in the first electrode group and the any electrode in the second electrode group are configured to determine the electrocardiography signal when the first electrode group and the second electrode group are implanted into the subcutaneous tissue specifically comprises:
when the first counter electrode is connected to a right leg drive circuit, the first counter electrode, the first working electrode, and the second reference electrode are configured to determine the electrocardiography signal when the first electrode group and the second electrode group are implanted into the subcutaneous tissue.

18. The measurement apparatus according to claim 14, wherein the first electrode group comprises a first electrocardiography electrode and a second electrocardiography electrode, the second electrode group comprises a third electrocardiography electrode, and the second electrocardiography electrode is connected to a right leg drive circuit; and
that the first electrode group and the second electrode group are configured to determine the electrocardiography signal when the first electrode group and the second electrode group are implanted into the subcutaneous tissue specifically comprises:
the first electrocardiography electrode, the second electrocardiography electrode, and the third electrocardiography electrode are configured to determine the electrocardiography signal when the first electrode group and the second electrode group are implanted into the subcutaneous tissue.

19. The measurement apparatus according to any one of claims 14 to 18, wherein the first electrode group is configured to determine a first physiological parameter when the first electrode group is implanted into the subcutaneous tissue; and/or the second electrode group is configured to determine a second physiological parameter when the second electrode group is implanted into the subcutaneous tissue.

20. The measurement apparatus according to claim 19, wherein the measurement apparatus further comprises a microcontroller unit MCU, the first electrode group comprises the first working electrode and the first counter electrode, and the first counter electrode forms the loop with the first working electrode; and
that the first electrode group is configured to determine the first physiological parameter when the first electrode group is implanted into the subcutaneous tissue specifically comprises:
the MCU is configured to determine the first physiological parameter when the first working electrode is implanted into the subcutaneous tissue;
or
the measurement apparatus further comprises a microcontroller unit MCU, the first electrode group comprises the first working electrode, the first reference electrode, and the first counter electrode, the first reference electrode is configured to control a voltage of the first working electrode, and
the first counter electrode forms the loop with the first working electrode; and
that the first electrode group is configured to determine the first physiological parameter when the first electrode group is implanted into the subcutaneous tissue specifically comprises:
the first working electrode is configured to generate a first current when the first working electrode is implanted into the subcutaneous tissue; and
the MCU is configured to determine the first physiological parameter based on the first current.

21. The measurement apparatus according to claim 19 or 20, wherein the measurement apparatus further comprises the microcontroller unit MCU;
the second electrode group further comprises the second counter electrode, and the second counter electrode forms a loop with the second working electrode; and
that the second electrode group is configured to determine the second physiological parameter when the second electrode group is implanted into the subcutaneous tissue specifically comprises:
the MCU is configured to determine the second physiological parameter when the second working electrode is implanted into the subcutaneous tissue;
or
the measurement apparatus further comprises the microcontroller unit MCU, the second electrode group comprises the second working electrode, the second reference electrode, and the second counter electrode, the second reference electrode is configured to control a voltage of the second working electrode, and the second counter electrode forms a loop with the second working electrode; and
that the second electrode group is configured to determine the second physiological parameter when the second electrode group is implanted into the subcutaneous tissue specifically comprises:
the second working electrode is configured to generate a second current when the second working electrode is implanted into the subcutaneous tissue; and
the MCU is configured to determine the second physiological parameter based on the second current.

22. The measurement apparatus according to any one of claims 14 to 21, wherein the measurement apparatus comprises a first microneedle sensor and a second microneedle sensor, the first microneedle sensor comprises the first electrode group, and the second microneedle sensor comprises the second electrode group.

23. The measurement apparatus according to any one of claims 14 to 21, wherein the measurement apparatus comprises a first array sensor and a second array sensor, the first array sensor comprises the first electrode group, and the second array sensor comprises the second electrode group.

24. The measurement apparatus according to any one of claims 14 to 23, wherein the measurement apparatus further comprises a first electrochemical circuit module, a second electrochemical circuit module, an electrocardiography circuit module, and the microcontroller unit MCU;
the first electrode group is connected to the first electrochemical circuit module, and the first electrode group is connected to the electrocardiography circuit module;
the second electrode group is connected to the second electrochemical circuit module, and the second electrode group is connected to the electrocardiography circuit module;
the MCU is connected to the first electrochemical circuit module, the second electrochemical circuit module, and the electrocardiography circuit module;
the first electrode group is configured to generate a first current signal;
the first electrode group is further configured to conduct the first current signal to the first electrochemical circuit module;
the first electrochemical circuit module is configured to determine a second current signal based on the first current signal;
the first electrochemical circuit module is further configured to conduct the second current signal to the MCU;
the MCU is configured to determine the first physiological parameter based on the second current signal;
the second electrode group is configured to generate a third current signal;
the second electrode group is further configured to conduct the third current signal to the second electrochemical circuit module;
the second electrochemical circuit module is configured to determine a fourth current signal based on the third current signal;
the second electrochemical circuit module is further configured to conduct the fourth current signal to the MCU;
the MCU is configured to determine the second physiological parameter based on the fourth current signal;
the first electrode group and the second electrode group are configured to obtain a first electrocardiography signal;
the first electrode group and the second electrode group are further configured to conduct the first electrocardiography signal to the electrocardiography circuit module;
the electrocardiography circuit module is configured to determine a second electrocardiography signal based on the first electrocardiography signal; and
the electrocardiography circuit module is further configured to conduct the second electrocardiography signal to the MCU.

25. The measurement apparatus according to claim 24, wherein the first electrode group comprises the first working electrode, the first reference electrode, and the first counter electrode; the first electrochemical circuit module comprises a first potentiostat circuit and a first transimpedance circuit; the first potentiostat circuit is configured to control voltages of the first working electrode and the first reference electrode; and the first transimpedance circuit is configured to amplify the first current signal; and
that the first electrode group is connected to the first electrochemical circuit module specifically comprises:
the first working electrode, the first reference electrode, and the first counter electrode are connected to the first potentiostat circuit; and
the first working electrode is connected to the first transimpedance circuit.

26. The measurement apparatus according to claim 25, wherein the second electrode group comprises the second working electrode, the second reference electrode, and the second counter electrode; the second electrochemical circuit module comprises a second potentiostat circuit and a second transimpedance circuit; the second potentiostat circuit is configured to control voltages of the second working electrode and the second reference electrode; and the second transimpedance circuit is configured to amplify the third current signal; and
that the second electrode group is connected to the second electrochemical circuit module specifically comprises:
the second working electrode, the second reference electrode, and the second counter electrode are connected to the second potentiostat circuit; and
the second working electrode is connected to the second transimpedance circuit.

27. The measurement apparatus according to claim 26, wherein the electrocardiography circuit module comprises the right leg drive circuit, an amplification circuit, and a filter circuit, the amplification circuit is connected to the filter circuit, the right leg drive circuit is configured to cancel a common-mode signal, the amplification circuit is configured to amplify the first electrocardiography signal, and the filter circuit is configured to perform filtering;
that the first electrode group is connected to the electrocardiography circuit module specifically comprises:
the first working electrode is connected to the amplification circuit, and the first counter electrode is connected to the right leg drive circuit;
that the second electrode group is connected to the electrocardiography circuit module specifically comprises:
the second reference electrode is connected to the amplification circuit;
that the first electrode group and the second electrode group are configured to obtain the first electrocardiography signal specifically comprises:
the first working electrode and the second reference electrode are configured to obtain the first electrocardiography signal;
that the first electrode group and the second electrode group are further configured to conduct the first electrocardiography signal to the electrocardiography circuit module specifically comprises:
the first working electrode and the second reference electrode are configured to conduct the first electrocardiography signal to the amplification circuit;
that the electrocardiography circuit module is configured to determine the second electrocardiography signal based on the first electrocardiography signal specifically comprises:
the amplification circuit is configured to: amplify the first electrocardiography signal, and then conduct an amplified first electrocardiography signal to the filter circuit; and
the filter circuit is configured to determine the second electrocardiography signal based on the amplified first electrocardiography signal; and
that the electrocardiography circuit module is further configured to conduct the second electrocardiography signal to the MCU specifically comprises:
the filter circuit is further configured to conduct the second electrocardiography signal to the MCU.

28. The measurement apparatus according to claim 26, wherein the first electrode group further comprises the first electrocardiography electrode and the second electrocardiography electrode, the second electrode group further comprises the third electrocardiography electrode, the electrocardiography circuit module comprises the right leg drive circuit, an amplification circuit, and a filter circuit, the amplification circuit is connected to the filter circuit, the right leg drive circuit is configured to cancel a common-mode signal, the amplification circuit is configured to amplify the first electrocardiography signal, and the filter circuit is configured to perform filtering; that the first electrode group is connected to the electrocardiography circuit module specifically comprises:
the first electrocardiography electrode is connected to the amplification circuit, and the second electrocardiography electrode is connected to the right leg drive circuit;
that the second electrode group is connected to the electrocardiography circuit module specifically comprises:
the third electrocardiography electrode is connected to the amplification circuit;
that the first electrode group and the second electrode group are configured to conduct the first electrocardiography signal to the electrocardiography circuit module specifically comprises:
the first electrocardiography electrode and the third electrocardiography electrode are configured to conduct the first electrocardiography signal to the amplification circuit; that the electrocardiography circuit module is configured to determine the second electrocardiography signal based on the first electrocardiography signal specifically comprises:
the amplification circuit is configured to: amplify the first electrocardiography signal, and then conduct an amplified first electrocardiography signal to the filter circuit; and
the filter circuit is configured to determine the second electrocardiography signal based on the amplified first electrocardiography signal; and
that the electrocardiography circuit module is further configured to conduct the second electrocardiography signal to the MCU specifically comprises:
the filter circuit is further configured to conduct the second electrocardiography signal to the MCU.

29. The measurement apparatus according to any one of claims 14 to 23, further comprising a first multiplexer, the first electrochemical circuit module, the electrocardiography circuit module, and the microcontroller unit MCU, wherein the first multiplexer comprises a first group of input ports, a second group of input ports, and a first group of output ports, and the first multiplexer is configured to connect the first group of input ports or the second group of input ports;
the first electrode group is connected to the first group of input ports, and the first electrode group is connected to the electrocardiography circuit module;
the second electrode group is connected to the second group of input ports, and the second electrode group is connected to the electrocardiography circuit module;
the first group of output ports are connected to the first electrochemical circuit module;
the MCU is connected to the first electrochemical circuit module and the electrocardiography circuit module;
the first electrode group is configured to generate a first current signal;
the first electrode group is further configured to conduct the first current signal to the first multiplexer;
the first multiplexer is configured to conduct the first current signal to the first electrochemical circuit module when the first group of input ports are connected;
the first electrochemical circuit module is configured to determine a second current signal based on the first current signal;
the first electrochemical circuit module is further configured to send the second current signal to the MCU;
the MCU is configured to determine the first physiological parameter based on the second current signal;
the second electrode group is configured to generate a third current signal;
the second electrode group is further configured to conduct the third current signal to the first multiplexer;
the first multiplexer is configured to conduct the third current signal to the first electrochemical circuit module when the second group of input ports are connected;
the first electrochemical circuit module is further configured to determine a fourth current signal based on the third current signal;
the first electrochemical circuit module is further configured to conduct the fourth current signal to the MCU;
the MCU is configured to determine the second physiological parameter based on the fourth current signal;
the first electrode group and the second electrode group are configured to obtain a first electrocardiography signal;
the first electrode group and the second electrode group are further configured to conduct the first electrocardiography signal to the electrocardiography circuit module;
the electrocardiography circuit module is configured to determine a second electrocardiography signal based on the first electrocardiography signal; and
the electrocardiography circuit module is further configured to conduct the second electrocardiography signal to the MCU.

30. The measurement apparatus according to claim 29, wherein the first electrode group comprises the first working electrode, the first reference electrode, and the first counter electrode; the first group of input ports comprise a first input port, a second input port, and a third input port; the second electrode group comprises the second working electrode, the second reference electrode, and the second counter electrode; and the second group of input ports comprise a fourth input port, a fifth input port, and a sixth input port;
the first working electrode is connected to the first input port, the first reference electrode is connected to the second input port, and the first counter electrode is connected to the third input port; and
the second working electrode is connected to the fourth input port, the second reference electrode is connected to the fifth input port, and the second counter electrode is connected to the sixth input port.

31. The measurement apparatus according to claim 30, wherein the first group of output ports comprise a first output port, a second output port, and a third output port;
that the first multiplexer is configured to connect the first group of input ports specifically comprises:
the first output port is configured to be connected to the first input port, the second output port is configured to be connected to the second input port, and the third output port is configured to be connected to the third input port; and
that the first multiplexer is configured to connect the second group of input ports specifically comprises:
the first output port is configured to be connected to the fourth input port, the second output port is configured to be connected to the fifth input port, and the third output port is configured to be connected to the sixth input port.

32. The measurement apparatus according to claim 31, wherein the first electrochemical circuit module comprises a first potentiostat circuit and a first transimpedance circuit; the first potentiostat circuit is configured to control voltages of the first working electrode and the first reference electrode; and the first transimpedance circuit is configured to amplify the first current signal; and that the first group of output ports are connected to the first electrochemical circuit module specifically comprises:
the first output port, the second output port, and the third output port are connected to the first potentiostat circuit; and
the first output port is connected to the first transimpedance circuit.

33. An electronic device, wherein the electronic device comprises the measurement apparatus according to claims 14 to 32.

34. A measurement circuit, wherein the measurement apparatus comprises a first electrode group, a second electrode group, a second multiplexer, a third multiplexer, a fourth multiplexer, a first electrochemical circuit module, an electrocardiography circuit module, and a processor; the second multiplexer comprises a third group of input ports, a fourth group of input ports, and a second group of output ports; and the second multiplexer is configured to connect the third group of input ports or the fourth group of input ports;
the first electrode group is connected to the third group of input ports, and the first electrode group is connected to the electrocardiography circuit module;
the second electrode group is connected to the fourth group of input ports, and the second electrode group is connected to the electrocardiography circuit module;
the third multiplexer and the fourth multiplexer are configured to control the second group of output ports to be connected to the first electrochemical circuit module or the electrocardiography circuit module;
the processor is connected to the first electrochemical circuit module and the electrocardiography circuit module;
the first electrode group and the second electrode group are configured to obtain a first electrocardiography signal;
the first electrode group and the second electrode group are further configured to conduct the first electrocardiography signal to the electrocardiography circuit module when the third multiplexer and the fourth multiplexer control the second group of output ports to be connected to the electrocardiography circuit module; and
the electrocardiography circuit module is configured to determine a second electrocardiography signal based on the first electrocardiography signal; and the electrocardiography circuit module is further configured to conduct the second electrocardiography signal to the processor.

35. The measurement circuit according to claim 34, wherein
the first electrode group is configured to generate a first current signal;
the first electrode group is further configured to conduct the first current signal to the second multiplexer;
the second multiplexer is configured to conduct the first current signal to the first electrochemical circuit module when the second multiplexer connects the third group of input ports, and the third multiplexer and the fourth multiplexer control the second group of output ports to be connected to the first electrochemical circuit module;
the first electrochemical circuit module is configured to determine a second current signal based on the first current signal;
the first electrochemical circuit module is further configured to send the second current signal to the processor; and
the processor is configured to determine a first physiological parameter based on the second current signal;
and/or
the second electrode group is configured to generate a third current signal;
the second electrode group is further configured to conduct the third current signal to the first multiplexer;
the second multiplexer is configured to conduct the third current signal to the first electrochemical circuit module when the second multiplexer connects the fourth group of input ports, and the third multiplexer and the fourth multiplexer control the second group of output ports to be connected to the first electrochemical circuit module;
the first electrochemical circuit module is further configured to determine a fourth current signal based on the third current signal;
the first electrochemical circuit module is further configured to conduct the fourth current signal to the processor; and
the processor is configured to determine a second physiological parameter based on the fourth current signal.

36. The measurement circuit according to claim 35, wherein
the electrocardiography circuit module comprises a right leg drive circuit, an amplification circuit,
the first potentiostat circuit, a first transimpedance circuit, and a filter circuit; the amplification circuit is connected to the filter circuit; the right leg drive circuit is configured to cancel a common-mode signal, the amplification circuit is configured to amplify the first electrocardiography signal, and the first potentiostat circuit is configured to control a voltage of a right leg drive electrode; the first transimpedance circuit is configured to amplify the first electrocardiography signal, and the filter circuit is configured to perform filtering on an amplified first electrocardiography signal; and the first electrode group comprises the right leg drive electrode, or the second electrode group comprises the right leg drive electrode; and
the first electrochemical module comprises the first potentiostat circuit, the first transimpedance circuit, and the filter circuit; the first potentiostat circuit is configured to control voltages of the first working electrode and the first reference electrode, or is configured to control voltages of the second working electrode and the second reference electrode; the first transimpedance circuit is configured to amplify the first current signal, or is configured to amplify the third current signal; and the filter circuit is configured to perform filtering on an amplified first current signal, or is configured to perform filtering on an amplified third current signal.

37. The measurement circuit according to claim 36, wherein that the first electrode group comprises the right leg drive electrode, or the second electrode group comprises the right leg drive electrode comprises:
the right leg drive electrode is any one of the following: the first working electrode, the first reference electrode, the first counter electrode, the second working electrode, the second reference electrode, or the second counter electrode.

38. A chip system, used in a first electronic device, wherein the chip system comprises a processing circuit and an interface circuit, the interface circuit is configured to receive code instructions and transmit the code instructions to the processing circuit, and the processing circuit is configured to run the code instructions, to enable the chip system to perform the method according to any one of claims 1 to 13.

39. A readable storage medium, comprising instructions, wherein when the instructions are run on a first electronic device, the first electronic device is enabled to perform the method according to any one of claims 1 to 13.
